(19) **Europäisches Patentamt / European Patent Office / Office européen des brevets**

(11) **EP 2 398 556 B1**

(12) **EUROPEAN PATENT SPECIFICATION**

(45) Date of publication and mention
of the grant of the patent:
**24.08.2016 Bulletin 2016/34**

(21) Application number: **10744439.0**

(22) Date of filing: **22.02.2010**

(51) Int Cl.:
*A61N 1/372* $^{(2006.01)}$      *A61N 1/362* $^{(2006.01)}$
*A61N 1/39* $^{(2006.01)}$      *A61N 1/05* $^{(2006.01)}$
*A61H 31/00* $^{(2006.01)}$      *H02N 11/00* $^{(2006.01)}$
*A61N 1/378* $^{(2006.01)}$      *H02K 35/02* $^{(2006.01)}$

(86) International application number:
**PCT/US2010/024939**

(87) International publication number:
**WO 2010/096774 (26.08.2010 Gazette 2010/34)**

(54) **IMPLANTABLE MICRO-GENERATOR DEVICES WITH OPTIMIZED CONFIGURATION**

IMPLANTIERBARE MIKROGENERATOREN MIT OPTIMIERTER KONFIGURATION

DISPOSITIFS DE MICROGÉNÉRATEUR IMPLANTABLES AVEC CONFIGURATION OPTIMISÉE

(84) Designated Contracting States:
**AT BE BG CH CY CZ DE DK EE ES FI FR GB GR HR HU IE IS IT LI LT LU LV MC MK MT NL NO PL PT RO SE SI SK SM TR**

(30) Priority: **20.02.2009   US 154170 P**
**20.02.2009   US 154035 P**
**20.02.2009   US 154043 P**
**20.02.2009   US 154223 P**

(43) Date of publication of application:
**28.12.2011   Bulletin 2011/52**

(73) Proprietor: **Endurance Rhythm, Inc.**
**Fremont, CA 94539 (US)**

(72) Inventors:
• **LARSON, Loren Robert**
**Fremont**
**California 94539 (US)**
• **LARSON, Brian Lane**
**Fremont**
**California 94539 (US)**
• **JACOBSON, Peter**
**Livermore**
**California 94550 (US)**
• **PASPA, Paul**
**Los Gatos**
**California 95030 (US)**

(74) Representative: **Price, Nigel John King**
**J A Kemp**
**14 South Square**
**Gray's Inn**
**London WC1R 5JJ (GB)**

(56) References cited:
**WO-A1-99/13940          WO-A1-2007/068284**
**WO-A1-2008/125866     WO-A2-2007/059386**
**WO-A2-2007/109272     US-A1- 2004 260 372**
**US-A1- 2005 256 549     US-A1- 2007 123 938**
**US-A1- 2008 195 188     US-B1- 6 269 266**

EP 2 398 556 B1

**Description**

In order to understand the application of the devices, systems, methods and kits disclosed herein, the disclosure is described in the context of implantable devices and more specifically implantable cardioverter defibrillators and treatment of sudden cardiac arrest. However, as will be appreciated by those skilled in the art, the disclosure can be applied to any device adapted and configured to be implanted within a mammalian body, which requires power in order to function or operate.

**SUDDEN CARDIAC ARREST**

Sudden cardiac arrest ("SCA") most often occurs without warning, striking people with no history of heart problems. It is estimated that more than 1000 people per day are victims of sudden cardiac arrest in the United States alone, which translates into a needless death every 2 minutes.

SCA results when the electrical component of the heart no longer functions properly; this results in an abnormal sinus rhythm. One such abnormal sinus rhythm, ventricular fibrillation ("VF"), is caused by abnormal and very fast electrical activity in the heart. VF may be treated by applying an electric shock to the patient's heart through the use of a defibrillator. The shock clears the heart of abnormal electrical activity (in a process called "defibrillation") by producing a momentary asystole and providing an opportunity for the heart's natural pacemaker areas to restore normal function. If, however, the heart has not been pumping blood for more than 5 minutes, there is an increased likelihood that the victim either will not be resuscitated or will suffer irreversible brain damage. Quick response is therefore necessary.

Once a patient has suffered SCA an implantable cardioverter defibrillator (ICD) is often implanted to monitor the patient's heart rhythm and provide a shock if VF is detected.

Implantable cardiac stimulation devices are well known in the art. Such devices may include, for example, implantable cardiac pacemakers and defibrillators. In humans, the devices are generally implanted anteriorly in a pectoral region of the chest beneath the skin of a patient within what is known as a subcutaneous pocket. The implantable control devices generally function in association with one or more electrode carrying leads, which are implanted within the heart. The electrodes are usually positioned within the right side of the heart, either within the right ventricle or right atrium, or both, for making electrical contact with their respective heart chamber. Conductors within the leads and a proximal connector carried by the leads couple the electrodes to the device to enable the device to sense cardiac electrical activity and deliver the desired therapy.

Ventricular pacing has been a useful technique for at least 50 years, and transvenous pacing for nearly that long. In the transvenous pacing system the lead is placed from a vein, usually in the thorax, and threaded into the right ventricle *22* of the heart *10.* The lead in the right ventricle *22* permits pacing and sensing within that chamber. Of course, pacing from the right ventricle *22* depolarizes the heart in a completely different way than the heart is normally depolarized and does not make use of the patient's own, usually diseased, conduction system. The indication for pacing is an impairment of the patient's conduction system which prevents the system from being able to transmit electrical impulses that allow the heart to depolarize. The depolarization process is what leads to contraction in the cardiac muscle and a beat of the heart.

Biventricular pacing is also indicated for patients with congestive heart failure (CHF) due to left ventricular dysfunction. It is estimated that in approximately 30% of patients with heart failure, an abnormality in the heart's electrical conducting system causes the heart to beat in an asynchronous fashion. That is, the left ventricle fails to contract toward its theoretical center of mass. This asynchrony greatly reduces the efficiency of the heart in some patients with heart failure. Biventricular pacing resynchronizes the contraction of the heart by shortening the actuation time of the ventricles. Biventricular pacemakers differ from other pacemakers, which pace only the right ventricle *22.* Biventricular pacing systems (BVPS), as they are currently constituted, require an operator to thread a catheter from an introducer into the coronary sinus *80*

In order to implant an endocardial lead within a heart chamber, a transvenous approach is typically utilized wherein the lead is inserted into and passed through the subclavian, jugular, or cephalic vein and through the superior vena cava into the right atrium or ventricle. An active or passive fixation mechanism is incorporated into the distal end of the endocardial lead and deployed to maintain the distal end electrode in contact with the endocardium position. More recently, endocardial pacing and cardioversion/defibrillation leads have been developed that are adapted to be advanced into the coronary sinus and coronary veins branching therefrom in order to locate the distal electrode(s) adjacent to the left ventricle or the left atrium. The distal end of such coronary sinus leads is advanced through the superior vena cava, the right atrium, the valve of the coronary sinus, the coronary sinus, and into a coronary vein communicating with the coronary sinus, such as the great vein. Typically, coronary sinus leads do not employ any fixation mechanism and instead rely on the close confinement within these vessels to maintain each electrode at the cardiac implantation site.

**IMPLANTABLE CARDIAC DEVICES: PACEMAKERS, PULSE GENERATORS, CRTs, ICDs, ETC.**

Implantable cardioverter defibrillators (ICD's) are capable of detecting fibrillation of the heart and delivering electrical shock therapy to one or more heart chambers to terminate the fibrillation. The defibrillating electrical energy may be applied, for example, in the superior vena cava (SVC) of the heart and/or the right ventricle (RV) of the heart. Defibrillating electrodes are generally quite large, as compared to pacing electrodes, and commonly take the form of elongated coils. The coils are usually formed of platinum, which has an electropositivity in the blood of about +125 mV and is considered biocompatible.

The energy consumption of the cardiac implant varies according to its function, this being determined by the pathology and activity of the patient. Thus, the duration (usable life) of the cardiac implant battery varies from 3 to 9 years, being essentially determined by the electrical energy consumption.

The electrical energy consumption, not considering the scale factor referring to the voltage amplitude, is proportional to the product between the median current value and the interval of time during which the same persists. This product characterizes the electrical charge that moves from the battery through the implant's electronic circuit. What is needed, therefore, are more reliable and long lasting devices and methods for providing power to implantable devices such as ICDs (implantable cardioverter defibrillators).

Implantable cardiac devices are used to treat cardiac arrhythmias, which left untreated can be fatal. These devices include pacemakers, ICDs, and CRT/ CRT-Ds (cardiac resynchronization therapy without and with defibrillation).

ICDs, CRT-Ds, and pacemakers have two major components: the "leads" and the "can," or implantable control housing, that encapsulates the electronics and other components forming a functional ICD while protecting the ICD components from the physiological environment into which they are implanted. The pulse generator implantable control housing is implanted underneath the skin near the collarbone. Wire leads run from the generator, through the vessels, and to the heart wall.

Pulse generators (PGs) are battery-powered medical devices that are implanted in patients and provide electrical pulses (therapy) to stimulate or shock the patient's heart. PGs include cardiac rhythm management (CRM) devices, such as pacemakers, heart failure devices, and defibrillators. One or more remote sensors may be under the control of the PG to provide information that may be used to determine whether to administer therapy to the patient. A battery may serve as the power source in each of the sensors, providing power to, for example, measure physiological parameters and transmit data related to the measured parameters via telemetry. Accurate determination of a battery charge of a remote device supports an effective assessment of the appropriate replacement or recharge time.

Pacemakers produce low voltage rhythmic electrical signals that remedy a diseased heart's defective ability to generate its own electrical signals, which may cause the heart to beat too fast, too slowly, or irregularly. The pacemaker continuously monitors the heart's electrical system, and delivers an electrical impulse to aid the heart when it detects a need. The vast majority of pacemakers are used to treat bradyarrhythmia or bradycardia, which is when the heart beats too slow due to a defect in the sinoatrial node or a blockage in the heart's own electrical conduction system, thus reducing blood flow and preventing the body from receiving the blood it needs.

An ICD, on the other hand, delivers electrical impulses to the heart when it detects heart beats that are too fast or asynchronous. The amount of energy delivered by an ICD is very large, allowing the heart to effectively reboot its electrical conducting system. They are about the twice the size of a pacemaker and are implanted in a similar location underneath the skin.

**BATTERY POWER/OPERATION**

Batteries have limited life spans. When the charge is depleted in a battery, the battery-powered device will cease to function. To circumvent loss of functionality, the battery must be replaced or recharged prior to charge depletion. Accurate determination of a battery's state of depletion is particularly important for battery-powered medical devices that are implanted in human patients. With an accurate determination of battery depletion state, an implanted medical device may be recharged or replaced in order to maintain monitoring and/or therapy. Understanding the amount of available battery life for a battery deployed in conjunction with an implanted medical device is particularly important because failure to replace the device and/or battery prior to exhaustion of usable battery power could result in a failure of the device to appropriately operate as required to treat the patient.

Both pacemakers and ICDs are powered by non-rechargeable batteries. Accordingly, battery failure often leads to replacement of the entire device. Approximately 76% of all replacement reoperations are for battery failure. According to data published by Hauser et al., there is a 50% cumulative probability of ICD device failure within a little over 5 years. For the increasingly popular CRT-D (Cardiac Resynchronization Therapy with Defibrillator, combining biventricular pacing and defibrillation functionality), device life is significantly lower still. Based on typical patient longevities, most patients will require at least one replacement device based on these current device life spans.

Modern day implantable electronic medical devices typically use non-rechargeable Lithium or high amperage Silver Vanadium Oxide batteries. Battery lifetime is virtually always the component that limits the lifespan of ICDs. As a result, the devices need to be replaced, requiring re-operations that are costly and create risks to the patient.

Devices that have been previously contemplated include, for example, U.S. Patent Pub. 2004/0222637A1 to Bednyak for Apparatus and Method for Generating Electrical Energy from Motion (see also corresponding U.S Patent 7,105,939 B2); PCT Publication WO 2004/032788 A2 to Holzer for Micro-Generator Implant. WO 99/13940 discloses an implantable micro-generator in accordance with the preamble of claim 1.

## STATUS OF THE CURRENT INDUSTRY

The cardiac rhythm management industry is broken up into four main sectors, ICDs, CRTs, pacemakers, and ablation. Three of the sectors are of particular interest from a power perspective, since the ablation market does not involve implantable medical devices.

The market for ICDs is currently growing at a rate of approximately 17% annually due to a confluence of factors: aging of the baby boomer generation, increased physician and patient adoption, broadened clinical indications, and implantation earlier in disease processes. ICD revenues in the U.S. are estimated to be $7 billion from the 250,000 ICDs implanted in 2008. The U.S. currently accounts for about 60% of the worldwide CRM market.

ICDs are a rapidly growing segment, with huge future potential. Studies estimate that only 15% of patients eligible for ICD treatment currently have implants. Current estimates project a significant increase in the CRM market size, with ICDs accounting for over $13 billion in 2012.

## REOPERATION RATES AND RISKS

Currently, re-operations for implantable devices make up a significant portion of the market. There were ~43,000 ICD re-operations in 2005 and about 22,000 ICD re-operations in 2004. The huge expense of this segment necessitates a better solution that would eliminate the need for these costly procedures, providing strong incentives from payers. The typical cost of an ICD implantation is estimated at $40,000-$50,000 (MedScape Today). The overall cost to the healthcare system is substantial. Combined, the need to replace devices costs health care systems over $1.2 billion today.

In addition to the high cost of existing implantable cardiac devices, there is a potential increase in the risk of surgical complications from re-operations. Overall, infection is the greatest risk ranging from 0.8% to 5.7%. Additionally, pocket hematomas (incidence: 4.9%) can also develop, which often require removal and re-operation that further increases morbidity associated with the device. Moreover, some studies suggest that the risk of complications may increase as much as three-fold in re-operations. Notwithstanding that fact, as those skilled in the art will appreciate that any procedure involves risk of infection and injury, therefore reducing the number of procedures is beneficial both from a healthcare cost perspective and a patient safety perspective. Accordingly, reducing complications that might arise from additional procedures and the effective unit cost of an implantable cardiac device by decreasing the frequency of re-operation is desirable.

The large number of re-operations illustrates the prevalence of battery depletion failure and an opportunity to provide an effective, cheaper device that would result in fewer follow-up complications.

In a time of extreme healthcare cost consciousness and a historic trend toward longevity, reducing the number of implantations will benefit patients, physicians, and the public. What is needed is a way to power implantable devices in such a way so that the longevity of the device is increased and the need for re-operation is reduced.

## SUMMARY OF THE INVENTION

An aspect of the disclosure is directed to a micro-generator adapted and configured to harness kinetic energy from, for example, the mammalian heart to power electronic implants. The micro-generator can be formed as an integral part of a fully-implanted pacer device, such that the micro-generator is formed as part of the device or acts in a unified manner with the device. Thus, at least some configurations of the device can be configured to essentially be a self-contained capsule with its own miniature battery and electrodes that attaches right into the heart with no lead running out of the heart. Such a configuration would be particularly suitable for pacemakers. The micro-generator can be designed to be attached to or formed integrally with a self-contained pacer capsule device to provide adjunct or full power. Since the human heart beats 60-100 times each minute, it provides a regular source of mechanical energy. Pacemakers and ICDs have two major components: a control device that sits underneath the skin near the collarbone, and a multi-wire lead connecting the device to the heart wall. The micro-generator according to this disclosure will be placed within or near the lead tip in one embodiment. Thus, as the heart muscle contracts and moves the lead, the generator converts this motion into electricity, providing adjuvant power for the device's primary battery. The generator may also be placed in a dedicated lead or other device attached to the inside or the outside of the heart, and the power routed back to the control device.

The present invention provides an implantable micro-generator, as defined by appended claim 1.

An aspect of the disclosure is directed to an implantable micro-generator. The micro-generator comprises: an elongated housing adapted and configured to be positioned distally within a tip of a cardiac lead wherein the housing has an elongated interior cavity, a first end and a second end; one or more longitudinally slidable elongated magnets; one or more coils positioned exteriorly, interiorly or integrally along at least a portion of the housing; a power wire in electrical communication with the one or more coils and with an implantable medical device; wherein the implantable micro-generator is adapted and configured to generate energy and communicate the generated energy to the implantable medical device. In some configurations, the longitudinally slidable elongated magnet has a longitudinal length greater than a cross-sectional diameter and a length less than a length of the housing cavity, positioned within the interior cavity of the elongated housing. Additionally, one or more coils can be configured such that the coils are an inductive coil. In some cases, at least a portion of the coil is at least one of embedded or sealed within at least one of the housing and lead. Moreover, in some configurations, the micro-generator is configured to attach to the cardiac lead. Still other configurations contemplate a design wherein the diameter of the micro-generator is greater than at least a diameter of the lead at a portion of its length, the cardiac lead further comprises a shock wire adjacent to at least a portion of the coil of the micro-generator, the interior cavity of the elongated housing has a cross-sectional dimension approaching a cross-sectional dimension of the magnet, the cross-sectional shape of the magnet is selected from the group comprising: round, triangular, tetragonal, pentagonal, hexagonal, heptagonal, octagonal, nonagonal, decagonal, oval, and ellipsoid, at least one of a first end or a second end of the magnet is configured to be rounded, and/or the housing further comprises one or more of at least one of springs and bumpers at either one or both of the first end and the second end of the cavity of the elongated housing. At least one of the one or more of at least one of springs and bumpers can be configured such that they are highly elastic, have a shape selected from conical, cylindrical, straight, square, nub, coiled, spherical, trapezoidal, leaf, are adapted and configured to be attached to an end wall of the housing, are positioned within the housing without attachment to an interior surface of the housing, are magnets, are a gaseous region within the housing adapted and configured to provide a spring characteristic between the magnet and at least one end of the housing, and/or are adapted and configured to allow a signal to pass through the micro-generator. Flexible wires can be used to allow a signal to pass through the micro-generator. Additionally, the springs are at least one of conical, cylindrical, straight, square, nub, coiled, spherical, trapezoidal, leaf and/or configured to engage the magnet at a first end and a second end to suspend the magnet between the ends of the cavity of the housing. In still other configuration, the micro-generator comprises one or more magnets positioned at least at one of the first end and the second end of the elongated housing, such as where the magnets are positioned within the cavity of the elongated housing. A sealing member may be provided in some configurations around at least a portion of the elongated housing. For example, a feed through in the sealing member through which the power wires travel and/or sealing material, such as epoxy, waterproof sealants, and biocompatible sealants can be used. In some configurations, the magnet is a flexible magnet which can itself comprise a plurality of magnets in flexible communication. Still other configurations have a curved cavity. In some embodiments, one or more sensors may be provided, for example, suitable sensors might include a generator monitor, voltage sensor, a physiological sensor, motion sensor, positional sensor, and a wake-up sensor. Sensors might be configured to be in communication with the implantable medical device via one or more signal wires. Additionally, sensors can be attached to the implantable micro-generator. The cardiac lead can further be modified to accommodate at least one of power wires, pacing wires, signal wires, and shock wires within an interior channel in the lead housing. Additionally, in some configurations, the micro-generator cavity is evacuated and the magnet is suspended in the vacuum. In still other configurations there is at least one or more fluid or gas (such as an inert gas) within the cavity. The longitudinally slidable elongated magnet can further be configured to define an aperture along its length which can also be a hollow lumen. In some configurations, a second, third or fourth implantable micro-generator is also provided - or however many is desired or desirable. In such a configuration having a plurality of micro-generators, the first and second micro-generators can be electrically connected - or any combination of connections that is desired. Additionally or in the alternative, the second micro-generator can be configured such that it is in communication with the first micro-generator and or any other micro-generator in the system. A second coil can be provided that is positioned distally or proximally from the first coil. The micro-generator can be variably positionable within the tip of the cardiac lead such that it can be moved within the lead either toward the tip or away from the tip, as is desired or desirable at a particular time. The useful life of the implanted medical device is extended more than 10%, but typically greater than 25%, more typically greater than 50%. The implantable medical device is selected from the group comprising pacemakers, implantable cardioverter defibrillators, cardiac resynchronization therapy devices, and cardiac resynchronization therapy devices with defibrillation. The lead is adapted and configured to attach to cardiac tissue, and can be attached, for example, epicardially, or within the ventricular or atrial chamber. Electronics can be provided that are adapted and configured to process a power signal received from the micro-generator. At least some of the electronics may be positioned within the lead and/or the micro-generator. Moreover, the electronics can be adapted and configured to buffer a power signal received from the micro-generator. A storage component can also be provided that is adapted and configured to store energy generated by the micro-generator, either temporarily or for an extended period of time. The micro-generator can further be configured to process, smooth, or rectify an oscillating voltage waveform. Additionally, the power wire can be positioned within the central hollow portion of the lead. In some instances, the housing is hermetically sealed. The housing can also be evacuated and sealed. In still other configurations, a secondary housing can be provided. The magnet can also be segmented. The wire can be routed at least one of centrally and alongside the micro-generator module, embedded in the wall of the housing, or routed in a tubing adapted and configured to house one or more wires. Additionally, the micro-generator can also comprise a core, such as a core of ferrite. Configurations with a core can be configured such that the core and the magnet are moveable relative to each other - for example, where both are capable or movement or where one is in a fixed location and the other component moves relative to the fixed component. One or more inductive coils can be positioned around the core. Additionally, the lead can be a power generation lead or a multi-function lead (i.e., a lead capable of power generation and one or more other functions). A generator can be positioned within an implantable medical device. Additionally, one or more rechargeable batteries can be provided. One or more capacitors can be provided that are adapted and configured to at least one of store and buffer power from the micro-generator. Additionally, the capacitors can be adapted and configured to be topped off. An attachment mechanism can also be provided that is adapted and configured to attach the lead to the implantable device. One or more coils and magnets can also be provided that are configured to maximize an integral of the square of the rate of change of flux over time. The micro-generator is used at least one of in parallel or in series with one or more standard or rechargeable batteries. A multiplexer can also be provided that is adapted and configured to multiplex one or more signals along the wires from a tip of the lead to the micro-generator and the implantable control device. Micro-generators will typically range from 7 French to 20 French, more specifically 8 French to 13 French, and even more specifically 9 French to 12 French. A lubricant, coating, film and/or plating can be used on some or all surfaces of the components of the micro-generator. Lubricants include, for example, silicon, carbon, and carbon spheres. Plating is typically nickel or other hard metal. The housing is typically formed from a material having a high transparency to magnetic flux, such as titanium, aluminum, ceramic, and glass. Additionally, the wires are often insulated. In at least some configurations, the spring is connected to the magnet. The one or more magnets are comprised at least two magnets and further wherein the magnets are positioned within the housing such that at least one of a north pole and a south pole of a first magnet is adjacent to a corresponding north pole or south pole of a second magnet.

Another aspect of the disclosure is directed to an implantable cardiac rhythm management system. The system comprises: a cardiac rhythm management device; an elongated housing adapted and configured to be positioned distally within a tip of a cardiac lead wherein the housing has an elongated interior cavity, a first end and a second end; one or more longitudinally slidable elongated magnets; one or more coils positioned exteriorly, interiorly or integrally along at least a portion of the housing; a power wire in electrical communication with the one or more coils and with an implantable medical device; wherein the implantable cardiac rhythm management system is adapted and configured to generate energy and communicate the generated energy to the implantable medical device. In some configurations, the longitudinally slidable elongated magnet has a longitudinal length greater than a cross-sectional diameter and a length less than a length of the housing cavity, positioned within the interior cavity of the elongated housing. Additionally, one or more coils can be configured such that the coils are an inductive coil. In some cases, at least a portion of the coil is at least one of embedded or sealed within at least one of the housing and lead. Moreover, in some configurations, the micro-generator is configured to attach to the cardiac lead. Still other configurations contemplate a design wherein the diameter of the micro-generator is greater than at least a diameter of the lead at a portion of its length, the cardiac lead further comprises a shock wire adjacent to at least a portion of the coil of the micro-generator, the interior cavity of the elongated housing has a cross-sectional dimension approaching a cross-sectional dimension of the magnet, the cross-sectional shape of the magnet is selected from the group comprising: round, triangular, tetragonal, pentagonal, hexagonal, heptagonal, octagonal, nonagonal, decagonal, oval, and ellipsoid, at least one of a first end or a second end of the magnet is configured to be rounded, and/or the housing further comprises one or more of at least one of springs and bumpers at either one or both of the first end and the second end of the cavity of the elongated housing. At least one of the one or more of at least one of springs and bumpers can be configured such that they are highly elastic, have a shape selected from conical, cylindrical, straight, square, nub, coiled, spherical, trapezoidal, leaf, are adapted and configured to be attached to an end wall of the housing, are positioned within the housing without attachment to an interior surface of the housing, are magnets, are a gaseous region within the housing adapted and configured to provide a spring characteristic between the magnet and at least one end of the housing, and/or are adapted and configured to allow a signal to pass through the micro-generator. Flexible wires can be used to allow a signal to pass through the micro-generator. Additionally, the springs are at least one of conical, cylindrical, straight, square, nub, coiled, spherical, trapezoidal, leaf and/or configured to engage the magnet at a first end and a second end to suspend the magnet between the ends of the cavity of the housing. In still other configuration, the micro-generator comprises one or more magnets positioned at least at one of the first end and the second end of the elongated housing, such as where the magnets are positioned within the cavity of the elongated housing. A sealing member may be provided in some configurations around at least a portion of the elongated housing. For example, a feed through in the sealing member through which the power wires travel and/or sealing material, such as epoxy, waterproof sealants, and biocompatible sealants can be used. In some configurations, the magnet is a flexible magnet which can itself comprise a plurality of magnets in flexible communication. Still other configurations have a curved cavity. In some embodiments, one or more sensors may be provided, for example, suitable sensors might include a generator monitor, voltage sensor, a physiological sensor, motion sensor, positional sensor, and a wake-up sensor. Sensors might be configured to be in communication with the implantable medical device via one or more signal wires. Additionally, sensors can be attached to the implantable micro-generator. The cardiac lead can further be modified to accommodate at least one of power wires, pacing wires, signal wires, and shock wires within an interior channel in the lead housing. Additionally, in some configurations, the micro-generator cavity is evacuated and the magnet is suspended in the vacuum. In still other configurations there is at least one or more fluid or gas (such as an inert gas) within the cavity. The longitudinally slidable elongated magnet can further be configured to define an aperture along its length which can also be a hollow lumen. In some configurations, a second, third or fourth implantable micro-generator is also provided - or however many is desired or desirable. In such a configuration having a plurality of micro-generators, the first and second micro-generators can be electrically connected - or any combination of connections that is desired. Additionally or in the alternative, the second micro-generator can be configured such that it is in communication with the first micro-generator and or any other micro-generator in the system. A second coil can be provided that is positioned distally or proximally from the first coil. The micro-generator can be variably positionable within the tip of the cardiac lead such that it can be moved within the lead either toward the tip or away from the tip, as is desired or desirable at a particular time. The useful life of the implanted medical device is extended more than 10%, but typically greater than 25%, more typically greater than 50%. The implantable medical device is selected from the group comprising pacemakers, implantable cardioverter defibrillators, cardiac resynchronization therapy devices, and cardiac resynchronization therapy devices with defibrillation. The lead is adapted and configured to attach to cardiac tissue, and can be attached, for example, epicardially, or within the ventricular or atrial chamber. Electronics can be provided that are adapted and configured to process a power signal received from the micro-generator. At least some of the electronics may be positioned within the lead and/or the micro-generator. Moreover, the electronics can be adapted and configured to buffer a power signal received from the micro-generator. A storage component can also be provided that is adapted and configured to store energy generated by the micro-generator, either temporarily or for an extended period of time. The micro-generator can further be configured to process, smooth or rectify an oscillating voltage waveform. Additionally, the power wire can be positioned within the central hollow portion of the lead. In some instances, the housing is hermetically sealed. The housing can also be evacuated and sealed. In still other configurations, a secondary housing can be provided. The magnet can also be segmented. The wire can be routed at least one of centrally and alongside the micro-generator module, embedded in the wall of the housing, or routed in a tubing adapted and configured to house one or more wires. Additionally, the micro-generator can also comprise a core, such as a core of ferrite. Configurations with a core can be configured such that the core and the magnet are moveable relative to each other - for example, where both are capable or movement or where one is in a fixed location and the other component moves relative to the fixed component. One or more inductive coils can be positioned around the core. Additionally, the lead can be a power generation lead or a multi-function lead (i.e., a lead capable of power generation and one or more other functions). A generator can be positioned within an implantable medical device. Additionally, one or more rechargeable batteries can be provided. One or more capacitors can be provided that are adapted and configured to at least one of store and buffer power from the micro-generator. Additionally, the capacitors can be adapted and configured to be topped off. An attachment mechanism can also be provided that is adapted and configured to attach the lead to the implantable device. One or more coils and magnets can also be provided that are configured to maximize an integral of the square of the rate of change of flux over time. The micro-generator is used at least one of in parallel or in series with one or more standard or rechargeable batteries. A multiplexer can also be provided that is adapted and configured to multiplex one or more signals along the wires from a tip of the lead to the micro-generator and the implantable control device. Micro-generators will typically range from 7 French to 20 French, more specifically 8 French to 13 French, and even more specifically 9 French to 12 French. A lubricant, coating, film and/or plating can be used on some or all surfaces of the components of the micro-generator. Lubricants include, for example, silicon, carbon, and carbon spheres. Plating is typically nickel or other suitable hard metal. The housing is typically formed from a material having a high transparency to magnetic flux, such as titanium, aluminum, ceramic, and glass. Additionally, the wires are often insulated. In at least some configurations, the spring is connected to the magnet. The one or more magnets are comprised at least two magnets and further wherein the magnets are positioned within the housing such that at least one of a north pole and a south pole of a first magnet is adjacent to a corresponding north pole or south pole of a second magnet.

Still another aspect of the disclosure is directed to an implantable neural stimulation device. The neural stimulation device comprises: a power supply; an electrode; a neural stimulator connected to the power supply, the neural stimulator being adapted to generate a neural stimulation signal for delivery to the neural stimulation target through the electrode; a controller connected to the power supply, and further connected to the neural stimulator to control the neural stimulator according to a neural stimulation protocol to deliver a neural stimulation therapy; a pressure sensor electrically connected to the controller; and an implantable micro-generator comprising, an elongated housing adapted and configured to be positioned distally within a tip of a cardiac lead wherein the housing has an elongated interior cavity, a first end and a second end, one or more longitudinally slidable elongated magnets, one or more coils positioned exteriorly, interiorly or integrally along at least a portion of the housing, a power wire in electrical communication with the one or more coils and with the neural stimulation device, wherein the implantable micro-generator is adapted and configured to generate energy and communicate the generated energy to the neural stimulation device. In some configurations, the longitudinally slidable elongated magnet has a longitudinal length greater than a cross-sectional diameter and a length less than a length of the housing cavity, positioned within the interior cavity of the elongated housing. Additionally, one or more coils can be configured such that the coils are an inductive coil. In some cases, at least a portion of the coil is at least one of embedded or sealed within at least one of the housing and lead. Moreover, in some configurations, the micro-generator is configured to attach to the cardiac lead. Still other configurations contemplate a design wherein the diameter of the micro-generator is greater than at least a diameter of the lead at a portion of its length, the cardiac lead further comprises a shock wire adjacent to at least a portion of the coil of the micro-generator, the interior cavity of the elongated housing has a cross-sectional dimension approaching a cross-sectional dimension of the magnet, the cross-sectional shape of the magnet is selected from the group comprising: round, triangular, tetragonal, pentagonal, hexagonal, heptagonal, octagonal, nonagonal, decagonal, oval, and ellipsoid, at least one of a first end or a second end of the magnet is configured to be rounded, and/or the housing further comprises one or more of at least one of springs and bumpers at either one or both of the first end and the second end of the cavity of the elongated housing. At least one of the one or more of at least one of springs and bumpers can be configured such that they are highly elastic, have a shape selected from conical, cylindrical, straight, square, nub, coiled, spherical, trapezoidal, leaf, are adapted and configured to be attached to an end wall of the housing, are positioned within the housing without attachment to an interior surface of the housing, are magnets, are a gaseous region within the housing adapted and configured to provide a spring characteristic between the magnet and at least one end of the housing, and/or are adapted and configured to allow a signal to pass through the micro-generator. Flexible wires can be used to allow a signal to pass through the micro-generator. Additionally, the springs are at least one of conical, cylindrical, straight, square, nub, coiled, spherical, trapezoidal, leaf and/or configured to engage the magnet at a first end and a second end to suspend the magnet between the ends of the cavity of the housing. In still other configuration, the micro-generator comprises one or more magnets positioned at least at one of the first end and the second end of the elongated housing, such as where the magnets are positioned within the cavity of the elongated housing. A sealing member may be provided in some configurations around at least a portion of the elongated housing. For example, a feed through in the sealing member through which the power wires travel and/or sealing material, such as epoxy, waterproof sealants, and biocompatible sealants can be used. In some configurations, the magnet is a flexible magnet which can itself comprise a plurality of magnets in flexible communication. Still other configurations have a curved cavity. In some embodiments, one or more sensors may be provided, for example, suitable sensors might include a generator monitor, voltage sensor, a physiological sensor, motion sensor, positional sensor, and a wake-up sensor. Sensors might be configured to be in communication with the implantable medical device via one or more signal wires. Additionally, sensors can be attached to the implantable micro-generator. The cardiac lead can further be modified to accommodate at least one of power wires, pacing wires, signal wires, and shock wires within an interior channel in the lead housing. Additionally, in some configurations, the micro-generator cavity is evacuated and the magnet is suspended in the vacuum. In still other configurations there is at least one or more fluid or gas (such as an inert gas) within the cavity. The longitudinally slidable elongated magnet can further be configured to define an aperture along its length which can also be a hollow lumen. In some configurations, a second, third or fourth implantable micro-generator is also provided - or however many is desired or desirable. In such a configuration having a plurality of micro-generators, the first and second micro-generators can be electrically connected - or any combination of connections that is desired. Additionally or in the alternative, the second micro-generator can be configured such that it is in communication with the first micro-generator and or any other micro-generator in the system. A second coil can be provided that is positioned distally or proximally from the first coil. The micro-generator can be variably positionable within the tip of the cardiac lead such that it can be moved within the lead either toward the tip or away from the tip, as is desired or desirable at a particular time. The useful life of the implanted neural stimulation device is extended more than 10%, but typically greater than 25%, more typically greater than 50%. Electronics can be provided that are adapted and configured to process a power signal received from the micro-generator. At least some of the electronics may be positioned within the lead and/or the micro-generator. Moreover, the electronics can be adapted and configured to buffer a power signal received from the micro-generator. A storage component can also be provided that is adapted and configured to store energy generated by the micro-generator, either temporarily or for an extended period of time. The micro-generator can further be configured to process, smooth or rectify an oscillating voltage waveform. Additionally, the power wire can be positioned within the central hollow portion of the lead. In some instances, the housing is hermetically sealed. The housing can also be evacuated and sealed. In still other configurations, a secondary housing can be provided. The magnet can also be segmented. The wire can be routed at least one of centrally and alongside the micro-generator module, embedded in the wall of the housing, or routed in a tubing adapted and configured to house one or more wires. Additionally, the micro-generator can also comprise a core, such as a core of ferrite. Configurations with a core can be configured such that the core and the magnet are moveable relative to each other - for example, where both are capable or movement or where one is in a fixed location and the other component moves relative to the fixed component. One or more inductive coils can be positioned around the core. Additionally, the lead can be a power generation lead or a multi-function lead (i.e., a lead capable of power generation and one or more other functions). A generator can be positioned within an implantable medical device. Additionally, one or more rechargeable batteries can be provided. One or more capacitors can be provided that are adapted and configured to at least one of store and buffer power from the micro-generator. Additionally, the capacitors can be adapted and configured to be topped off. An attachment mechanism can also be provided that is adapted and configured to attach the lead to the implantable device. One or more coils and magnets can also be provided that are configured to maximize an integral of the square of the rate of change of flux over time. The micro-generator is used at least one of in parallel or in series with one or more standard or rechargeable batteries. A multiplexer can also be provided that is adapted and configured to multiplex one or more signals along the wires from a tip of the lead to the micro-generator and the implantable control device. Micro-generators will typically range from 7 French to 20 French, more specifically 8 French to 13 French, and even more specifically 9 French to 12 French. A lubricant, coating, film and/or plating can be used on some or all surfaces of the components of the micro-generator. Lubricants include, for example, silicon, carbon, and carbon spheres. Plating is typically nickel or other suitable hard metal. The housing is typically formed from a material having a high transparency to magnetic flux, such as titanium, aluminum, ceramic, and glass. Additionally, the wires are often insulated. In at least some configurations, the spring is connected to the magnet. The one or more magnets are comprised at least two magnets and further wherein the magnets are positioned within the housing such that at least one of a north pole and a south pole of a first magnet is adjacent a corresponding north pole or south pole of a second magnet.

Another aspect of the disclosure is directed to an implantable tissue stimulation device. The tissue stimulation device comprises: a power supply; an electrode; a neural stimulator connected to the power supply, the neural stimulator being adapted to generate a tissue stimulation signal for delivery to the tissue stimulation target through the electrode; a controller connected to the power supply, and further connected to the neural stimulator to control the neural stimulator according to a tissue stimulation protocol to deliver a tissue stimulation therapy; a pressure sensor electrically connected to the controller; and an implantable micro-generator comprising, an elongated housing adapted and configured to be positioned distally within a tip of a cardiac lead wherein the housing has an elongated interior cavity, a first end and a second end, one or more longitudinally slidable elongated magnets, one or more coils positioned exteriorly, interiorly or integrally along at least a portion of the housing, a power wire in electrical communication with the one or more coils and with the tissue stimulation device, wherein the implantable micro-generator is adapted and configured to generate energy and communicate the generated energy to the tissue stimulation device. In some configurations, the longitudinally slidable elongated magnet has a longitudinal length greater than a cross-sectional diameter and a length less than a length of the housing cavity, positioned within the interior cavity of the elongated housing. Additionally, one or more coils can be configured such that the coils are an inductive coil. In some cases, at least a portion of the coil is at least one of embedded or sealed within at least one of the housing and lead. Moreover, in some configurations, the micro-generator is configured to attach to the cardiac lead. Still other configurations contemplate a design wherein the diameter of the micro-generator is greater than at least a diameter of the lead at a portion of its length, the cardiac lead further comprises a shock wire adjacent to at least a portion of the coil of the micro-generator, the interior cavity of the elongated housing has a cross-sectional dimension approaching a cross-sectional dimension of the magnet, the cross-sectional shape of the magnet is selected from the group comprising: round, triangular, tetragonal, pentagonal, hexagonal, heptagonal, octagonal, nonagonal, decagonal, oval, and ellipsoid, at least one of a first end or a second end of the magnet is configured to be rounded, and/or the housing further comprises one or more of at least one of springs and bumpers at either one or both of the first end and the second end of the cavity of the elongated housing. At least one of the one or more of at least one of springs and bumpers can be configured such that they are highly elastic, have a shape selected from conical, cylindrical, straight, square, nub, coiled, spherical, trapezoidal, leaf, are adapted and configured to be attached to an end wall of the housing, are positioned within the housing without attachment to an interior surface of the housing, are magnets, are a gaseous region within the housing adapted and configured to provide a spring characteristic between the magnet and at least one end of the housing, and/or are adapted and configured to allow a signal to pass through the micro-generator. Flexible wires can be used to allow a signal to pass through the micro-generator. Additionally, the springs are at least one of conical, cylindrical, straight, square, nub, coiled, spherical, trapezoidal, leaf and/or configured to engage the magnet at a first end and a second end to suspend the magnet between the ends of the cavity of the housing. In still other configuration, the micro-generator comprises one or more magnets positioned at least at one of the first end and the second end of the elongated housing, such as where the magnets are positioned within the cavity of the elongated housing. A sealing member may be provided in some configurations around at least a portion of the elongated housing. For example, a feed through in the sealing member through which the power wires travel and/or sealing material, such as epoxy, waterproof sealants, and biocompatible sealants can be used. In some configurations, the magnet is a flexible magnet which can itself comprise a plurality of magnets in flexible communication. Still other configurations have a curved cavity. In some embodiments, one or more sensors may be provided, for example, suitable sensors might include a generator monitor, voltage sensor, a physiological sensor, motion sensor, positional sensor, and a wake-up sensor. Sensors might be configured to be in communication with the implantable medical device via one or more signal wires. Additionally, sensors can be attached to the implantable micro-generator. The cardiac lead can further be modified to accommodate at least one of power wires, pacing wires, signal wires, and shock wires within an interior channel in the lead housing. Additionally, in some configurations, the micro-generator cavity is evacuated and the magnet is suspended in the vacuum. In still other configurations there is at least one or more fluid or gas (such as an inert gas) within the cavity. The longitudinally slidable elongated magnet can further be configured to define an aperture along its length which can also be a hollow lumen. In some configurations, a second, third or fourth implantable micro-generator is also provided - or however many is desired or desirable. In such a configuration having a plurality of micro-generators, the first and second micro-generators can be electrically connected -or any combination of connections that is desired. Additionally or in the alternative, the second micro-generator can be configured such that it is in communication with the first micro-generator and or any other micro-generator in the system. A second coil can be provided that is positioned distally or proximally from the first coil. The micro-generator can be variably positionable within the tip of the cardiac lead such that it can be moved within the lead either toward the tip or away from the tip, as is desired or desirable at a particular time. The useful life of the implanted tissue stimulation device is extended more than 10%, but typically greater than 25%, more typically greater than 50%. Electronics can be provided that are adapted and configured to process a power signal received from the micro-generator. At least some of the electronics may be positioned within the lead and/or the micro-generator. Moreover, the electronics can be adapted and configured to buffer a power signal received from the micro-generator. A storage component can also be provided that is adapted and configured to store energy generated by the micro-generator, either temporarily or for an extended period of time. The micro-generator can further be configured to process, smooth or rectify an oscillating voltage waveform. Additionally, the power wire can be positioned within the central hollow portion of the lead. In some instances, the housing is hermetically sealed. The housing can also be evacuated and sealed. In still other configurations, a secondary housing can be provided. The magnet can also be segmented. The wire can be routed at least one of centrally and alongside the micro-generator module, embedded in the wall of the housing, or routed in a tubing adapted and configured to house one or more wires. Additionally, the micro-generator can also comprise a core, such as a core of ferrite. Configurations with a core can be configured such that the core and the magnet are moveable relative to each other - for example, where both are capable or movement or where one is in a fixed location and the other component moves relative to the fixed component. One or more inductive coils can be positioned around the core. Additionally, the lead can be a power generation lead or a multi-function lead (i.e., a lead capable of power generation and one or more other functions). A generator can be positioned within an implantable medical device. Additionally, one or more rechargeable batteries can be provided. One or more capacitors can be provided that are adapted and configured to at least one of store and buffer power from the micro-generator. Additionally, the capacitors can be adapted and configured to be topped off. An attachment mechanism can also be provided that is adapted and configured to attach the lead to the implantable device. One or more coils and magnets can also be provided that are configured to maximize an integral of the square of the rate of change of flux over time. The micro-generator is used at least one of in parallel or in series with one or more standard or rechargeable batteries. A multiplexer can also be provided that is adapted and configured to multiplex one or more signals along the wires from a tip of the lead to the micro-generator and the implantable control device. Micro-generators will typically range from 7 French to 20 French, more specifically 8 French to 13 French, and even more specifically 9 French to 12 French. A lubricant, coating, film and/or plating can be used on some or all surfaces of the components of the micro-generator. Lubricants include, for example, silicon, carbon, and carbon spheres. Plating is typically nickel or other suitable hard metal. The housing is typically formed from a material having a high transparency to magnetic flux, such as titanium, aluminum, ceramic, and glass. Additionally, the wires are often insulated. In at least some configurations, the spring is connected to the magnet. The one or more magnets are comprised at least two magnets and further wherein the magnets are positioned within the housing such that at least one of a north pole and a south pole of a first magnet is adjacent a corresponding north pole or south pole of a second magnet.

Yet another aspect of the disclosure is directed to a method of generating power for an implantable device. The method comprises: implanting a cardiac rhythm management device in a body cavity of a mammal; advancing a lead from the cardiac rhythm management device containing a micro-generator into at least one of an atrium and a ventricle of a mammalian heart; adhering the lead to an interior surface of the at least one of the atrium and the ventricle; wherein a magnet positioned within a housing in the micro-generator moves along an axis within the housing of the micro-generator with each heart beat; and further wherein energy from the movement of the magnet along an axis is transferred to the cardiac rhythm management device. In some aspects of the method, the method can further comprise the step of inducing a change in a magnetic field of the magnet through a coil to produce electrical energy. Additionally, the method can include one or more of the step of storing the electrical energy in a capacitor, and using the energy to power the cardiac rhythm management device. Suitable cardiac rhythm management devices include, for example, pacemakers, implantable cardioverter defibrillators, cardiac resynchronization therapy devices, and cardiac resynchronization therapy devices with defibrillation. The housing can further comprise gas between at least a portion of the housing and the magnet and the magnet further comprising facets along a portion of its length, the method further comprising the step of moving the gas within the housing along a length of the magnet in a gap between the magnet and an interior housing wall. Additionally, the lead is adhered on an interior wall of the ventricle corresponding to an apex of the heart. In some methods, the method includes varying at least one of gauge of the wire, number of turns of the wire, wire material coatings, nano-coatings and insulation of the coil wires, and/or varying at least one of number of coils, number of magnets, size of magnets, orientation of magnets, in a micro-generator. In some methods, the signal can be multiplexed along the wires. Additionally, varying the strength magnetic material might be desirable in some situations and/or impedance matching a power generation circuit to an optimized power output. The housing can further comprise at least one of a coating, a plating, and a lubricant on at least one of the magnet and micro-generator capsule wall.

Still another aspect of the disclosure is directed to a method for generating power for an implantable device. The method comprises: implanting a neural stimulation device in a body cavity of a mammal; advancing an electrode from the neural stimulation device containing a micro-generator into target mammalian tissue; adhering the electrode to the target mammalian tissue; wherein a magnet positioned within a housing in a micro-generator moves along an axis within the housing of the micro-generator in response to mammalian movement; and further wherein energy from the movement of the magnet along an axis is transferred to the neural stimulation device. Methods can include the step of inducing a change in a magnetic field of the magnet through a coil to produce electrical energy, storing the electrical energy in a capacitor, using the energy to power the neural stimulation device. In some aspects, the neural stimulation device is adapted and configured to stimulate a tissue selected from the group comprising gastric, bone, brain, skin, kidney, liver, pancreas. Furthermore, the housing can further be configured to comprise gas between at least a portion of the housing and the magnet and the magnet further comprising facets along a portion of its length, the method further comprising the step of moving the gas within the housing along a length of the magnet in a gap between the magnet and an interior housing wall. In some configurations, the lead is adhered on an interior wall of the ventricle corresponding to an apex of the heart. In some methods, at least one of gauge of the wire, number of turns of the wire, wire material coatings, nano-coatings and insulation of the coil wires are varied, and/or number of coils, number of magnets in a micro-generator. Additional methods include multiplexing signals along wires, using varying strength magnetic material, impedance matching a power generation circuit to an optimized power output. Additionally, for at least some methods, connecting a cardiac generator device to a neural or tissue stimulation device is performed wherein the cardiac generator is at a first physiological position within a mammalian body and the neural stimulation device is at a second physiological position different than the first physiological position within the mammalian body.

Still another aspect of the disclosure is directed to a method for generating power for an implantable device. The method comprises: implanting a tissue stimulation device in a body cavity of a mammal; advancing an electrode from the tissue stimulation device containing a micro-generator into target mammalian tissue; adhering the electrode to the target mammalian tissue; wherein a magnet positioned within a housing in a micro-generator moves along an axis within the housing of the micro-generator in response to mammalian movement; and further wherein energy from the movement of the magnet along an axis is transferred to the tissue stimulation device. Methods can include the step of inducing a change in a magnetic field of the magnet through a coil to produce electrical energy, storing the electrical energy in a capacitor, using the energy to power the tissue stimulation device. In some aspects, the tissue stimulation device is adapted and configured to stimulate a tissue selected from the group comprising gastric, bone, brain, skin, kidney, liver, pancreas. Furthermore, the housing can further be configured to comprise gas between at least a portion of the housing and the magnet and the magnet further comprising facets along a portion of its length, the method further comprising the step of moving the gas within the housing along a length of the magnet in a gap between the magnet and an interior housing wall. In some configurations, the lead is adhered on an interior wall of the ventricle corresponding to an apex of the heart. In some methods, at least one of gauge of the wire, number of turns of the wire, wire material coatings, nano-coatings and insulation of the coil wires are varied, and/or number of coils, number of magnets in a micro-generator. Additional methods include multiplexing signals along wires, using varying strength magnetic material, impedance matching a power generation circuit to an optimized power output. Additionally, for at least some methods, connecting a cardiac generator device to a neural stimulation device is performed wherein the cardiac generator is at a first physiological position within a mammalian body and the tissue stimulation device is at a second physiological position different than the first physiological position within the mammalian body. Additionally, a neural stimulation device can be used in combination with a tissue stimulation device, as desired.

Yet another aspect of the disclosure is directed to a networked apparatus. The networked apparatus comprises: a memory; a processor; a communicator; a display; and an implantable cardiac rhythm management system further comprising a cardiac rhythm management device, an elongated housing adapted and configured to be positioned distally within a tip of a cardiac lead wherein the housing has an elongated interior cavity, a first end and a second end, one or more longitudinally slidable elongated magnets, one or more coils positioned exteriorly, interiorly or integrally along at least a portion of the housing, a power wire in electrical communication with the one or more coils and with an implantable medical device, wherein the implantable cardiac rhythm management system is adapted and configured to generate energy and communicate the generated energy to the implantable medical device.

Still another aspect of the disclosure is directed to a networked apparatus comprising: a memory; a processor;

a communicator; a display; and a neural stimulation device comprising a power supply, an electrode, a neural stimulator connected to the power supply, the neural stimulator being adapted to generate a neural stimulation signal for delivery to the neural stimulation target through the electrode, a controller connected to the power supply, and further connected to the neural stimulator to control the neural stimulator according to a neural stimulation protocol to deliver a neural stimulation therapy, a pressure sensor electrically connected to the controller; and an implantable micro-generator comprising, an elongated housing adapted and configured to be positioned distally within a tip of an electrode wherein the housing has an elongated interior cavity, a first end and a second end, one or more longitudinally slidable elongated magnets, one or more coils positioned exteriorly, interiorly or integrally along at least a portion of the housing, a power wire in electrical communication with the one or more coils and with the neural stimulation device, wherein the implantable micro-generator is adapted and configured to generate energy and communicate the generated energy to the neural stimulation device.

Additional aspects of the disclosure are directed to a communication system. The communication system comprises: an implantable cardiac rhythm management system further comprising a cardiac rhythm management device, an elongated housing adapted and configured to be positioned distally within a tip of a cardiac lead wherein the housing has an elongated interior cavity, a first end and a second end, one or more longitudinally slidable elongated magnets, one or more coils positioned exteriorly, interiorly or integrally along at least a portion of the housing, a power wire in electrical communication with the one or more coils and with an implantable medical device, wherein the implantable cardiac rhythm management system is adapted and configured to generate energy and communicate the generated energy to the implantable medical device; a server computer system; a measurement module on the server computer system for permitting the transmission of a measurement from the implantable cardiac rhythm management system over a network; at least one of an API engine connected to at least one of the implantable cardiac rhythm management system and the cardiac rhythm management device to create a message about a sensed parameter and transmit the message over an API integrated network to a recipient having a predetermined recipient user name, an SMS engine connected to at least one of the implantable cardiac rhythm management system and the cardiac rhythm management device to create an SMS message about the measurement and transmit the SMS message over a network to a recipient device having a predetermined measurement recipient telephone number, and an email engine connected to at least one of the implantable cardiac rhythm management system and the cardiac rhythm management device to create an email message about the measurement and transmit the email message over the network to a recipient email having a predetermined recipient email address. Additionally, the system further comprising a storing module on the server computer system for storing the measurement on the implantable cardiac rhythm management system server database. At least one of the implantable cardiac rhythm management system and the cardiac rhythm management device can be connectable to the server computer system over at least one of a mobile phone network and an Internet network, and a browser on the measurement recipient electronic device is used to retrieve an interface on the server computer system. Additionally, a plurality of email addresses are held in a implantable cardiac rhythm management system database and fewer than all the email addresses are individually selectable from the diagnostic host computer system, the email message being transmitted to at least one recipient email having at least one selected email address. In at least some configurations of the system at least one of the implantable cardiac rhythm management system and the cardiac rhythm management device is connectable to the server computer system over the Internet, and a browser on the measurement recipient electronic device is used to retrieve an interface on the server computer system. Additionally, a plurality of user names are held in the implantable cardiac rhythm management system database and fewer than all the user names are individually selectable from the diagnostic host computer system, the message being transmitted to at least one measurement recipient user name via an API. Moreover, the measurement recipient electronic device is connectable to the server computer system over the Internet, and a browser on the measurement recipient electronic device is used to retrieve an interface on the server computer system. In some instances, the measurement recipient electronic device is connected to the server computer system over a cellular phone network, for example, where the measurement recipient electronic device is a mobile device. Additional configurations of the system comprise: an interface on the server computer system, the interface being retrievable by an application on the mobile device. The SMS measurement can be received by a message application on the mobile device. A plurality of SMS measurements can be received for the measurement, each by a respective message application on a respective recipient mobile device. At least one SMS engine typically is configured to receive an SMS response over the cellular phone SMS network from the mobile device and stores an SMS response on the server computer system. A measurement recipient phone number ID can also be transmitted with the SMS measurement to the SMS engine and is used by the server computer system to associate the SMS measurement with the SMS response. In some cases, the server computer system is connectable over a cellular phone network to receive a response from the measurement recipient mobile device. The SMS measurement can also include a URL that is selectable at the measurement recipient mobile device to respond from the measurement recipient mobile device to the server computer system, the server computer system utilizing the URL to associate the response with the SMS measurement. Furthermore some configurations of the system can include a downloadable application residing on the measurement recipient mobile device, the downloadable application transmitting the response and a measurement recipient phone number ID over the cellular phone network to the server computer system, the server computer system utilizing the measurement recipient phone number ID to associate the response with the SMS measurement. Other configurations can include a transmissions module that transmits the measurement over a network other than the cellular phone SMS network to a measurement recipient user computer system, in parallel with the measurement that is sent over the cellular phone SMS network. Still other systems can include a downloadable application residing on the measurement recipient host computer, the downloadable application transmitting a response and a measurement recipient phone number ID over the cellular phone network to the server computer system, the server computer system utilizing the measurement recipient phone number ID to associate the response with the SMS measurement.

Still other aspects are directed to a communication system, comprising: a neural stimulation device comprising a power supply, an electrode, a neural stimulator connected to the power supply, the neural stimulator being adapted to generate a neural stimulation signal for delivery to the neural stimulation target through the electrode, a controller connected to the power supply, and further connected to the neural stimulator to control the neural stimulator according to a neural stimulation protocol to deliver a neural stimulation therapy, a pressure sensor electrically connected to the controller; and an implantable micro-generator comprising, an elongated housing adapted and configured to be positioned distally within a tip of an electrode wherein the housing has an elongated interior cavity, a first end and a second end, one or more longitudinally slidable elongated magnets, one or more coils positioned exteriorly, interiorly or integrally along at least a portion of the housing, a power wire in electrical communication with the one or more coils and with the neural stimulation device, wherein the implantable micro-generator is adapted and configured to generate energy and communicate the generated energy to the neural stimulation device; a server computer system; a measurement module on the server computer system for permitting the transmission of a measurement from the implantable neural stimulation device over a network; at least one of an API engine connected to at least one of the neural stimulation system and the cardiac rhythm management device to create a message about a sensed parameter and transmit the message over an API integrated network to a recipient having a predetermined recipient user name, an SMS engine connected to at least one of the neural stimulation system and the cardiac rhythm management device to create an SMS message about the measurement and transmit the SMS message over a network to a recipient device having a predetermined measurement recipient telephone number, and an email engine connected to at least one of the neural stimulation system and the cardiac rhythm management device to create an email message about the measurement and transmit the email message over the network to a recipient email having a predetermined recipient email address. The system can further comprise a storing module on the server computer system for storing the measurement on the neural stimulation system server database. At least one of the neural stimulation system and the cardiac rhythm management device can further be connectable to the server computer system over at least one of a mobile phone network and an Internet network, and a browser on the measurement recipient electronic device is used to retrieve an interface on the server computer system. A plurality of email addresses can be held in a neural stimulation system database and fewer than all the email addresses are individually selectable from the diagnostic host computer system, the email message being transmitted to at least one recipient email having at least one selected email address. Moreover, at least one of the neural stimulation system and the cardiac rhythm management device is connectable to the server computer system over the Internet, and a browser on the measurement recipient electronic device is used to retrieve an interface on the server computer system. In some configurations a plurality of user names are held in the neural stimulation system database and fewer than all the user names are individually selectable from the diagnostic host computer system, the message being transmitted to at least one measurement recipient user name via an API. Additionally, the measurement recipient electronic device is connectable to the server computer system over the Internet, and a browser on the measurement recipient electronic device is used to retrieve an interface on the server computer system. In at least some instances, the measurement recipient electronic device is connected to the server computer system over a cellular phone network, for example where the measurement recipient electronic device is a mobile device. Additionally, an interface can be provided on the server computer system, the interface being retrievable by an application on the mobile device. The SMS measurement can be received by a message application on the mobile device. Additionally, a plurality of SMS measurements can be received for the measurement, each by a respective message application on a respective recipient mobile device. In some configurations, the at least one SMS engine receives an SMS response over the cellular phone SMS network from the mobile device and stores an SMS response on the server computer system. A measurement recipient phone number ID is transmitted with the SMS measurement to the SMS engine and is used by the server computer system to associate the SMS measurement with the SMS response. Additionally, the server computer system can be configured such that it is connectable over a cellular phone network to receive a response from the measurement recipient mobile device. The SMS measurement can also be configured to include a URL that is selectable at the measurement recipient mobile device to respond from the measurement recipient mobile device to the server computer system, the server computer system utilizing the URL to associate the response with the SMS measurement. In some configurations, the system includes at least one of a downloadable application residing on the measurement recipient mobile device, the downloadable application transmitting the response and a measurement recipient phone number ID over the cellular phone network to the server computer system, the server computer system utilizing the measurement recipient phone number ID to associate the response with the SMS measurement and a transmissions module that transmits the measurement over a network other than the cellular phone SMS network to a measurement recipient user computer system, in parallel with the measurement that is sent over the cellular phone SMS network. Other configurations can include a downloadable application residing on the measurement recipient host computer, the downloadable application transmitting a response and a measurement recipient phone number ID over the cellular phone network to the server computer system, the server computer system utilizing the measurement recipient phone number ID to associate the response with the SMS measurement.

Still other aspects of the disclosure are directed to kits for managing a cardiac rhythm. The kits comprise: a cardiac rhythm management device; one or more micro-generator devices comprising an elongated housings adapted and configured to be in communication with the cardiac rhythm management device wherein the housing has an elongated interior cavity, a first end and a second end; a longitudinally slidable elongated magnet having a longitudinal length greater than a cross-sectional diameter positioned within the interior cavity of the elongated housing; a coil along at least a portion of the housing; a power wire in electrical communication with the coil and with the cardiac rhythm management device; wherein the micro-generator is adapted and configured to generate energy and communicate the generated energy to the cardiac rhythm management device. The kits may also include one or more of each of the following: scissors, scalpels, staples, sutures, electrocautery, needles, syringes, clips, betadine, tissue preparation material, and trays.

Yet another aspect is directed to a kit for neural stimulation. The kits comprise: a neural stimulation device; one or more micro-generator devices comprising an elongated housings adapted and configured to be in communication with the neural stimulation device wherein the housing has an elongated interior cavity, a first end and a second end; a longitudinally slidable elongated magnet having a longitudinal length greater than a cross-sectional diameter positioned within the interior cavity of the elongated housing; a coil along at least a portion of the housing; a power wire in electrical communication with the coil and with the neural stimulation device; wherein the micro-generator is adapted and configured to generate energy and communicate the generated energy to the neural stimulation device. The kits can also be configured to include one or more of each of the following: scissors, scalpels, staples, sutures, electrocautery, needles, syringes, clips, betadine, tissue preparation material, and trays.

Still another aspect of the disclosure is directed to a lead delivery system. The lead delivery system comprises: a stylet wire; an introducer catheter dimensioned to received a cardiac lead therethrough and having an internal stylet lumen dimensioned to receive the stylet wire; a cardiac lead containing one or more elongated housings adapted and configured to be positioned within a distal tip of the cardiac lead wherein the housing has an elongated interior cavity, a first end and a second end; a longitudinally slidable elongated magnet having a longitudinal length greater than a cross-sectional diameter positioned within the interior cavity of the elongated housing; a coil positioned exteriorly along at least a portion of the housing; a power wire in electrical communication with the coil and with an implantable medical device; wherein the implantable micro-generator is adapted and configured to generate energy and communicate the generated energy to the implantable medical device. The system can further comprise an internal stylet lumen adapted and configured to be collapsible upon removal of the stylet wire.

Yet another aspect of the disclosure is directed to a method for implanting a cardiac lead. The method includes the steps of (a) introducing a sheath, (b) steering the sheath to a target location within a mammalian heart, (c) advancing the cardiac lead containing one or more elongated housings adapted and configured to be positioned within a distal tip of the cardiac lead wherein the housing has an elongated interior cavity, a first end and a second end; a longitudinally slidable elongated magnet having a longitudinal length greater than a cross-sectional diameter positioned within the interior cavity of the elongated housing; a coil positioned exteriorly or interiorly along at least a portion of the housing; a power wire in electrical communication with the coil and with an implantable medical device through a lumen of the sheath to a target location within the mammalian heart, (d) identifying the target location with the lead, (e) coupling the lead to cardiac tissue at the posterior summit or side wall of at least one of the left ventricular an left atrium at a first end, and (f) removing the sheath.

Still another aspect of the disclosure is directed to a method for implanting a cardiac lead. The method includes the steps of: (a) advancing a cardiac lead containing one or more elongated housings adapted and configured to be positioned within a distal tip of the cardiac lead wherein the housing has an elongated interior cavity, a first end and a second end, a longitudinally slidable elongated magnet having a longitudinal length greater than a cross-sectional diameter positioned within the interior cavity of the elongated housing; a coil positioned exteriorly or interiorly along at least a portion of the housing; a power wire in electrical communication with the coil and with an implantable medical device through a lumen of the sheath to a target location within the mammalian heart, (b) identifying the target location with the lead, and (c) coupling the lead to cardiac tissue at the posterior summit or side wall of at least one of the left ventricular an left atrium at a first end.

## BRIEF DESCRIPTION OF THE DRAWINGS

The novel features of the invention are set forth with particularity in the appended claims. A better understanding of the features and advantages of the present invention will be obtained by reference to the following detailed description that sets forth illustrative embodiments, in which the principles of the invention are utilized, and the accompanying drawings of which:

**FIG. 1A** illustrates a cross-sectional view of the normal ventricular conduction mechanism;

**FIG. 1B** illustrates a postero inferior view of the heart;

**FIG. 1C** is a chart that illustrates patient survival by ejection fraction vs. the service life of an ICD;

**FIG. 2A** illustrates a chest cavity with a single chamber CRM device therein, along with a micro-generator containing a lead positioned relative to the heart; **FIG.2B** illustrates an ICD routed to the heart;

**FIGS.3A-B** illustrate ends of a cardiac lead that is insertable into, and attachable to the inside wall of the heart;

**FIG. 4A** illustrates a lengthwise cross-section of the CRM lead containing the micro-generator device; **FIGS.** 4B-D illustrate a cross section of the lead and micro-generator device at B, C, D in **FIG. 4A;**

**FIGS. 5A-D** illustrate an external view and a lengthwise cross-section of the micro- generator portion only;

**FIG. 6** illustrates a typical ICD or other CRM implantable control device and a section of lead and wires, with sensing, pacing and signal control provided by a controller;

**FIG. 7A-C** illustrates a micro-generator capsule tube with wire coils for inductive power generation wrapped around it with about 4mm outer diameter;

**FIG. 8A** illustrates the peak voltage and power generation results of tests done with one configuration of device;

**FIG. 8B** illustrates the output voltage vs. time curve for a single oscillation of the magnet inside the coil;

**FIG. 9** illustrates a configuration of a magnet positioned within a housing having a coil and elastic bumpers as end stops;

**FIG. 10** illustrates a configuration in which elastic springs are used as end stops;

**FIG. 11A** illustrates an alternative configuration in which a moving magnet is suspended linearly between the two ends of a micro-generator capsule by springs;

**FIG. 11B** illustrates an alternative configuration in which a moving magnet having a north and a south pole is suspended between two magnets positioned at either end of a micro-generator capsule;

**FIG. 12** illustrates a sealed generator;

**FIG. 13** illustrates a configuration where the non-biocompatible wire power generation induction coils are positioned on the outside surface of the micro-generator, are sealed to prevent contact with bodily fluids, and are attached to biocompatible wires that pass outside the sealed area into the lead;

**FIGS. 14A-B** illustrates a micro-generator capsule and induction coils contained inside another sealed capsule, with sealed feed-throughs to the biocompatible main wires inside the lead, and an attachment point joining these to the non-biocompatible coil wires;

**FIG. 15** illustrates a configuration wherein the induction coils are wrapped inside the micro-generator module walls, and are fully contained within the sealed capsule, with connection to biocompatible wires that exit through feed-throughs to the lead;

**FIGS. 16A-D** illustrations variations of cross-sectional shapes as well as edge shapes of the ends of the magnet, employable in the various designs of the micro-generators;

**FIGS. 17A-B** illustrate a micro-generator module consisting of multiple short magnets to allow flexibility of the module;

**FIGS. 17C-D** illustrate a flexible magnet wherein the magnet is made of a flexible polymeric or other material allowing it to bend when needed

**FIGS. 18A-B** illustrate a mechanism for passing one or more additional wires past a micro-generator module by embedding the wire in the capsule wall;

**FIGS. 19A-C** illustrate a region of a cardiac lead near the tip which includes an asymmetrical bulge in the wall of the lead to allow space for sensing or pacing wires;

**FIGS. 20A-C** illustrate a variant in which the lead wall not only has a bulge, but a separate tubing compartment with a space separating the lead section containing the micro-generator;

**FIGS. 21A-B** illustrate two variants of using the conduction of the magnet and end springs or wires to pass the signal wire signal;

**FIGS. 22A-B** illustrate a mechanism for passing signal wire signals along the micro-generator module surface without increasing or significantly increasing the diameter of the micro-generator module or the cardiac lead;

**FIGS. 23A-B** illustrate an approach for routing signal or other wires through a center tube in the micro-generator module itself;

**FIG. 24A** illustrates an approach for avoiding the need for a separate self-contained micro-generator module; **FIG. 24B** illustrates an approach for embedding the coil wires within the walls of the self contained micro-generator module itself

FIG. 25 illustrates an optimal placement of the micro-generator module in the cardiac lead as a tradeoff between distance from the lead tip and transferring the maximum possible mechanical energy from the heart's beat along the lead walls;

FIG. 26A-C illustrate an approach in which the micro-generator module is a distinct section of the cardiac lead;

FIG. 27 illustrates the use of multiple micro-generator modules in a cardiac lead;

FIG. 28 illustrates a micro-generator module configured so that it has two (or more) separate inductive coils;

FIGS. 29A-C illustrate energy harvesters which can be improved by arranging one or more permeable cores to collect a maximum of flux from the moving magnet(s);

FIGS. 30A-D illustrate a coil and magnet arrangement configured to maximize power output and minimize the number of voltage sign changes

FIG. 31 illustrates a stylet for guiding the lead with micro-generator into place, and a stylet lumen for mating with the stylet;

FIG. 32 illustrates a coil and magnet configuration having three magnets and three coils; and

FIG. 33A is a block diagram illustrating a representative example of a logic device through which dynamic a modular and scalable system can be achieved; and FIG. 33B is a block diagram illustrating the cooperation of exemplary components of a system suitable for use in a system where dynamic data analysis and modeling is achieved.

## DETAILED DESCRIPTION OF THE INVENTION

For purposes of illustration of the inventive concepts contained herein, the disclosure is described in terms of a cardiac rhythm management device used in a mammalian heart. However, as will be appreciated by those skilled in the art, the power generation concepts can be applied to other implantable devices having power needs including, but not limited to, gastric devices, neurostimulation devices (shown to be effective for reducing the symptoms of Parkinson's Disease and alleviating some types of chronic pain), tissue stimulation devices (e.g., used in conjunction with controlling food intake, tissue growth, bone growth, etc.). See, for example, U.S. Patent Nos. 7,616,990 for Implantable and rechargeable neural stimulator; 7,580,753 for Method and system for stimulating a dorsal root ganglion; 7,555,344 for Selective neurostimulation for treating epilepsy; 7,346,382 for Brain stimulation models, systems, devices, and methods; 7,494,459 for Sensor-equipped and algorithm-controlled direct mechanical ventricular assist device; and 5,941,906 for Implantable, modular tissue stimulator.

Generally speaking, micro-generators of this disclosure have an expected power output from 5uW to 100uW, and more particularly 50uW to 100uW. However, as will be appreciated by those skilled in the art, the size and configurations of the micro-generators can be varied from the examples provided below to optimize the power generated for a particular use. Additionally, the size and configuration can be changed to accommodate particular anatomical considerations of the application of the micro-generator. Such changes are within the scope of this disclosure.

## I. ANATOMICAL REVIEW

In order to appreciate the novelty and usefulness of the devices, methods, systems and kits described herein, it is important to understand the basics of the human conduction system of the heart **10**. The normal human conduction system carries an impulse from the atria **20, 24** to the ventricles **22, 26** and distributes the electrical impulse very efficiently so that the entire ventricle is electrically activated in less than 100 milliseconds. This permits effective ventricular contraction. In contrast, RV apex pacing activates the heart **10** in 150-200 or more milliseconds. This longer time results in a less synchronous ventricular contraction and often to lower cardiac output and the other complications described above.

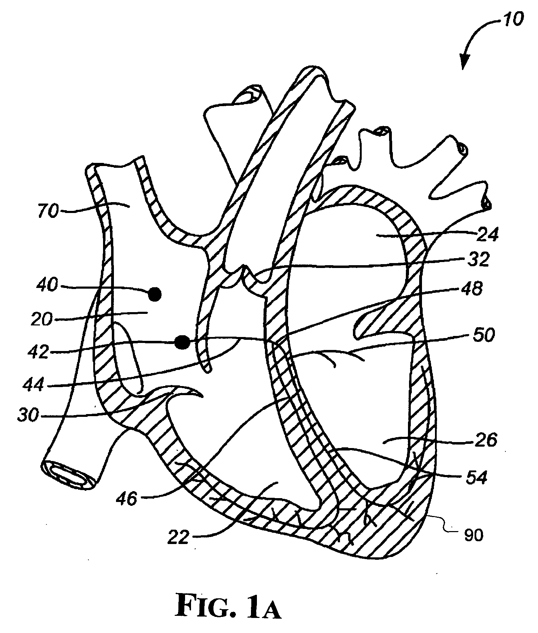

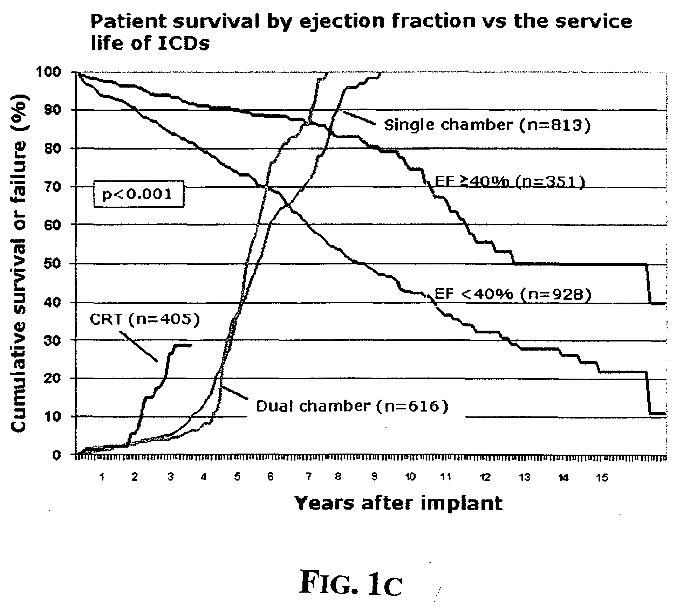

FIG. 1A depicts the normal ventricular conduction mechanism of a heart **10.** The normal ventricular conduction mechanism starts with a bridge from the atrium to the ventricles called the atrioventricular node (AV node) **42.** The AV node **42** is activated by the sinu-atrial node (SA node) **40.** Once an impulse passes through the AV node **42,** the impulse then passes through the bundle of His **44,** which is at the base of the ventricles **22, 26.** Thereafter, the conduction system divides into a left main branch **48** and right main branch **46.** The left branch **48,** which activates the left ventricle **26,** almost immediately divides into a small anterior branch **50** and a much larger posterior branch of the left main branch **54** that swings around the left ventricle **26** and basically surrounds the posterior mitral annulus (not shown) before it spreads out over the ventricles **22, 26.** This important left posterior branch **54** has not been well understood until recently. The posterior branch **54** activates the left ventricle summit early in systole and starts the process by which the mitral valve **30** closes.

As it turns out, the heart **10** as a pump cannot generate much force until the mitral valve **30** is closed and isovolumic systole can begin. At that point, the heart **10** can generate force because the blood inside it is trapped until the pressure inside that chamber exceeds that of aortic pressure at which point the blood is ejected from the ventricle into the aorta.

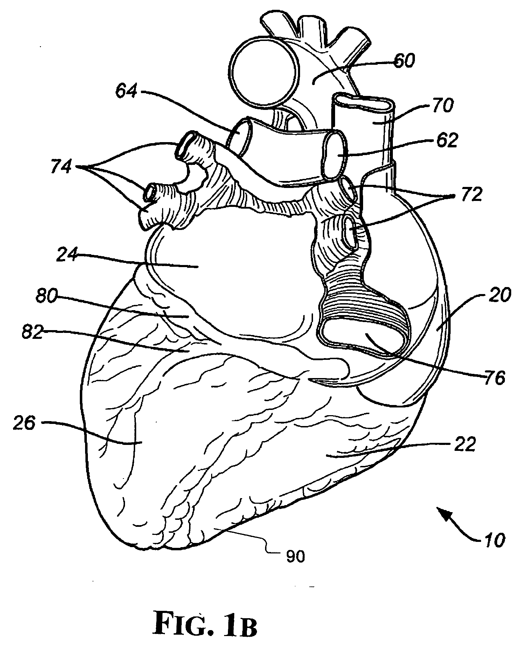

**FIG.1B** depicts a posteroinferior view of the heart **10,** showing the relative positions of the aortic arch **60,** the left pulmonary artery **64,** the right pulmonary artery **62,** the left pulmonary veins **74,** the right pulmonary veins **72,** the left atrium **24,** the coronary sinus **80,** a branch of the coronary sinus **82,** the left ventricle **26,** the right atrium **20,** the right ventricle **22,** the superior vena cava **70,** and the inferior vena cava **76.** If the summit of the left ventricle **26** is not activated early, the mitral valve **30** leaks and the heart cannot generate as much force. A lower cardiac output ensues.

In order to understand the configurability, adaptability and operational aspects of the devices, it is helpful to understand the anatomical references of the body with respect to which the position and operation of the devices, and components thereof, are described. There are three anatomical planes generally used in anatomy to describe the human body and structure within the human body: the axial plane, the sagittal plane and the coronal plane. Additionally, devices and the operation of devices are better understood with respect to the caudad (towards the feet) direction and/or the cephalad direction (towards the head). Devices positioned within the body can be positioned dorsally (or posteriorly) such that the placement or operation of the device is toward the back or rear of the mammalian body. Alternatively, devices can be positioned ventrally (or anteriorly) such that the placement or operation of the device is toward the front of the mammalian body. Various embodiments of the devices and systems of the present disclosure may be configurable and variable with respect to a single anatomical plane or with respect to two or more anatomical planes. For example, a component may be described as lying within and having adaptability in relation to a single plane. Additionally, the various components can incorporate differing sizes and/or shapes in order to accommodate differing patient sizes and/or anticipated loads.

## II. DEVICES

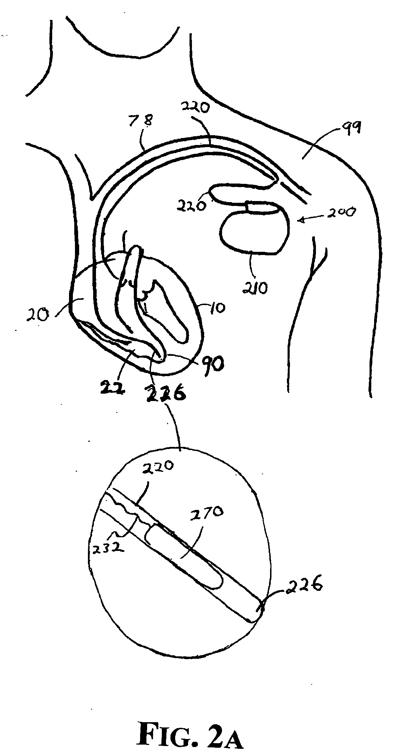

**FIG. 2A** illustrates the placement of a standard single or dual-chamber cardiac rhythm management (CRM) device **200.** The power and control implantable control device **210** is surgically embedded under the mammalian skin of the patient, typically near the patient's left clavicle **99.** A lead **220** consisting of a hollow tube adapted and configured to contain a plurality of control and signal wires is fed through the subclavian vein **78** through the right atrium **20** to the right ventricle **22,** and the tip **226** of the lead **220** is attached to the heart **10** at a position within an interior wall of the ventricle at a position near the apex **90** of the ventricle. The apex of the heart is the lowest supeficial (exterior) part of the heart and is typically directed downward (caudal), ventrally (forward), and to the left.

In a one lead device, the lead **220** will be implanted in the right ventricle **22** and secured to the inner chamber wall of the ventricle. However, a plurality of lead devices can use both ventricles **22, 26** or atria **20, 24** of the heart **10.**

The tip **226** of the lead **220** containing the micro-generator device **270** may be placed in a number of sites within a ventricle **22, 26** or atrium **20, 24.** The micro-generator is configured to convert mechanical energy into electrical energy. The site may be selected to optimize the range and velocity of motion as well as orientation relative to the heart's motion. An orientation of the lead tip **226** generator device **270** that is orthogonal to the heart wall's motion is optimized for transmitting kinetic energy to the micro-generator device. Based on analysis of computerized tomography scans of patients in heart failure, it is estimated that there is a heart displacement of approximately 18mm at the apex in a diseased heart. The apex **90** is also a desirable location because the walls of the ventricle at the apex will tend to help keep the lead **220** and generator **270** aligned with an axis that is optimized for transmitting motion at the apex **90.** However, numerous locations for attachment on the side walls of the ventricle **22, 26** may provide advantages in energy or range of motion available, and may also be successfully employed without departing from the scope of the disclosure.

The micro-generator device **270** is positioned in the lead **220** near the tip **226** where attachment to the heart **10** occurs. As the heart **10** beats, motion of the heart **10** shakes the micro-generator module **270,** causing a magnet positioned within the module to move or slide along a single axis through a coil, in the simplest embodiment. Magnet and coil configurations are discussed in more detail below. Energy is thus generated by the movement of the magnet, as shown and described more fully below, and energy is then returned through electrical wires **232** inside the lead **220** back to an implantable control device housing **210** that has been adapted and configured to receive energy from the micro-generator device **270** and store or otherwise process the energy received from the micro-generator device **270** for use to power the implantable device **200.**

As will be appreciated by those skilled in the art, a dual lead CRM device **200** could be configured with more than one generator. For example, generators could be positioned in both leads, thus increasing the rate of power generation.

Positioning the micro-generator **270** nearer the tip **226** of the lead **220** (e.g., closer to the attachment point of the heart **10** and distal from the implanted device **200**) should provide better transfer of mechanical energy from the heart **10** to the micro-generator **270.** However, as will be appreciated by those skilled in the art, positioning one or more micro-generators **270** near the tip of the lead **220** may stiffen the tip thus impairing or impacting maneuverability as the lead **220** is inserted in the heart **10.** Thus it will be possible for the micro-generator **270** to be placed some distance from the tip **226** to enable easier guidance on insertion and still transfer motion to the micro-generator. Moreover, the micro-generator may be positioned away from the tip to improve maneuverability and then repositioned after the tip has reached its final location.

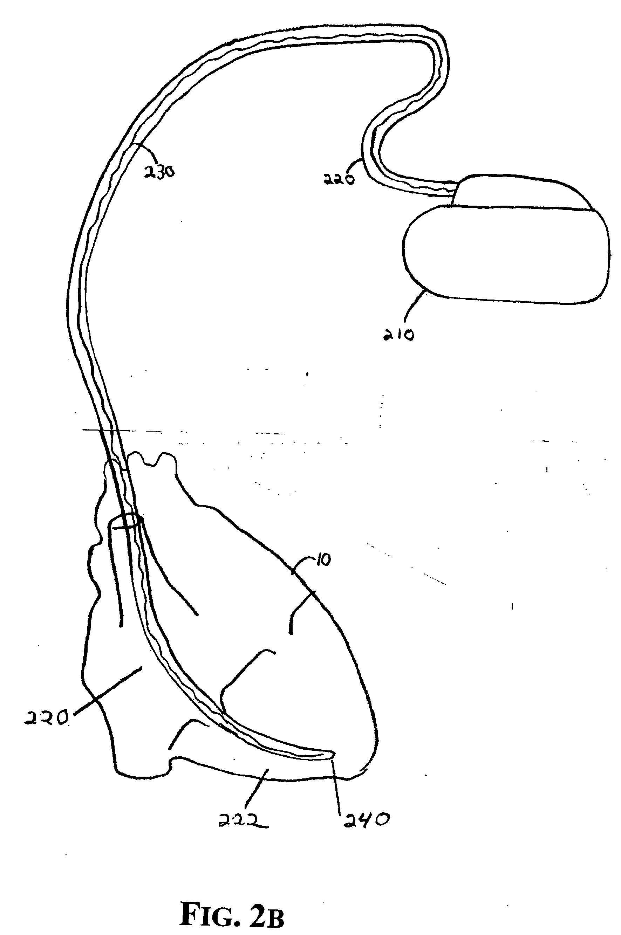

FIG. 2_B illustrates the control housing device 210 of a standard ICD or pacemaker in the tip of the lead 220 positioned within the heart 10. The control housing device 210 has one or more signal or shock wires 230 which travel to the contacts 240 at the tip of the lead.

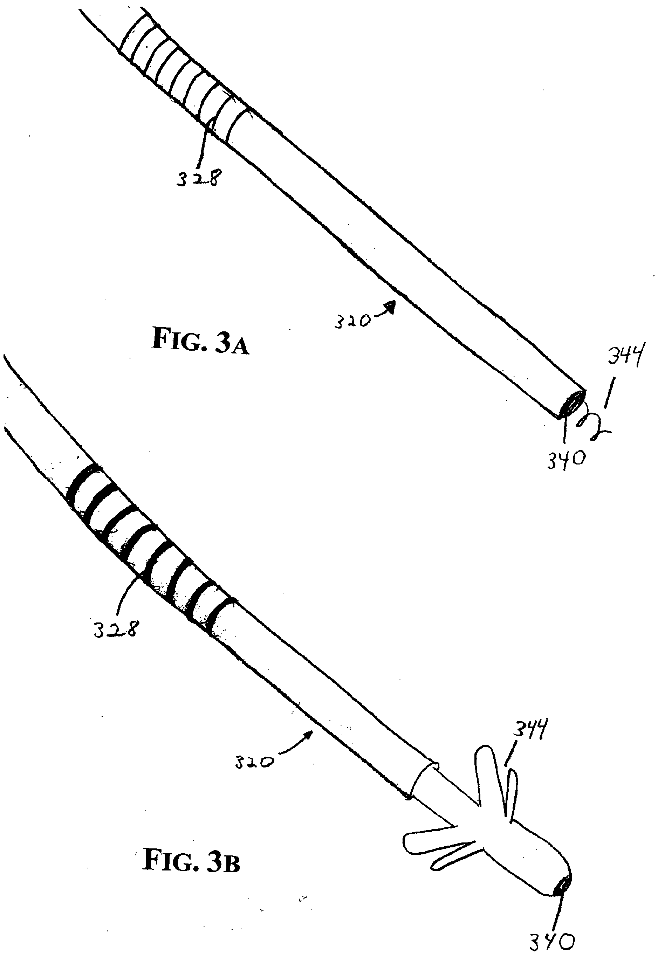

FIGS. 3A-B illustrate possible configurations of an end of a cardiac lead 320 adapted and configured for insertion into and attachment to the inside wall of the heart 10. In an ICD or combined ICD/ pacing lead, the walls of the lead 320 have wire in coils 328, forming a shock coil, or other arrangement to create maximum surface area and contact with the inside of the heart 10 to deliver the defibrillation shock and improve efficacy of the delivered shock. Moreover, the leads 320 have attachment features 344, for example springs or tines that facilitate attachment of the lead 320 to the wall of the heart. The heart encapsulates these attachment features over time, forming a nearly permanent attachment to the lead tip 326. The leads 320 may also have sensing, pacing etc. contacts 340 at or near the tip 326. As will be appreciated by those skilled in the art, coils adapted and configured to deliver a therapeutic shock to heart tissue should not come in contact with power generator coils of the micro-generators described herein. The designs are optimized to ensure that these two coils, or functional components, do not come into contact.

Standard ICDs without power generation capability typically use a standard connector configuration to attach the cardiac lead with defibrillation wires to the implantable control device. This allows interchangeability of leads and controllers so that, for example, a new controller replacement could be surgically implanted (with fresh batteries, features, or software upgrades) and still be attached to an existing implanted standard cardiac lead already in place. Recently a new International Organization for Standardization (ISO) standard was drafted (IS-4) for compatibility. It is contemplated, therefore, that the connection system will include power leads that correspond to the power generator's function to deliver power to the implantable control device. This extra functionality could be built into an IS-4 compatible connector. Alternatively, the lead and control device could have separate connections, for example where one is US-4 (or SJ4) compatible and a second that delivers the power from the micro-generator to the control device. In this way, the lead could then be backward compatible to allow future connection to a standard ICD or CRT-D control device. Any unused power wires could be capped or otherwise suitably isolated from body contact. This would allow compatibility and similarly, this approach could allow for future implantation of a control device containing, for example, the power management circuitry of this invention, which could be used (without power generation capability) with an existing standard lead. Thus, implementation can be optimized for power generation and use features or flexibility and interchangeability.

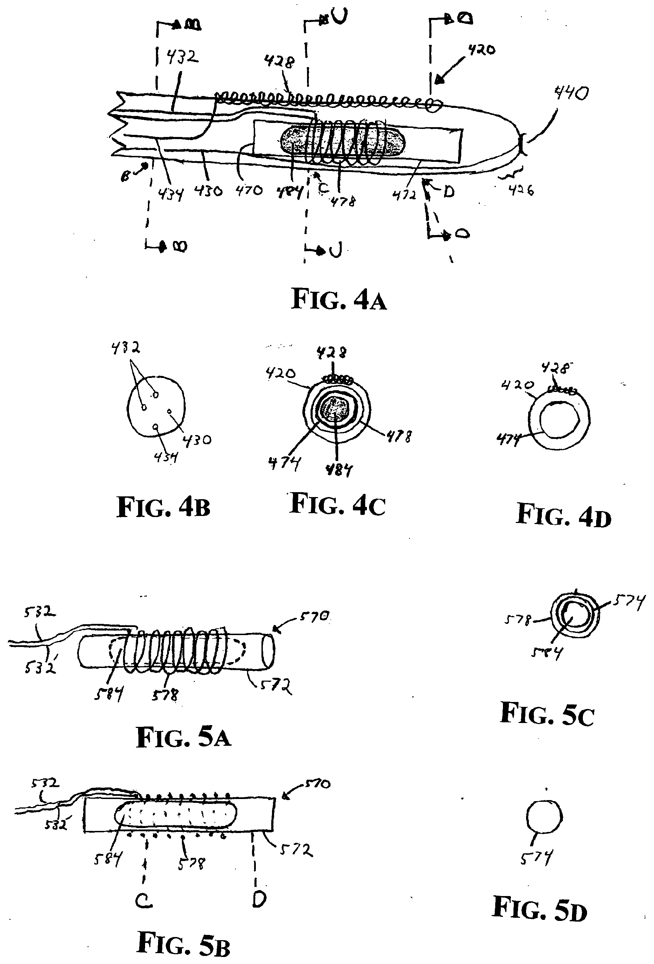

FIGS. 4A-D show cross-sections of a CRM lead 420 containing an examplar micro-generator device. The lengthwise view in FIG. 4A shows the region near the tip 426 of the cardiac lead 420. The lead 420 is typically a cylindrical tube with a nominal diameter around 3-4 mm in a modern ICD. This typically plastic tube is wrapped on the outer surface with contact wire 428. This biocompatible contact wire 428 is often designed with loops or other design configurations adapted and configured to increase effective surface area and promote better wire 428 contact with the inside heart wall. This provides the greatest possible flow of energy to the heart during, for example, a defibrillation event. The wire 434 supplying power to this contact wire passes through the center of the hollow lead 420 to the implantable control device (shown, e.g., in FIG. 2).

In a normal ICD lead, the tip 426 of the lead 420 provides an attachment mechanism (plastic fingers or other approach) that embeds in the heart wall, and becomes essentially permanently attached as the tissue surrounds it over time (see FIG. 3B). This provides excellent coupling of the lead 420 to the heart wall, which is beneficial for transferring mechanical motion to the micro-generator device 470. It may also have one or more sensors adapted and configured to detect, for example, a pulse signal. The resulting signal is then passed by wire 430 up the center of the hollow lead 420 to the implantable control device.

The lead 420 houses the micro-generator device 470. The micro-generator typically comprises a cylindrical capsule, or housing, forming an outer shell, which may be sealed to prevent ingress of outside moisture into the housing and egress of material within the housing to the environment. It may also be transparent or nearly transparent to magnetic flux, and may be made of any suitable material including, for example, aluminum, ceramic, glass, titanium or any other suitable non-conductive material. Positioned within the housing 472 of the micro-generator 470 is one or more magnets 484 which is able to move longitudinally through the housing 472. One or more coils 478 of wire (number of turns, gauge, etc. optimized for achieving desired power generation) are wrapped around the capsule cylinder 472. The wire forming a particular coil 478 may be insulated or otherwise treated to prevent contact between adjacent coils. The ends of a coil wire 432 travel within the lead 420 toward the control housing (typically in a cephalad direction for a standard implantation from the apex of the heart to the clavicle implantation site of the CRM control device), where energy generated by the micro-generator 470 is processed and stored or used directly to power the implanted device.

Although the micro-generator will be optimized to maximize power generation, suitable configurations will be constrained by dimensional limitations imposed by anatomy. For example, length and ability to steer through the tortuous vasculature, as well as diameter and ability to fit through a vein will be important considerations to the optimization of designs. Additional limitations may be placed on the diameter of the devices if the micro-generator is placed inside a CRM lead without increasing the lead diameter, which is typically 4mm (12 French). Micro-generators can be 7 French to 20 French, more typically 8 French to 13 French, and more typically still 9 French to 12 French.

To those skilled in the art, the more coil turns provided (through multiple layers, greater length of coil region, finer gauge wire, etc.) which are then cut by magnetic flux lines, use of more conductive wire material, along with a larger magnet diameter, magnet length and flux strength, will provide greater induced voltage in the coil wire. All of these characteristics will tend to increase a generator's power output. Thus, a clear tradeoff exists when one or more dimensions are constrained. Thus, individual designs will necessarily balance the number of turns that can fit into a given coil length and diameter and coil wire gauge. Similarly, for a given coil design, there will be an attendant physical limitation on the size of the magnet that it can accommodate. Optimal designs will be derived from maximizing these parameters within the physiological and dimensional limitations. Thus a wide range of possible combinations of parameters is possible without departing from the scope of the disclosure.

The magnet **484** is shown in the center of the cylindrical micro-generator housing **472.** Surrounding the cylinder are the coils of fine gauge wire in which a voltage is induced by the moving magnet. And surrounding this entire micro-generator assembly are the walls of the plastic device lead **420,** which may contain contact wires if the device is an ICD. The drawing shows these in a common tightly looped configuration **428,** while **FIG. 3** illustrates these shock coils **328** (in this case flat or round wire) wrapped around the outer surface of the lead **420.**

As is well known in the art, the strongest available permanent magnets consist largely of neodymium (Nd), a rare earth metal with an atomic number of 60. Since Nd is a brittle, slightly toxic metal that easily corrodes in air, commercial magnets often are coated with nickel, another familiar magnetic metal, which is less likely to chip or corrode. In many cases magnets are typically made of an alloy of neodymium, iron, and boron. Alloys of different elements make stronger, longer-lasting magnets because pure magnetic materials usually demagnetize quickly. The reason is that the magnetic forces favor breaking up the domains whose magnetization point different ways and cancel out. When there are sufficient impurities in the material, the boundaries between the domains get stuck, keeping most of the domains from losing their alignment. Consequently, permanent magnets are often made of alloys like AlNiCo wherein one of the components is not magnetic.

As the magnetic flux lines of the moving magnet **484** oscillating through the length of the micro-generator **470** cut the coils of wire **478,** a voltage is created inductively, proportional to the rate of change of magnetic flux. This induced voltage will cause a current flow if a load is connected across the wires, such as to charge or otherwise store or use energy in the implantable control device.

Coils and magnet are arranged to maximize the integral of the square of the rate of change of flux over time. The coils **478** positioned around the magnet **484** are in communication with, or connected to, the power wires **432** which transmit the power generated to the control device housing. Signal, control and/or pacing wires **430** travel from the ICD through the tip **426** of the lead and communicate with a sensor or contact **440.** The looped wire **486** or surface wire **328** is in communication with or connected to the shock delivery wire **434** which is connected to the ICD control housing.

The complete micro-generator consists of the housing shell or case, the interior magnet, the wire coils that generate the voltage, and the wires that carry the generated power from the micro-generator **470** to the implantable control device..Additional configurations and features of the micro-generator **470** are described elsewhere in this disclosure.

**FIGS. 5A-D** show external views and a lengthwise cross-section of the micro-generator device **570** portion only. The capsule **572** contains a magnet **584,** and coils **578.** In the cross-section of **FIGS. 5B,** only the end view of these coils rings **578** is shown, as they have been sliced through. The cross-section of the magnet **584** and coil section shows the capsule wall **574,** surrounded by the coils **578,** and in the center the magnet **584.** The coils **578** are in communication with power wires **532, 532'** which transmit power generated by the magnet **584** moving through the coils **578** to the implantable control device.

## A. ELECTRONICS

Unlike existing CRM devices, the micro-generator device system's implantable control device will include additional circuitry and components designed to receive power back from the micro-generator and store and/or manage it. These functions may include the ability to rectify the oscillating output waveform of the micro-generator module, storing energy for future use in rechargeable batteries or capacitors, processing the energy so that it is immediately available to power the implantable control device's functions, etc.

Numerous different approaches can be envisioned to someone skilled in the art. One approach would be to use the micro-generator to constantly top off large super-capacitors. However, normal leakage from these may make this approach wasteful. Another approach is rechargeable batteries. Rechargeable batteries have lower power density than, for example, Silver Vanadium Oxide (SVO) or other standard primary batteries used in these devices today, and thus may waste space. They may also have reduced lifespan in terms of charging cycles and deterioration. In addition, they may not have the desired high amperage capability needed to charge capacitors fully and repeatedly during a defibrillation event, compared to SVO. One preferred approach is to employ a combination of a standard primary SVO or similar battery, and smaller capacitors or other storage to buffer and store energy provided by the micro-generator. In this approach, the primary SVO battery would power the defibrillation event and any other high-current requirements only, such as periodic Capacitor Reform, Telemetry, and Diagnostics. Other rectifying, storage and buffering circuitry would manage receiving, processing, and storing the output of the micro-generator, and supply the normal background activities such as Sensing, Standby Power, Housekeeping, Condition Monitoring, etc. It could also power the requirements of normal cardiac pacing which requires less power than defibrillation.

The micro-generator delivers its power to the implantable control device through two additional wires, which will run from the micro-generator to the implantable control device through a hollow lead.

One of the benefits of the designs and configurations disclosed herein is that fine wires are used to run power from the micro-generator to the implantable control device. In addition, specially designed local microcircuitry may be scaled to fit within the cardiac lead and sealed to prevent interference from moisture. This circuitry can be used to rectify or process the power generated by the micro-generator locally or even to buffer or store some of the power to provide a short return path for powering, for example, the pacing function using a control signal from the controller. The local microcircuitry may be configured to perform pre-processing of the power output prior to sending the power signal to the CRM control device.

Since space is at a premium within the cardiac lead, many of the approaches discussed herein are optimized to minimize any increase in diameter that may result from running multiple signal and power wires through the lead and past the micro-generator, e.g. positioning the power wires within the central hollow portion of the lead. As will be appreciated by those skilled in the art, signals and power for any of these configurations may also be multiplexed to and from the implantable control device. Where multiplexed signals are used, a single or smaller number of wires can be used to carry multiple types of signals which are then sorted out by the control device and possibly local microcircuitry to determine the type of signal and/or the purpose of the signal.

The inclusion of a micro-generator may hinder steering somewhat when the lead is being inserted into the heart. Several alternative configurations are possible to manage that issue. For example, a steerable stylet could be designed into the lead tip to assist guiding the lead around bends in the internal of veins and heart when placing the lead. The micro-generator could also be attached epicardially as well, which would significantly reduce the design limitations on size and shape, since the device could occupy more space in the thoracic cavity. The generator could also be configured to wrap around the heart like a band, thereby using the motion of the heart to move the generator.

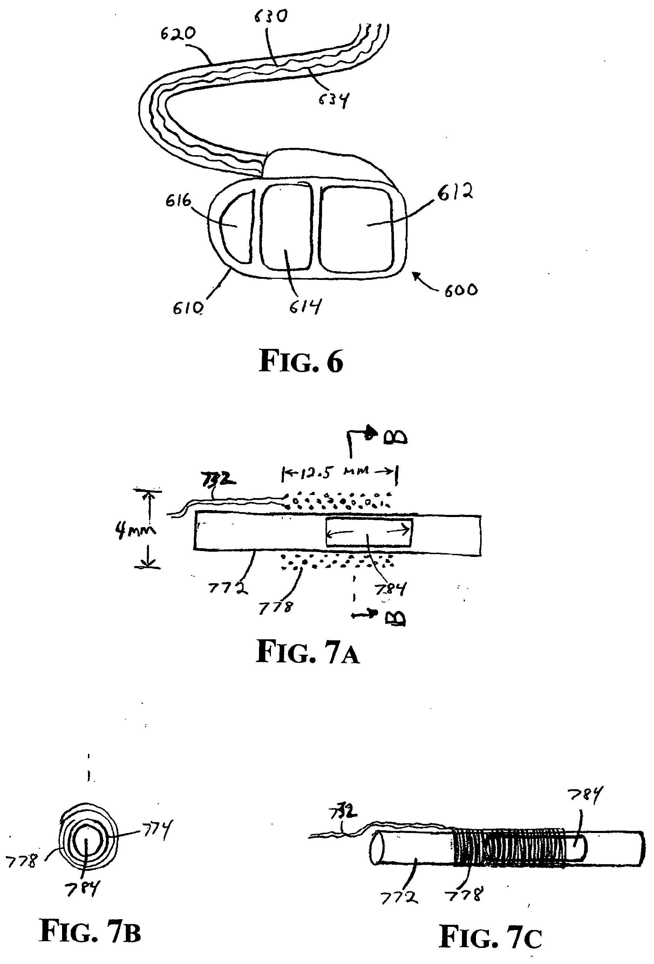

As shown in **FIG. 6**, in a typical ICD or other CRM device **600,** power, sensing, and signal control are provided by a controller positioned within the implantable control device **610** that houses electronics **616,** power supply batteries **612**, capacitor **614,** etc. Electronics can include a variety of components including, for example, circuits, power storage, and processors. These components may be arranged in numerous configurations within the control device **610.** Standard CRM devices are described fully elsewhere. In these standard devices, wires **630** run from the device down the hollow center of the lead **620** to the coils on the outside of the lead **620** in the heart, to provide the electrical current needed in a defibrillation event. These devices typically also have an electrical sensor at the tip of the lead **620** that provides the implantable control device **610** with information, e.g., on cardiac pulse and effectiveness of the defibrillation action. In a pacemaker, or a CRT-D device, the implantable control device **610** may also provide an electronic pulse to pace the heart. These are well-described elsewhere, and the wires **630**, **634** for these activities also run down the hollow lead **620** tube.

In one variant, power provided by the micro-generator module is rectified and processed and requires no, or little, storage, but can be used directly to power the CRM device electronics and functions.

In the prototype illustrated in **FIGS. 7A-C,** the diameter of the micro-generator capsule tube **772** with wire coils **778** wrapped around the capsule walls **774** was about 4mm total outer diameter. The coil **778** consisted of 12,000 turns of 51 gauge copper wire. In some configurations the use of copper is desirable to make inductive coil wires because copper is a high efficiency conductor that can also be configured into a wire having a very skinny gauge. However, copper is not considered biocompatible so in configurations where copper is employed, other design considerations will be employed, as discussed elsewhere, to shield the mammalian body from the wires.

In this prototype, the inductive coil **778** was 12.5 mm long. The magnet **784** was 12.5 mm long, 1.6 mm diameter, and made of medium-high magnetic flux strength N42 material. As will be appreciated by those skilled in the art, different lengths of coil, length of magnet, number of turns of coil, etc. could impact the performance of the micro-generator. In addition, stronger flux magnets are also available, and optimal designs will include the highest flux strength possible, for example N52 or stronger in rare earth magnets. Many magnetic materials besides rare earth can be used as well. Some cardiac leads are about 3mm diameter, and the device could be designed to fit within a range of diameters. These would all be optimized to obtain the greatest possible electrical output within dimensional and other constraints. As with other configurations, the coil **778** is in communication with the implantable control device via power wires **732**. As will be appreciated by those skilled in the art, the coil and wire considerations discussed with respect to this prototype also apply to other configurations disclosed herein.

The interior ends of the micro-generator capsule may alternatively be simple stops, springs, or rubber or other highly elastic bumpers. Length, diameter, size of coil, size of magnet, etc. will be optimized to, for example, take into consideration patient implantation parameters, energy needs of the device that the micro-generator is powering, etc. For use within the heart, the size of the heart chamber, avoiding the valve between chambers, ability to steer the lead tip, etc. will impact the desirable size and position of the micro-generator. In some configurations it is desirable to enable a totally elastic collision between one or more magnets and one or more end caps or other features. Springs can be particularly useful if they are deformed in an elastic region. The springs can take a variety of shapes, including, but not limited to, conical, cylindrical, straight, square, nub, coiled, spherical, trapezoidal, leaf.

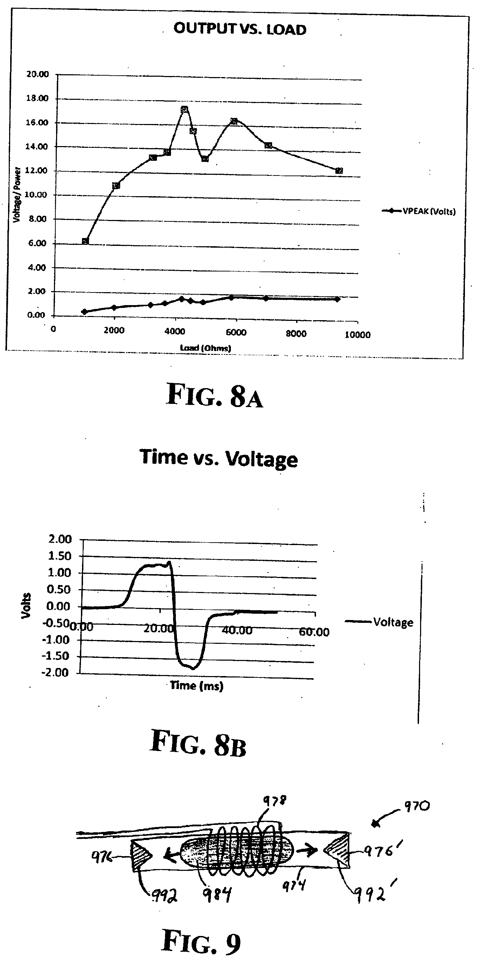

Actual scale prototypes built to date such as the one described above have demonstrated the ability to generate substantial amounts of power at usable voltages. **FIG. 8A** shows the results of tests done with the configurations of device in **FIG.** 7.

In **FIG. 8A**, a device using 12,000 turns of 51 gauge wire was tested while applying varying resistances to match impedance. Optimal power output is achieved where the load impedance matches the output impedance of the micro-generator device. At the optimal power output in this simple experiment, over 17 microwatts were generated at a usable peak voltage of 1.59 volts, at an output resistance load of 4170 Ohms. Designs can be refined to optimize for length, diameter, wire gauge, number of turns, magnet shape and size, etc.

| LOAD (Ohms) | VPEAK (Volts) | POWER-2 PASS (uW) |
| --- | --- | --- |
| 998 | 0.37 | 6.25 |
| 1977 | 0.77 | 10.87 |
| 3165 | 1.04 | 13.28 |
| 3630 | 1.20 | 13.73 |
| 4170 | 1.59 | 17.32 |
| 4470 | 1.45 | 15.51 |
| 4880 | 1.36 | 13.26 |
| 5800 | 1.79 | 16.40 |
| 6950 | 1.78 | 14.48 |
| 9300 | 1.82 | 12.46 |

**FIG. 8B** shows the output voltage vs. time curve for a single oscillation of the magnet inside the coil. Since the output of the generator will normally be an oscillating current as shown in **FIG. 8B**, the electronics of the control and storage can will need to be able to rectify the signal in order to store the energy or to use the output directly to power the device.

Based on the output and future optimization, the useful life of an implanted CRM device could be increased from 10-200%, more typically 50-150%, and even more typically 75-125%.

**FIG.** 9 illustrates a configuration of micro-generator module consisting of a magnet *984* positioned within a micro-generator capsule *972* having an inductive coil *978.* Numerous configurations are possible to help maximize the use and conservation of kinetic energy available to move the magnet and generate power. A totally elastic collision between the magnet and the end cap, including a hard knock, will preserve kinetic energy and therefore result in high efficiency.

Features such as elastic bumpers *992, 992*' could be added to help accomplish highly elastic collisions of the magnet *984* with the capsule end walls *976, 976'* and to eliminate any noise of collisions. These could be made of a variety of elastic materials, and could take a variety of shapes-conical bumpers are illustrated. The moving magnet *984* bumps against the bumpers *992, 992'* at either or both ends of travel inside the micro-generator *970,* and is bounced back toward the center of the micro-generator.

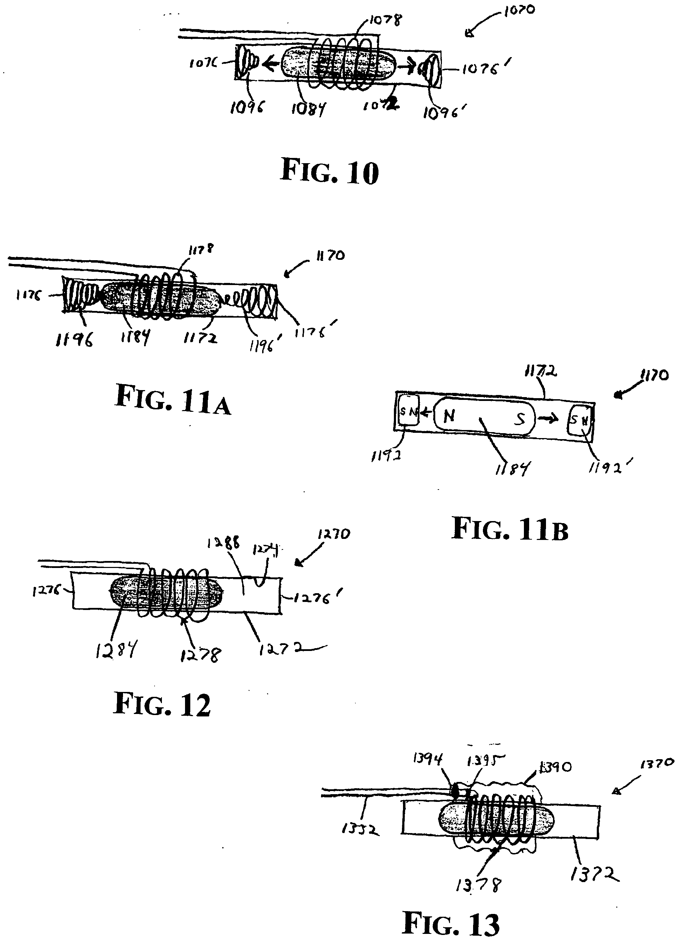

**FIG. 10** illustrates another variation of the micro-generator *1070.* **FIG. 10** shows a configuration in which elastic springs *1096, 1096'* are used at either or both ends *1076, 1076'* as end stops. These could take a variety of shapes-conical springs are illustrated, but many other shapes would occur to persons skilled in the art. The springs *1096, 1096'* are designed to flex in their elastic region for maximum conservation of kinetic energy. The moving magnet *1084* bumps against the springs *1096, 1096'* at each end of travel inside the micro-generator, and is bounced back toward the center of the micro-generator. The inductive coil wire *1078* is wrapped around the capsule or housing *1072* and is in communication with the CRM implanted control device (not shown), providing generated power back to the control device.

**FIG. 11A** illustrates an alternative configuration in which the moving magnet *1184* is suspended linearly between the two ends of the micro-generator capsule *1172* by springs *1196, 1196'.* One end of each spring *1196, 1196'* is attached to an end *1176, 1176'* of the micro-generator capsule housing *1172,* the other end of the spring is attached to an end of the magnet *1184.* In this configuration, the magnet *1184* may maximize its oscillations within the micro-generator *1170,* moving back and forth freely as a result of most motions of the heart and body, generating power continually through even small oscillations (potentially even between heartbeats). The inductive coil wire *1178* is wrapped around the capsule or housing *1172* and is in communication with the CRM implanted control device (not shown), providing generated power back to the control device.

In a variation, the magnet could be attached to the magnet on one end only.

FIG. 11B illustrates an alternative configuration of a micro-generator capsule *1170* in which the moving magnet *1184* having a north and a south pole is suspended between two magnets *1192, 1192'* at the ends of the micro-generator capsule *1172*. Magnet *1192* is oriented such that its pole facing toward the moving magnet *1184* is the same as the pole of the moving magnet *1184* facing it. For example only in the drawing, the north pole of magnet *1192* faces the north pole of the moving magnet *1184*. Similarly, by example, the south pole of magnet *1192'* faces the south pole of moving magnet *1184*. Since like magnetic poles repel, magnet *1184* is free to oscillate within the micro-generator capsule *1172*, but is effectively "bounced" off the ends by the opposing magnets *1192, 1192'*. This is a desirable configuration as no mechanical energy losses are created, and there are no springs or other mechanical parts to fatigue. Additionally, as magnet *1184* would be repelled from either of the opposing magnets *1192, 1192',* before coming into contact with the opposing magnets *1192, 1992'* any noise that might otherwise be generated by collision would thereby be eliminated.

FIG. 12 illustrates a micro-generator *1270* with capsule *1272* that is sealed to prevent moisture from intruding into the capsule, which could impair motion of the magnet *1284* and cause corrosion of the components. As with previous configurations, the micro-generator *1270* includes a magnet *1284* positioned within a capsule housing 1272 which has an inductive coil *1278* around it.

In a variant on this approach, the space *1288* inside the capsule *1272* is evacuated. This has the benefit of allowing free motion of the magnet *1284*. Best electrical power generation performance will occur when the flux lines are as close as possible to the coils *1278*. Thus, the magnet *1284* outside diameter should be as close as possible to the inside diameter of the capsule walls *1274*. However, if any fluid or gas is present, the magnet *1284* will displace the fluid as it moves, forcing the fluid to pass to the other end of the capsule through the small gap between the magnet *1284* and the capsule walls *1274*. This creates an impeding force to motion, and reduces the speed and efficiency of the magnet's *1284* motion. Evacuating the capsule *1272* eliminates this resistance.

In another variant on this approach, the capsule chamber *1272* could be partially evacuated (optimal pressure will be defined) and the magnet *1284* designed to form a close near-seal with the walls *1274* of the capsule. In this configuration, the gas trapped at each of the ends of the capsule *1272* acts as a spring, providing the benefits described elsewhere. One or two weak mechanical springs could be used to keep the magnet's *1284* motion centered in the length of the capsule *1272*. Additionally, magnets *1192, 1192'* with like poles opposing the poles of the moving magnet *1284,* could provide the centering force to suspend the magnet *1284*.

In another variant to reduce friction, the outside surface of the magnet *1284,* or the inside surface of the micro-generator capsule walls *1274,* or both, can be coated with a lubricant. Many types of lubricants can be envisioned by someone skilled in the art, including liquids, coatings, as well as thin deposited surfaces and films.

As will be appreciated by those skilled in the art, implantable devices, such as those described herein, should be constructed of materials that are biocompatible with the human body, and safe with body chemistry. While copper wiring is a highly efficient conductor for power transfer, copper is not a biocompatible metal. Turning now to FIG. 13, although the micro-generator module *1370* is embedded inside a CRM device lead, these leads are made of plastics that typically allow transfer of moisture. Thus, if the induction coils *1378* are on the outside of the micro-generator, they are effectively exposed to the bodily fluids, and thus must be made of biocompatible materials (platinum is often used). These metals are expensive and are typically less efficient carriers of electrical energy than copper.

However, the area outside the housing capsule *1372* of the micro-generator module *1370* where the inductive wire coils *1378* are located could be sealed with an epoxy or other waterproof/ moisture proof or moisture resistant sealant *1390* effectively isolating the non-biocompatible wires from the environment. The biocompatible power wires *1332* can pass through a sealed feed-through *1394* to connect to an attachment point *1395* with the non-biocompatible coil wires *1378* inside the sealed region. Thus the very fine gauge induction coil wires can be made of copper or other efficient, non-biocompatible material, with a sealed feed-through to the main wires that run through the cardiac lead to the implantable control device. These wires must be biocompatible due to fluid leakage through the walls of the lead.. This approach could have the advantage of providing the thinnest possible approach to sealing, thus keeping overall diameter as low as possible.

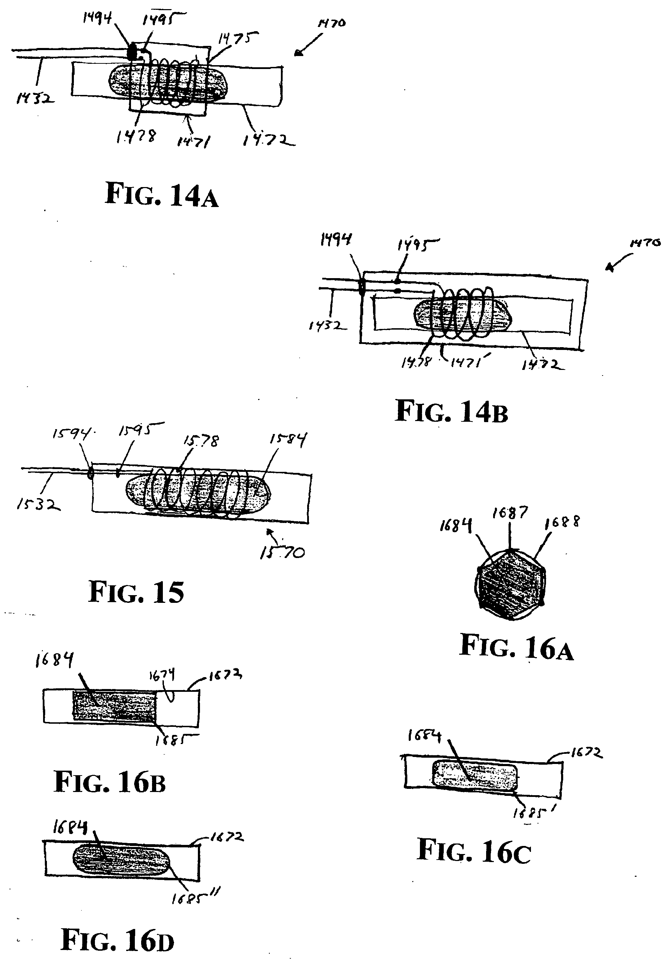

FIGS. 14A-B illustrate a micro-generator capsule *1472* and induction coils *1478* contained inside another, secondary sealed capsule *1471,* again with sealed feed-throughs *1494* providing access for the biocompatible main power wires *1432* inside the lead, to an attachment point *1495* inside the secondary capsule *1471,* joining these to the non-biocompatible coil wires *1478*. This capsule *1471* could be attached and sealed to the walls of the micro-generator capsule *1472* only in the area of the inductive coils *1478,* or could fully contain the micro-generator module *1470* as shown in FIG. 14B.

FIG. 15 illustrates a single micro-generator module *1570 fully* containing an induction coil *1578* inside the micro-generator walls. This coil could be coated, lubricated, or otherwise protected to guard against abrasion from the moving magnet *1584.* Coil wire *1578* could thus be copper or other high efficiency conductor, and sealed feed-throughs *1594* allow the biocompatible main wires to pass out of the capsule to the inside of the lead, and an attachment point *1595* joining these to the non-biocompatible coil wires *1578.*

Many alternatives to magnet *1684* shapes and cross-sections relative to the cross-section of the micro-generator housing *1672* can be envisioned to improve performance and reduce friction. **FIGS. 16A-D** show some of the possibilities, while other configurations will be apparent to those skilled in the art. The best power generation performance is derived when the magnet walls are as close as possible to the induction coils. This argues for as little space as feasible between the moving magnet and the micro-generator capsule walls. However, this configuration will increase the likelihood of friction between the walls and the magnet.

**FIG. 16A** shows an end view of a magnet *1684* that has been shaped with facets instead of a circular outside (illustrated here as an example only as having a hexagonal cross-section). In this configuration, some portion of the cross-section of the magnet will be longitudinal corner ridges *1687* that engage or nearly engage an interior surface of an interior wall of the housing while other sections have a space *1688* between the magnet and the interior surface of the housing. The outer points of the facet corners *1687 will* be the only contact area with the inside wall of the micro-generator capsule *1674.* The best generation performance may occur as the cross-sectioned shape approaches a circle, so that the magnetic flux lines are close to the induction coil. The optimal design will be based on a tradeoff of electrical performance and minimizing frictional losses. This faceted approach would also allow air or other gas to move easily past the magnet *1684* as it oscillates, in the design where the micro-generator capsule *1674* is not evacuated. The variety of cross-sectional shapes is numerous and will be a function of optimizing. Other shapes include, but are not limited to, round, triangular, tetragonal, pentagonal, heptagonal, octagonal, nonagonal, decagonal, oval, and ellipsoid.

**FIGS. 16B-D** also show various lengthwise side views of magnets *1684.* The longitudinal cross-sectioned shape of the magnet ends *1685, 1685', 1685"* varies from square (**FIG 16B**) to slightly rounded (**FIG 16C**) near the micro-generator capsule walls *1674* to reduce friction from sharp edges, to fully rounded (**FIG 16D).** Shape will be optimized for best performance.

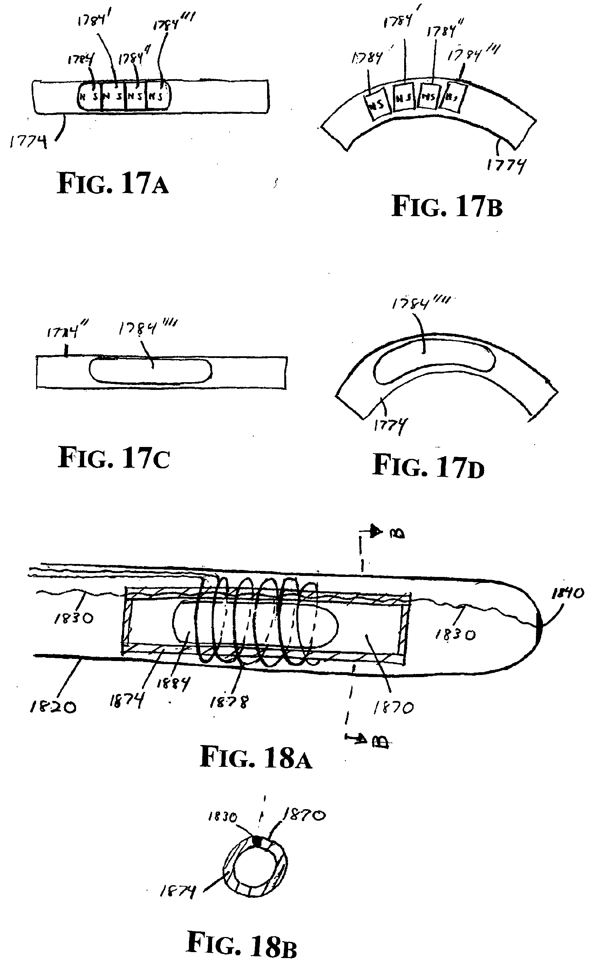

**FIGS. 17AB** illustrate a micro-generator module *1770* comprising multiple short magnets *1784* to allow flexibility of the module and lead tip. This could be beneficial in steering the micro-generator module into place in the heart.

As shown in **FIGS. 17A-B,** when the generator containing separate magnets *1784, 1784', 1784", 1784"'* is bent the magnets separate from each other to form a temporarily curved segmented magnet.

**FIGS. 17C-D** show a similar flexible magnet approach. However, in this design the magnet *1784* is made of a flexible polymeric or other magnetic material allowing it to bend when needed within the module *1770.*


## B. ROUTING DEVICE LEADS

**FIGS. 18A-B** show a mechanism for passing one or more additional wires *1830* past the micro-generator module *1870* without requiring the lead *1820* diameter to increase in the bypass region. The walls *1874* of the micro-generator capsule *1872* are thick enough to enable molding one or more signal wires *1830* for heart beat sensing, pacing, or other wires into the walls themselves. This requires either insulating the wire thus embedded if the micro-generator capsule walls *1874* are made of a conductive material such as aluminum or titanium, or using a non-conductive material such as plastic, glass, or ceramic for the capsule walls, thus not requiring insulating the wires. The signal/control wire *1830* attaches at attachment point *1840* to the distal tip on the interior of the tip of the lead for pacing or sensing. A magnet *1884* is positioned within a capsule housing *1874,* which forms part of the micro-generator module. An inductive coil *1878* surrounds at least part of the capsule.

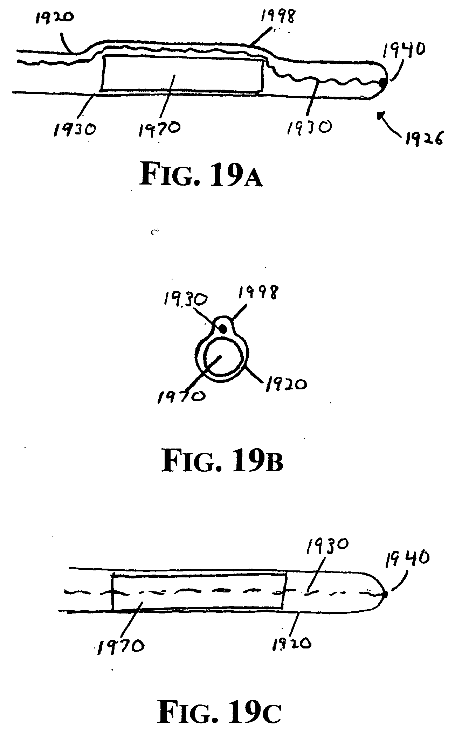

**FIGS. 19A-C** illustrate a region of the cardiac lead near the tip *1926,* the complete micro-generator module *1970,* and an asymmetrical bulge *1998* in the wall of the lead *1920* to allow space for the sensing or pacing wire or wires *1930* to pass by the micro-generator module *1970,* which mostly fills the interior space of the lead *1920.* The sensing or pacing wire or wires *1930* attaches at an attachment point *1940* to a sensing or pacing contact at the tip.

In the end view shown in **FIG.19B**, the bulge *1998* is on the top, and has the sensing wire *1930* inside the bulge *1998.* In the top view shown in **FIG 19c**, the sensing wire *1930* passes down the inside of the bulge *1998* on the top of the lead *1920,* shown as a dashed line, but the side walls *1974* are symmetrical and no larger than normal.

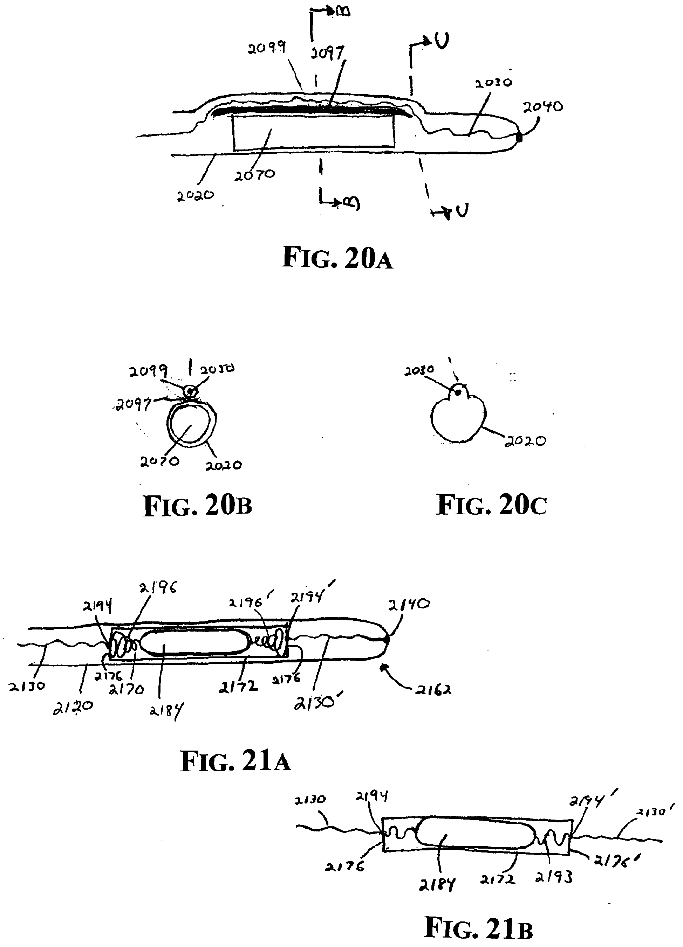

**FIGS. 20A-C** shows a variant in which the lead wall of lead *2020* not only has a bulge, but a separate tubing compartment with a space *2097* separating the lead *2020* section containing the micro-generator module *2070* from the tube *2099* containing the sensing wire *2030,* thus creating two separate tubes in this region. The cross-sections show the region just as the separate tube is forming, but not yet completely separated, and the region of the generator where the separate bypass tube is fully formed with a separation region where the main lead *2020* and bypass each forms a full cylinder tube. This approach might improve flexibility/maneuverability of the lead tip for steering into place in the heart or enhance the ability to seal the micro-generator module to the inside of the lead.

**FIGS. 21A-B** illustrate two variants of using the conduction of a magnet *2184* to pass the signal wire *2130* signal. In the first, the magnet *2184* is positioned within a capsule housing *2172* within a lead tip *2162* and is suspended between two springs *2196, 2196'*. Signal or other wires *2130* are connected to these springs *2196, 2196'* through feed-throughs *2194, 2194' in* the end walls *2176, 2176'* of the micro-generator capsule *2172*. Thus the signal from the signal wire *2130* passes through a first end wall *2176'*, through the first spring *2196*, through the magnet *2184*, through the second spring *2196'*, and out the opposite end wall *2175'* to continue toward its destination. This avoids the need to grow the diameter of the lead *2120* in order to pass lead wires past the micro-generator module *2170*. In **FIG. 21B**, a variant is shown in which the magnet *2184* is attached to flexible wires *2193* rather than springs. These wires stretch and compress inside the micro-generator capsule *2170* between the capsule end wall *2176, 2176' and* the magnet *2184*, maintaining an electrical connection. Both of these approaches work best when the micro-generator capsule is made of a non-conductive material, or when the magnet and/ or capsule are coated with a non-conductive film.

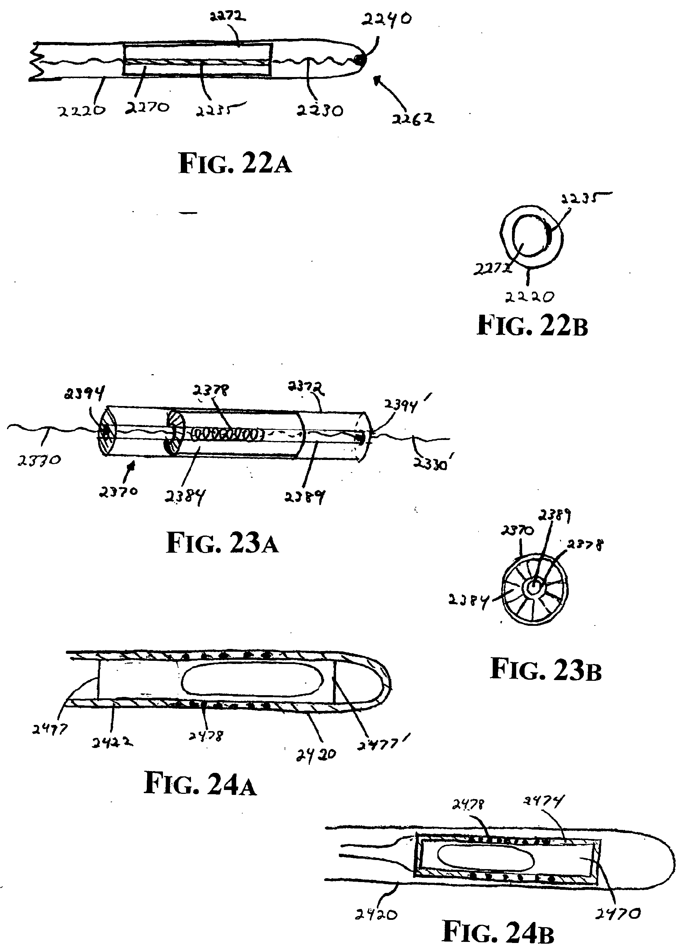

**FIGS. 22A-B** illustrate a configuration for passing signal wire *2230* signals past the micro-generator module *2270* without increasing or significantly increasing the diameter of the micro-generator module *2270* or the cardiac lead *2220*. The signal wire *2230* is connected to the distal end of the lead tip *2262* at a connection point *2240*. In this configuration, conductive traces *2235* are coated, sputtered, glued, embedded, or otherwise attached to the outside surface of the micro-generator capsule *2272*. These can be made flat, coated with an insulating material, etc. In the case of a non-conductive capsule material like normal ceramic, the conductive material could be attached or deposited directly on the capsule without the need for insulation. In the case of a titanium or other conductive capsule material, the traces would need to be separated from the walls of the conductive capsule by insulation material.

**FIGS. 23A-B** show an approach for routing signal or other wires *2330* through the micro-generator module *2370*. It uses a sealed tube *2389* down the center of the micro-generator module *2370*. The signal wire *2330* passes through sealed feed-throughs *2394* into the generator *2370*, down the tube *2389*, and out a corresponding feed-through *2394'* at the opposite end of the micro-generator capsule *2372*. This requires the magnet *2384* to be a hollow cylinder with an aperture running axially down its longitudinal center through which wires *2330* can pass. This will waste magnetic flux lines, so the center cylindrical aperture should be as small in diameter as possible. In a variant, the central tube could contain a separate set of inductive coils *2378* to use the inside flux lines of the magnet to generate additional power. A hollow magnet *2384* would also allow air or other gas to move easily past the magnet as it oscillates, in the design where the micro-generator capsule *2372* is not evacuated.

**FIG. 24A** shows an approach for avoiding the need for a separate self-contained micro-generator module. In this variant, the walls *2422* of the cardiac lead *2420* contain the inductive coils *2478* molded within the lead material. Since plastics used to make leads *2420* are permeable to moisture, this would work best in the case where moisture is not a problem, perhaps with increased spacing between the walls of the magnet *2484* and the lead walls *2422*. To define the limits to the motion of the magnet *2484*, end walls *2477, 2477' or* other stops, springs, etc. as described elsewhere, would be built into the lead *2420*.

**FIG. 24B** illustrates an approach for embedding the inductive power coil wires *2478* within the capsule walls *2474* of the self contained micro-generator module *2470* itself. The sealed walls *2474* protect the coil wires *2478* from surrounding fluids, provide a physical protection barrier to the coils *2478*, and offer a minimal diameter for the overall micro-generator module within the CRM lead *2420*.

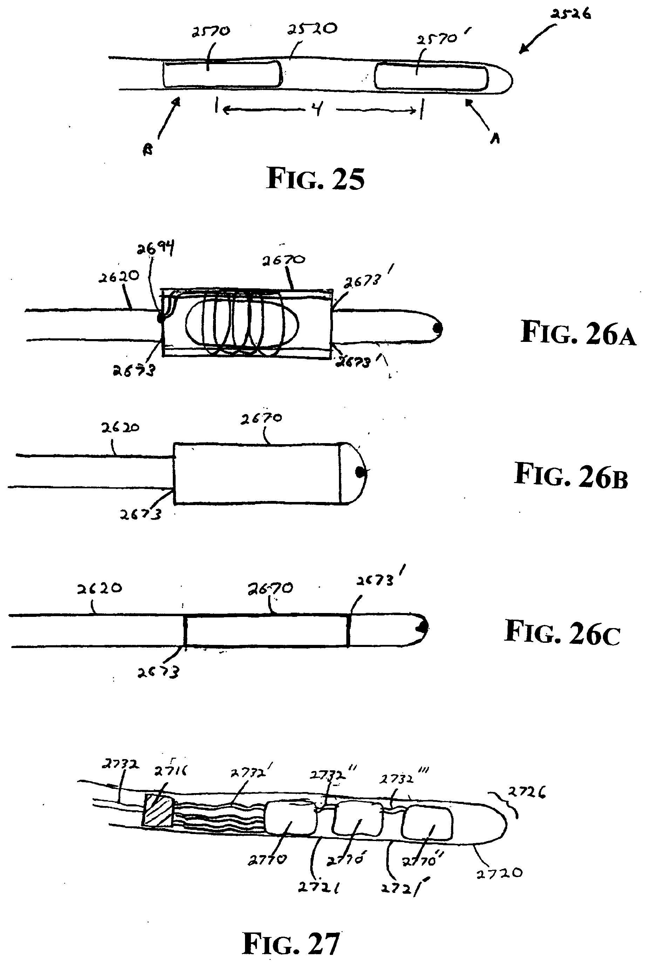

As shown in **FIG. 25**, optimal placement of the micro-generator module *2570* in the cardiac lead will be determined as a tradeoff between transferring the maximum possible mechanical energy from the heart's beat along the lead walls to the micro-generator module (which argues for placing near the lead tip *2526* (position **A**), versus maximizing lead *2520* flexibility at the tip *2526*, for example, to enhance steering upon insertion by the physician (which would argue for moving the module further from the tip (position **B**). The acceptable range of positions is a tradeoff and optimization that will be determined for best placement.

**FIGS. 26A-C** shows an approach in which the micro-generator module *2670* is a distinct section of the cardiac lead *2620*. This approach avoids the requirement that the micro-generator module *2670* outer diameter be smaller than the inside diameter of the cardiac lead *2620*. In this approach, the lead *2620* is attached to the sealed micro-generator module *2670* at attachment area *2673*. If the lead *2620* continues past the micro-generator module *2670*, another attachment area *2673'* occurs at the start of the next segment. Sealed feed-throughs *2694* allow micro-generator module *2670* wires to pass into the not moisture resistant area inside the main lead *2620*. As illustrated in **FIG. 26B** the micro-generator module *2670* may also be positioned at the end of the cardiac lead *2620*. In this approach, the micro-generator module *2670* outer diameter may thus be smaller, equal to **(FIGS. 26c)** or greater than the diameter of the cardiac lead *2620*, offering more flexibility of design, and potential for greater power generation in a given length, made possible by greater diameter, magnet size, coil turns and thickness, etc. One or more attachment points *2673, 2673'* are provided between the micro-generator module and the lead.

**FIG. 27** shows multiple modules in a cardiac lead *2720*. In order to generate more power from each heartbeat, while maintaining small size (to fit into lead *2720*) and flexibility (to enable placement and steering), it is possible to design a lead containing more than one micro-generator module. **FIG. 27** shows a cardiac lead *2720* containing three separate micro-generator modules *2770, 2770', 2770"*, and their respective power leads *2732', 2732", 2732'''* running from the distal end or tip of the lead *2726*, combining into a single lead *2732* which runs toward the control device (not shown) end of the lead *2720.*

The nominal single micro-generator module described previously is short and should not make it unduly difficult to guide and implant a lead into the proper location in the heart. However, if needed, **FIG. *27*** shows one configuration by which the micro-generator *2770* might be designed to increase the overall effective generator length, and thus power output, but still remain flexible for insertion. By using separate micro-generator modules *2770, 2770', 2770'',* the regions *2721, 2721' between* the stiff micro-generator modules *2770* are more flexible and easily steered for insertion into the heart.

In **FIG. 27**, the needed power is provided by multiple, shorter micro-generator modules *2770,* either connected together electronically inside the lead *2720,* or with wires from each running up the lead to the implanted control device for processing and storing the generated power. This might also be an ideal use for local electronics *2716* to process the power generated by these multiple independent generators before delivering a combined power signal through a single wire pair *2732* back to the control device.

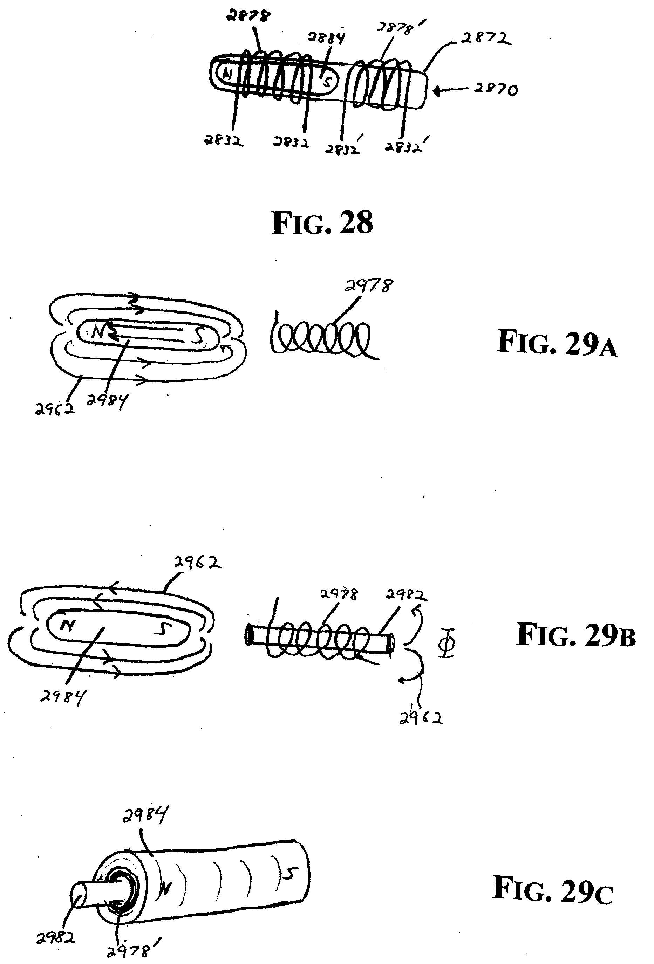

**FIG. 28** shows a micro-generator module *2870* configured so that it has two (or more) separate inductive coils *2878, 2878'.* The first *2878* is wound in the first direction, and the second coil *2878'* is wound in the second direction. In the case of additional separate coils, winding direction would continue to alternate. The inductive coils *2878, 2878'* are in communication with respective power wires *2832, 2832'.* As the magnet *2884* moves from left to right (from one end to the other within the housing), the flux created by the north pole of the magnet *2884* cuts through the first coil *2878* as the magnet *2884* approaches or passes through the coil *2878.* This generates a voltage in one sign through the turns of the coil *2878.* At the same time, the south pole of the magnet *2884* is moving through the coil *2878',* similarly generating a voltage of one sign through the turns of coil *2878'.* Similarly, as the magnet reverses and passes from left to right, a reverse voltage is created in the turns of coils *2878, 2878'.* Output wires *2832, 2832'* of these coils *2878, 2878'* could run separately to the control device for processing and storage of power. Alternatively, they could be connected in series or parallel, and wound and connected together so that the combined voltage generated in each pass of one direction of the magnet *2884* generates a combined voltage of one sign, thereby increasing the output of the generator. By properly combining the output of these power wires, coils could be physically wound in either direction, and effectively combined electrically.

In addition, in the nominal design, a single pass of the magnet *2884* completely through the coil (so that the magnet ends pass the ends of the coil, produces a reversing voltage as the midpoint of the magnet passes the midpoint of the coil. Thus, in order to be used, the output must be rectified. However, in this two coil configuration, with coils wound or outputs combined in opposite direction, a single pass of the magnet from right to left or left to right will produce voltage of only one sign. The sign of the voltage output will only change as the magnet's direction reverses.

**FIGS. 29A-C** illustrate an energy harvester micro-generator improved by arranging coils *2978* to collect a maximum of flux from the moving magnet(s). **FIGS. 29A** shows flux lines *2962* from a cylindrical magnet *2984.* From **FIG. 29A,** a coil *2978* will collect a maximum of flux if its inner diameter most closely approaches the outer diameter of the magnet so that no flux can leak around the magnet *2984* without passing through the coil *2978.*

As shown in **FIG. 29B,** it could be advantageous to pass a core *2982* made of a high-permeability material such as a ferrite through the coil *2978,* and displace only the ferrite core *2982,* or the coil *2978* and the ferrite *2982,* or the magnet *2984,* or any combination of these. The ferrite will tend to pull lines of flux *2962* through it and thus through the coil. As the ferrite core moves relative to the magnet and/or the coil, it will cause a change of flux in the coil, generating a voltage $\varepsilon$.

In an embodiment with a tubular magnet *2984* and an internal coil *2978'* as previously described, it could be advantageous to provide the inner coil *2978'* with a ferrite core *2982* shown in **FIGS. 29c**, again to ensure maximum flux through the coil. A tubular magnet would have flux inside the tube and outside the tube. Power generating coils can be positioned both inside the coil of the magnet and outside the coil of the magnet.

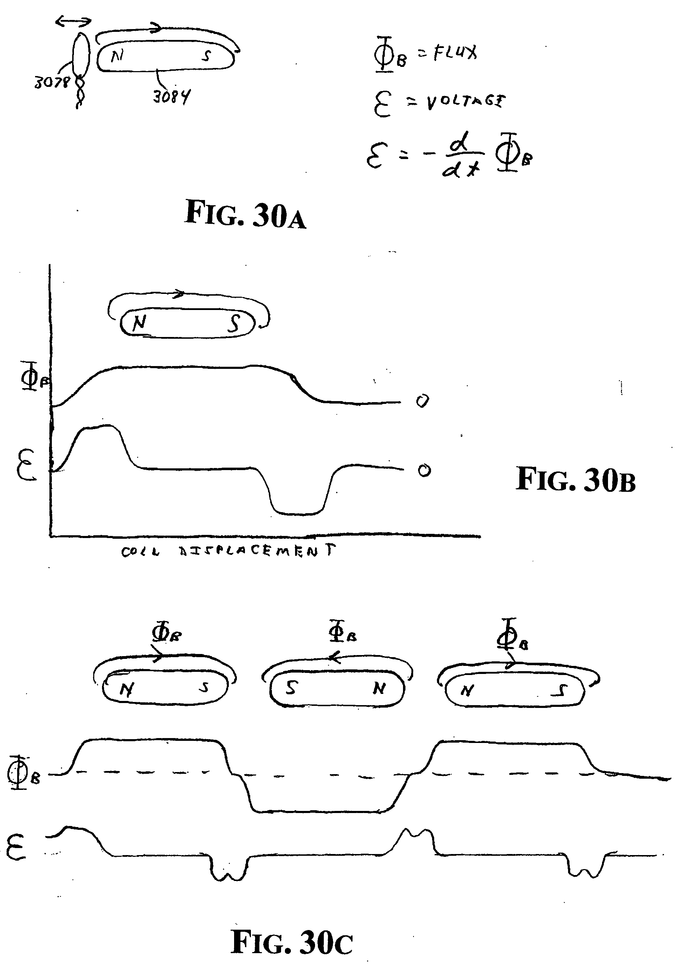

**FIGS. 30A-D.** To visualize the inductive generation approach, a single magnet *3084* may be arranged to be displaced relative to a wire loop. By changing the frame of reference, consider the movement of the loop *3078* over the magnet *3084* in **FIG. 30A.** The movement will cause a change in magnetic flux $\Phi_B$ through the loop, which will induce a voltage $\varepsilon$ around the loop.

**FIG. 30B** shows the $\Phi_B$ and $\varepsilon$ as a function of position as the loop *3078* moves along the axis of the magnet 3084. Voltage is determined using the following equation:

$$\varepsilon = -(d/dt)\, \Phi_B$$

where: $\Phi_B$ = Flux, and $\varepsilon$ = Voltage

When multiple magnets are arranged in series, for example in a micro-generator module (illustrated above) to serve as an energy harvester, it is advantageous to arrange their polarities in alternate directions as shown in **FIG. 30c**.

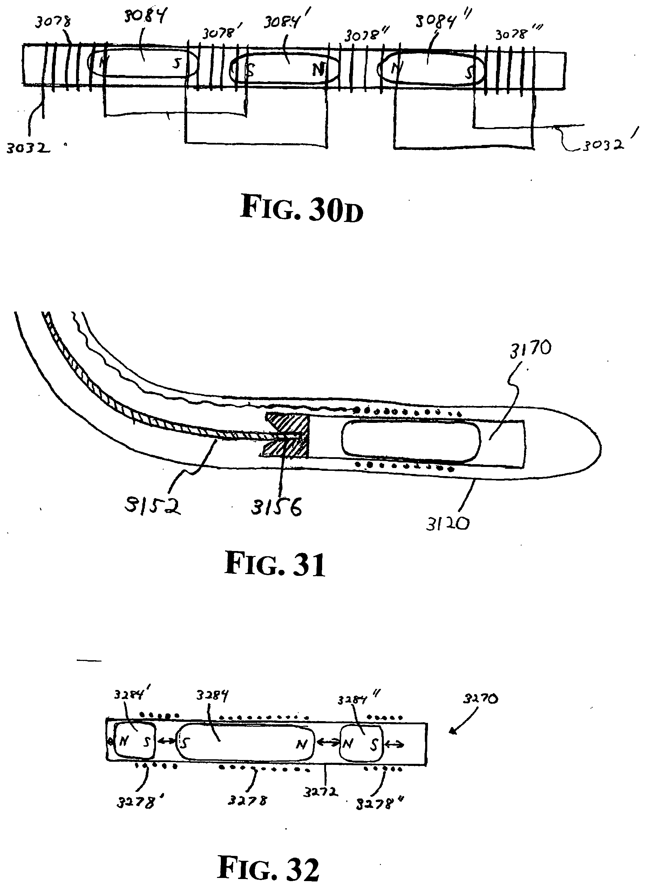

**FIG. 30c** again illustrates Φ$_B$ and coil displacement. Consider a loop moving outside the three magnets and along a single axis (such as the X axis). The alternating magnet polarities produce voltages of the same sign as the coil passes the gap between magnets. This simplifies performance and power output of a micro-generator module energy harvester **3070** in **FIG. 30D.**

Magnets **3084, 3084', 3084"** are arranged with their north and south poles in alternate directions, with like poles facing each other, between separate coils **3078, 3078', 3078", 3078'"**. Connecting the coils in opposite directions provides additive voltages graphed above at terminal wires **3032, 3032'**. When the leading edge of a magnet is approaching a turn of a coil, the increasing flux will generate a voltage of one sign in that turn. When the body of the magnet is moving through that turn, the constant flux will generate no voltage, and when the trailing edge of the magnet is moving beyond the same turn, the decreasing flux will reverse the sign of the voltage in that turn.

In **FIG. 31** a micro-generator module **3170** is illustrated positioned within a tip of a CRM lead **3120.** The micro-generator module has a stylet lumen **3156** at its proximal end (end closest to the implantable control device) into which a steerable stylet **3152** can be positioned. The steerable stylet facilitates placement of the CRM lead, containing the micro-generator module within the distal tip of the lead, into the proper position in the heart. Use of a steerable stylet is common practice in placing such leads in the heart. Additionally, the stylet lumen **3156** helps guide the stiff micro-generator section during steering.

**FIG. 32** illustrates a coil and magnet configuration having three magnets and three coils similar to that shown in **FIG.11B.** The micro-generator capsule **3270** has a moving magnet **3284** having a north and a south pole which is located between two magnets **3284'**, **3284"** toward the ends of the micro-generator capsule **3272.** Additionally, end magnets **3284' and 3284"** are oriented such that their poles facing toward the moving center magnet **3284** are the same as the pole of the moving magnet **3284** facing them. For example only, in the drawing, the south pole of moving end magnet **3284'** faces the south pole of the moving center magnet **3284.** Similarly, by example, the north pole of moving end magnet **3284"** faces the north pole of moving center magnet **3284.** Since like magnetic poles repel, center magnet **3284** is free to oscillate within the micro-generator capsule **3272,** but is effectively "bounced" off the ends by the opposing magnets **3284', 3284",** which themselves are free to impact the ends of the capsule **3272.** This is a desirable configuration as no mechanical energy losses are created between the center and end magnets, and there are no springs or other mechanical parts to fatigue. Additionally, as center magnet **3284** would be repelled from either of the opposing end magnets **3284', 3284",** before coming into contact with the opposing magnets **3284', 3284"** any noise that might otherwise be generated by collision would thereby be eliminated by the repelling effect of the opposing magnets. Additionally, three coils **3278, 3278', 3278"** having the same windings or differing windings can be positioned such that each coil surrounds an exterior of at least a portion each of the magnets. In some instances, and at least at some points in time, one magnet may be surrounded by more that one coil.

## II. METHOD OF IMPLANTATION, USE AND OPERATION

In a method of implanting a cardiac lead, an implantable cardiac lead extends through an introducer catheter. The lead is typically configured such that it has an elongated lead body extending from the lead connector end assembly to a distal end where co-axially wound, coiled wire lead conductors are disposed (see, e.g., **FIG.** 4). The conductors can be configured such that they are separated by an insulating sheath. A lumen is formed internal to the lead as a feature of the micro-generator, and is adapted to receive a stiffening stylet wire. The stylet extends proximally from the lumen end opening so that a knob may be manipulated by a physician to rotate or axially extend or withdraw the stylet wire with respect to the catheter.

In other configurations and methods, a separate introducer catheter is not required. For example, the stylet can be configured such that it runs down the inside of the CRM lead, and attaches to the a connector associated with the stylet lumen on the micro-generator.

The internal stylet lumen may be formed, for example, by a curved appendage that is configured to attach to an inner wall of the introducer catheter at first and second attachment points. Practically, the introducer size may be dimensioned to include a range of various dimensions that are compatible with the lumen and stylet as implemented and disclosed herein. The lumen may also be configured such that it is collapsible when the stylet wire is removed. A reduced wall thickness may be associated with the appendage that forms the stylet lumen near the attachment points. The thinning of the wall thickness at this point facilitates the collapsibility of the lumen. A thicker wall section may be provided between the attachment points such that the thicker wall section resists perforation by the stylet. If desired, a thin wall PTFE liner could be provided inside lumen to further protect against perforation and reduce friction.

In delivering the lead, the lead and catheter are advanced into position using the stylet to assist in placement. Once the lead is positioned and fixated to the cardiac wall (e.g., in at least one of an atrium and a ventricle), the stylet is removed from the lumen. When the stylet is removed, the lumen then collapses. With the lumen collapsed, there is adequate space for the lead and connector to pass through the introducer catheter as it is pulled out.

In practice, the devices described above have the advantage of allowing a physician to essentially make no changes to the procedure normally followed when implanting a cardiac lead, since the micro-generator module is adapted and configured to be contained inside a normal looking lead. An alternative would be to provide a separate lead whose function is primarily to house the micro-generator module which is adapted and configured to facilitate delivery to the ventricle, attaching it to the ventricle wall, and providing a path for the power wires from the micro-generator module back to the implantable control device. In this approach, a separate, normal ICD or CRT-D or other cardiac lead would be implanted and operate in the traditional fashion.

A device, such as those described above, is implanted in a mammal, such as a human. A cardiac lead of the device is attached to an area optimized relative to the apex of the heart to provide movement of a magnet within a micro-generator, resulting from the cardiac lead motion. Movement of the magnet within the cardiac lead results in energy which is transferred to the implanted control device where the energy is either consumed by the implantable control device in operation of the control device or stored by the device for use later on using a suitable energy storage system.

## III. SYSTEMS

Typical systems according to the disclosure include a battery operated CRM device, as described above, with the battery configured to power the CRM device, and a remote device configured to communicate with the CRM control device and battery to determine status of at least one of the CRM device and battery.

Each CRM device may be configured to perform one or more designated functions, which may include taking one or more physiological measurements and/or delivering a desired therapy. The implantation sites for CRM device are determined based on the particular therapeutic needs of the patient.

As discussed above, the CRM device includes power supply components (e.g., a battery) for providing electrical power to the various components and/or circuitry for performing the functions described above. The CRM device is desirably made as small as possible, however, which constrains the space within the CRM device that is available for power supply components.

As will be appreciated by those skilled in the art, space constraints within the mammalian body limit the capacity of these power supply components. In an effort to maximize the longevity of a CRM device, its power consumption is minimized, and thus, the average power consumption is desirably very low. For example, in one embodiment, size constraints may limit CRM device to a 100 microampere-hour, non-rechargeable battery. In such a case, the average power consumption of CRM device must be less than 10.0 nA to provide, for example, a 10 year longevity.

In order to achieve this low power consumption, a CRM device may be configured such that it is normally in a "sleep" or "sleeping" state (i.e., an inactive state) characterized by a power consumption of from essentially zero (i.e., a completely powered off state) to a low power state in which only a minimal circuitry (e.g., a timer or comparator) are energized and consuming electrical power. The CRM device is awakened (i.e., powered on) to an active state in which it can perform one or more designated functions. Once awake or awakened, the operation of powering on or energizing one or more aspects of the CRM device to an active state is completed, such that the awakened portion can perform a designated function.

Circuitry within the CRM device can be adapted to detect a wake-up field generated by a primary device or an external device, which then causes the CRM device to awaken and perform its designated functions. The external device is desirably in the active state only to the extent necessary to perform its designated diagnostic and/or therapeutic function(s), after which time it returns to its inactive, sleep state in order to minimize the power usage of the battery within the CRM device. Additionally, in some embodiments, to maximize longevity of the CRM device, the power consumption of the various circuitry for waking up the CRM device is desirably less than about 10 percent of the total power consumption of the remote CRM device.

The external monitoring device operates, in some embodiments, to wake the CRM device from the sleep state, and may further be configured to direct the CRM device to perform one or more designated functions. In this way, the external monitoring device functions as a "master" device while the CRM device functions as a "slave" device. The external monitoring device itself may also be configured to perform therapeutic functions or to take physiologic measurements. For example, the external monitoring device may, in some embodiments, be a pulse generator for providing a cardiac pacing and/or defibrillation stimulus. The therapeutic functions are not limited to any particular type and can include, for example, drug delivery therapy, or any other therapy capable of being administered with a CRM device. Additionally, external monitoring device may be configured to measure physiologic parameters such as blood pressure, temperature, blood or fluid flow, strain, electrical, chemical, or magnetic properties within the body.

It should be noted that neither CRM device nor primary external monitoring device are limited to any particular type or types of devices. For example, CRM device can be any CRM device that is normally in a sleep state to minimize power consumption and is awakened only as necessary to perform a desired function. Similarly, primary external monitoring device can be any device that operates, at least in part, to cause CRM device to wake from a sleep state. Thus, CRM device may sometimes also function as a primary monitoring device in a given embodiment. That is, CRM device may be configured such that, in its active state, it can cause another CRM device to wake and perform one or more desired functions.

As the system is configured, a primary CRM device includes battery, primary controller, including memory and processor, sensing and/or therapy module, communication module, and wake-up field generator. In some embodiments, the primary CRM device may not include the sensing and/or therapy module. As will be appreciated by those skilled in the art, any structure suitable for the contemplated use can be employed without departing from the scope of the disclosure. Thus, the module can include components and circuitry integrated into a single unit as well as individual, discrete components and circuitry that are functionally related, integrally formed or act in a unified manner.

Batteries, such as those disclosed above, operate to provide operating power to controller, sensing and/or therapy module, communication module, and wake-up field generator. Controller operates to control sensing and/or therapy module, communication module, and wake-up field generator, all of which are operatively coupled to and communicate with the controller. For example, controller may command sensing and/or therapy module to deliver a desired therapy, such as a pacing or defibrillation stimulus. In addition, controller may command communication module to transmit and/or receive data from external device or remote CRM device. Furthermore, controller may command wake-up field generator to generate a field (e.g., electromagnetic, magnetic, E-field) that can be detected by a sensor in CRM device, as discussed below.

Controller includes processor, which may be a microprocessor or microcontroller, coupled to memory, which may include operating instructions and/or software for processor. In addition, memory may store parameters related to current and charge consumption information for primary CRM device and remote CRM device. For example, memory may store predetermined current and charge consumption information for an active state of remote CRM device, during which remote CRM device performs at least one function, and an inactive state of remote CRM device, during which remote CRM device is in the sleep state and performs no functions.

Primary CRM device may also include timing circuitry (not shown) which operates to schedule, prompt, and/or activate primary CRM device to perform various activities. For example, in one embodiment, the timing circuitry may be utilized to determine the appropriate time at which one or more remote CRM device should wake in order to perform a designated function. In one embodiment, the timing circuitry may be an internal timer or oscillator, while in other embodiments timing may be performed by specific hardware components that contain hardwired logic for performing the steps, or by any combination of programmed computer components and custom hardware components.

Communication module is configured to allow primary CRM device to communicate with other devices, such as external device or remote CRM device. In one embodiment, primary CRM device may communicate with other devices via a wireless connection. Various types of wireless communication that may be used include, but are not limited to, ultrasonic waves, acoustic communications, radio frequency communications, and the like, as will be appreciated by those skilled in the art. In some embodiments, communication module includes an acoustic transmitter/receiver configured for acoustic telemetry.

Sensing and/or therapy module, if present, operates to perform the therapeutic and/or diagnostic functions described above. In one embodiment, sensing and/or therapy module delivers a cardiac pacing and/or defibrillation stimulus. Again, sensing and/or therapy module is not limited to performing any particular type of physiologic measurement or therapy.

Wake-up field generator operates to generate a field (i.e., a wake-up field) that can be detected by a sensing module in remote CRM device for the purpose of causing remote CRM device to wake from the sleep state.

Various types of wake-up fields may be used without departing from the scope of the disclosure, including electromagnetic, magnetic, and electric fields. The particular type of wake-up field utilized will depend on variables such as the available power supply and the implantation site(s) of primary CRM device and remote CRM device, and their proximity to one another.

A remote CRM device can be configured to include a battery, a voltage sensor, a physiological sensor, a communication module, a wake-up sensor, a power control circuitry, and a remote CRM device controller. Battery may be non-rechargeable or rechargeable. Battery, such as those disclosed above, operates to supply power to voltage sensor, physiological sensor, communication module, wake-up sensor, and controller. Power control circuitry is operatively connected to the battery and the wake-up sensor, and operates to regulate the supply of power from the battery to voltage sensor, physiological sensor, communication module, wake-up sensor, and controller.

Controller may be of substantially the same type as or identical to the controller of primary CRM device, and may include a microprocessor or microcontroller coupled to a memory device that includes operating instructions and/or software for the microprocessor or microcontroller. Remote CRM device, and in particular the controller, may also include timing circuitry which operates to direct the activities of the remote CRM device (e.g., taking and storing physiologic measurements, uploading measurement data) after it has been awakened from its sleep state. Alternatively, remote CRM device controller may have reduced functionality as compared to primary CRM device controller, in configurations where the functional requirements of remote CRM device are less extensive.

Physiological sensors can be provided which are adapted and configured to perform functions related to measurement of physiological parameters, and is not limited to any particular type of physiological measurement. For example, physiological sensor may be a pressure sensor adapted to measure internal pressure in a blood vessel. In one such embodiment, remote CRM device is implanted in the patient's pulmonary artery, and physiological sensor is adapted to measure blood pressure therein. An example remote CRM device operable to measure blood pressure, which is suitable for use in conjunction with the present disclosure, is disclosed in U.S. Patent Pub. 2009-0312650 A1, entitled "Implantable Pressure Sensor with Automatic Measurement and Storage Capabilities." Remote CRM device may also have the capability to perform one or more therapeutic functions (e.g., cardiac pacing, drug delivery) in addition to, or in lieu of, one or more measurement functions. In one such configuration, remote CRM device includes a therapy delivery module and does not include physiological sensor.

Communication module operates to allow remote CRM device to communicate with other devices, such as external device, primary CRM device, or other remote CRM devices. As discussed above and in more detail below, remote CRM device can communicate with other devices via a wireless connection (i.e., telemetry). As with primary CRM device, the specific type and/or style of wireless communication that can be used is not limited. For example, ultrasonic waves, acoustic communications, radio frequency communications, and the like may be used by the communication circuitry.

In an embodiment, communication module is an acoustic telemetry module and includes an acoustic transmitter/receiver adapted to transmit and receive acoustic signals to/from primary CRM device communication module. In one such embodiment, the transmitter/receiver includes an ultrasonic transducer and associated circuitry.

In some embodiments, voltage sensor, physiological sensor, communication module, and controller may be integrated into an integrated circuit, while in other embodiments one or more of these elements may be discrete hardware and circuitry.

Wake-up sensor includes one or more sensors and circuitry adapted and configured to detect and/or to react to the presence of a wake-up field generated by wake-up field generator of primary CRM device. Wake-up sensor is further adapted to cause, upon detecting the presence of such a wake-up field, physiological sensor, communication module, and/or controller to be awakened, via power control circuitry, as appropriate for performing one or more designated functions such as those described above. In some embodiments, remote CRM device is configured such that, upon wake-up sensor detecting a wake-up field, controller is initially awakened. Thereafter, controller directs the subsequent wake-up and operation of the other functional portions (e.g., voltage sensor, physiological sensor, and/or communication module).

When remote CRM device is implanted in a patient, it is important to monitor the remaining capacity of battery to continuously ensure that remote CRM device has sufficient capacity to measure physiological parameters and/or deliver therapy. Various events associated with remote CRM device that reduce the available charge in battery should be taken into consideration to accurately determine the remaining charge capacity of battery. For example, each function performed by remote CRM device consumes an amount of energy from battery. In addition, when remote CRM device is in a sleep state, a very small amount of leakage current (e.g., less than 1 nA) may still be consumed from battery by the circuit.

Furthermore, when the device comes out of the deep sleep state to perform a function, an amount of current may be consumed by the device. While all of these events that reduce the available charge in battery should be considered, the small size and equilibrium state of battery may prevent accurate *in-situ* measurement of the charge consumed by these events.

Primary CRM device and remote CRM device according to the present disclosure are configured to determine the remaining capacity of battery of remote CRM device based on predetermined charge consumption parameters for the various states of remote CRM device. For example, memory of primary CRM device stores information related to the amount of charge consumed by each function of remote CRM device when remote CRM device is awake (i.e., in the active state). In some embodiments, memory stores information about the amount of current consumed by each function and, for fixed-duration functions, memory stores the amount of charge consumed each time the fixed-duration function is performed. Memory may also store information about the leakage current drawn by remote CRM device from battery when remote CRM device is in the inactive, sleep state.

Furthermore, memory may store current and/or charge consumption information for other types of events that draw energy from battery, such as the current or charge consumed to wake remote CRM device from the sleep state, or to transmit data from remote CRM device via communication module. In essence, memory stores predetermined current and/or charge consumption values for all functions or events that consume charge from battery. The charge consumption parameters stored in memory are "predetermined" in that they are measured or predicted prior to implantation of remote CRM device, such as during or after fabrication of remote CRM device. It should be noted that while the subsequent discussion describes primary CRM device as providing the charge monitoring function for battery, it will be appreciated that other devices may also be adapted to perform this function, including external device.

Examples of functions performed by remote CRM device that consume energy from battery, and for which memory stores predetermined current and/or charge consumption information, include: waking up to perform a function; performing a function, such as measuring a physiological parameter; transmitting data from remote CRM device to another device; measuring voltage information about battery; waking up in response to a wake-up field not intended for remote CRM device; waking up in response to noise around remote CRM device; and storing information to a memory internal to remote CRM device. It will be appreciated that this list is only representative, and that memory can store current and/or charge consumption information for any type of function or event performed by remote CRM device that consumes charge from battery.

A process for determining a remaining charge capacity of battery includes, for example, determining an initial charge capacity of battery and storing the charge capacity in memory. The initial charge capacity represents the charge capacity of battery as most recently determined by primary CRM device. Determining remaining battery power by subtracting usage from initial charge provides only an approximation of remaining power, not a direct measurement of battery charge.

As will be appreciated by those skilled in the art, the more features a CRM device has and the more querying of device status that is performed by a remote system, the more power is used. The micro-generators described and disclosed herein supplement the power available to a CRM device either partially or fully for operation of a particular feature.

After implantation of remote CRM device, the initial charge capacity stored in memory may be the assumed full charge capacity of battery. This determination can be done concurrently with implantation or within a reasonable time following implantation.

Primary CRM device activates remote CRM device from the inactive (sleep) state to the active state to perform one or more functions. Primary CRM device generates a wake-up field via wake-up field generator, which is sensed by wake-up sensor of remote CRM device. Primary CRM device then communicates instructions to remote CRM device related to the function or functions that remote CRM device is to perform while awake. In an alternative embodiment, remote CRM device automatically awakens periodically to perform one or more functions based on, for example, timing signals internal to remote CRM device.

Primary CRM device receives data related to the one or more functions from remote CRM device. Remote CRM device may transmit the data after each function is performed, or remote CRM device may store the data related to the functions for later transmission to primary CRM device. The data related to the functions performed by remote CRM device may include, for example, physiological parameter measurement data (e.g., blood pressure data) and duration of the function. Other information received by primary CRM device may include the type of function performed by remote CRM device and voltage measurement data as sensed by voltage sensor.

Remote CRM device returns to the inactive state: either automatically after remote CRM device performs the functions it was awakened to perform or in response to a signal from primary CRM device.

Processor of primary CRM device computes the charge consumed by remote CRM device during the active state based on the stored information in memory. For example, for functions having a fixed duration, memory stores data about the charge consumed by the fixed-duration functions each time they are performed by remote CRM device.

Charge consumption can then be calculated using the information obtained from the CRM device. Leakage current consumed by remote CRM device while in the inactive state is determined prior to implantation of remote CRM device. Processor thus determines the charge consumed by remote CRM device in the inactive state by multiplying the stored leakage current information by the time remote CRM device is in the inactive state.

The processor can also be configured to determine the remaining charge capacity of battery by subtracting an active state charge consumption computed and an inactive state charge consumption calculated from an initial charge capacity stored in memory. The remaining charge capacity is then stored in memory, and becomes the new initial charge capacity when the remaining charge capacity is next calculated. The remaining charge capacity may be transmitted to external device(s) for review and analysis by a medical professional.

The frequency at which the remaining charge capacity stored in memory is updated may also be programmed into controller. For example, controller may update the remaining charge capacity periodically, such that the total charge consumed from battery over a period of time is subtracted from the initial charge capacity stored in memory to determine the remaining charge capacity. Controller may alternatively update the remaining charge capacity after the occurrence of an event, such as after each time remote CRM device returns to the inactive state. Controller may also continuously update the remaining charge capacity stored in memory in real-time. Controller may also store a running total of the total charge consumed by remote CRM device from implantation and compare this total to the charge capacity of battery at implantation to determine the remaining charge capacity.

The remaining charge available from battery is oftentimes closely related to the voltage of battery. Thus, the voltage of battery may also be monitored to ensure an accurate assessment of the amount of charge remaining in battery. Remote CRM device includes voltage sensor operable to generate signals related to the voltage of battery. Primary CRM device may wake remote CRM device and command remote CRM device to measure the voltage of battery. Primary CRM device may be programmed to perform this action periodically or on aperiodic occasions.

Alternatively, remote CRM device may be programmed to automatically awaken and measure the voltage of battery. In any case, remote CRM device transmits the voltage measured by voltage sensor to primary CRM device.

Remote CRM device may then return to the inactive, sleep state. In some embodiments, the amount of current or charge consumed by remote CRM device to perform this measurement and transmit the data is also stored in memory.

Primary CRM device may then determine the amount of charge consumed by remote CRM device based on the voltage of battery. In some embodiments, memory stores an algorithm executed by processor for calculating the remaining charge capacity of battery based on the measured voltage of battery. In other embodiments, a battery voltage profile is stored in memory that correlates a measured voltage of battery to the remaining charge capacity of battery.

## IV. IMPLANTABLE CARDIAC STIMULATION DEVICES, BATTERIES AND COMMUNICATION NETWORKS

As will be appreciated by those skilled in the art, modular and scalable system employing the implantable devices having an optimized micro-generator discussed above can be provided which are comprised of a controller and more than one implantable device having an optimized micro-generator. Controller communicates with each implantable device having an optimized micro-generator over a communication media. For example, the system can be configured to enable a single healthcare provider to be in communication with all, or a selected number, of the implantable devices having an optimized micro-generator that have been implanted into that healthcare provider's patients.

Communication media may be a wired point-to-point or multi-drop configuration. Examples of wired communication media include Ethernet, USB, and RS-232. Alternatively communication media may be wireless including radio frequency (RF) and optical. The cardiac device may have one or more slots for fluid processing devices. Networked devices can be particularly useful in some situations. For example, networked devices that provide battery-monitoring results to a care provider (such as a doctor) can facilitate background analysis of usage of device, battery usage or projected battery life, which could then trigger earlier intervention by a healthcare provider when results begin trending in a clinically undesirable direction or approach an unacceptable threshold level. Additionally, automatic messages in response to sample measurements can be generated to either the patient and/or to the care provider. In some instances, automatic messages may be generated by the system. The networked communication system therefore enables background health monitoring and early intervention, which can be achieved at a low cost with the least burden to health care practitioners.

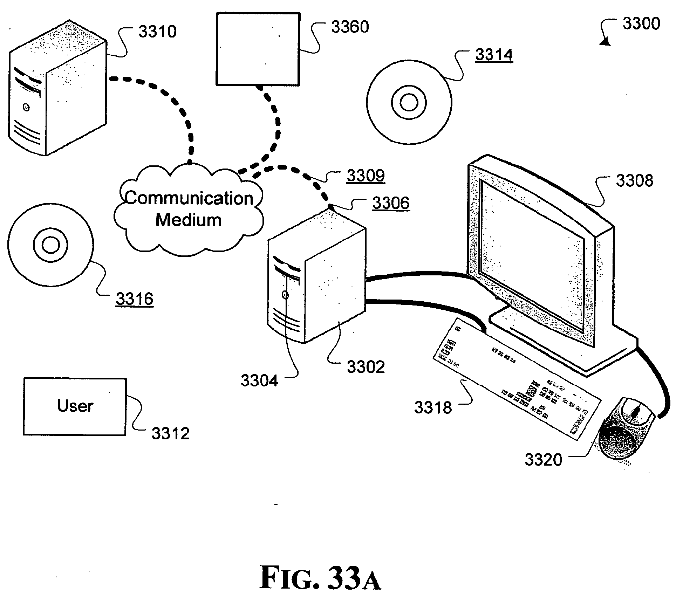

To further appreciate the networked configurations of multiple implantable devices having an optimized micro-generator in a communication network, **FIG. 33A** is a block diagram showing a representative example logic device through which a browser can be accessed to control and/or communicate with implantable devices having an optimized micro-generator and/or diagnostic devices as described above. A computer system (or digital device) **3300,** which may be understood as a logic apparatus adapted and configured to read instructions from media **3314** (such as computer readable media) and/or network port **3306,** is connectable to a server **3310,** and has a fixed media **3316.** The computer system **3300** can also be connected to the Internet or an intranet. The system includes central processing unit (CPU) **3302,** disk drives **3304,** optional input devices, illustrated as keyboard **3318** and/or mouse **3320** and optional monitor **3308.** Data communication can be achieved through, for example, communication medium **3309** to a server **3310** at a local or a remote location. The communication medium **3309** can include any suitable means of transmitting and/or receiving data. For example, the communication medium can be a network connection, a wireless connection, or an internet connection. It is envisioned that data relating to the use, operation or function of one or more CRM devices (shown for purposes of illustration here as **3360**) can be transmitted over such networks or connections. The computer system can be adapted to communicate with a user (users include healthcare providers, physicians, lab technicians, nurses, nurse practitioners, patients, and any other person or entity which would have access to information generated by the system) and/or a device used by a user. The computer system is adaptable to communicate with other computers over the Internet, or with computers via a server. Moreover the system is configurable to activate one or more devices associated with the network (e.g., CRM device) and to communicate status and/or results of tests performed by the CRM device.

For the purposes of this disclosure, media such as a computer readable medium, stores computer data, which data can include computer program code that is executable by a computer, in machine-readable form. By way of example, and not limitation, a computer readable medium may comprise computer readable storage media, for tangible or fixed storage of data, or communication media for transient interpretation of code-containing signals. Computer readable storage media, typically includes a physical or tangible storage and includes without limitation volatile and non-volatile, removable and non-removable storage media implemented in any method or technology for the tangible storage of information such as computer-readable instructions, data structures, program modules or other data. Computer readable storage media includes, but is not limited to, RAM, ROM, EPROM, EEPROM, flash memory or other solid state memory technology, CD-ROM, DVD, or other optical storage, magnetic cassettes, magnetic tape, magnetic disk storage or other magnetic storage devices, or any other physical or material medium which can be used to tangibly store the desired information or data or instructions and which can be accessed by a computer or processor.

As is well understood by those skilled in the art, the Internet is a worldwide network of computer networks. Today, the Internet is a public and self-sustaining network that is available to many millions of users. The Internet uses a set of communication protocols called TCP/IP (i.e., Transmission Control Protocol/Internet Protocol) to connect hosts. The Internet has a communications infrastructure known as the Internet backbone. Access to the Internet backbone is largely controlled by Internet Service Providers (ISPs) that resell access to corporations and individuals.

The Internet Protocol (IP) enables data to be sent from one device (e.g., a phone, a Personal Digital Assistant (PDA), a computer, etc.) to another device on a network. There are a variety of versions of IP today, including, e.g., IPv4, IPv6, etc. Other IPs are no doubt available and will continue to become available in the future, any of which can, in a communication network adapted and configured to employ or communicate with one or more CRM devices, be used without departing from the scope of the disclosure. Each host device on the network has at least one IP address that is its own unique identifier and acts as a connectionless protocol. The connection between end points during a communication is not continuous. When a user sends or receives data or messages, the data or messages are divided into components known as packets. Every packet is treated as an independent unit of data and routed to its final destination - but not necessarily via the same path.

The Open System Interconnection (OSI) model was established to standardize transmission between points over the Internet or other networks. The OSI model separates the communications processes between two points in a network into seven stacked layers, with each layer adding its own set of functions. Each device handles a message so that there is a downward flow through each layer at a sending end point and an upward flow through the layers at a receiving end point. The programming and/or hardware that provides the seven layers of function is typically a combination of device operating systems, application software, TCP/IP and/or other transport and network protocols, and other software and hardware.

Typically, the top four layers are used when a message passes from or to a user and the bottom three layers are used when a message passes through a device (e.g., an IP host device). An IP host is any device on the network that is capable of transmitting and receiving IP packets, such as a server, a router or a workstation. Messages destined for some other host are not passed up to the upper layers but are forwarded to the other host. The layers of the OSI model are listed below. Layer 7 (i.e., the application layer) is a layer at which, e.g., communication partners are identified, quality of service is identified, user authentication and privacy are considered, constraints on data syntax are identified, etc. Layer 6 (i.e., the presentation layer) is a layer that, e.g., converts incoming and outgoing data from one presentation format to another, etc. Layer 5 (i.e., the session layer) is a layer that, e.g., sets up, coordinates, and terminates conversations, exchanges and dialogs between the applications, etc. Layer-4 (i.e., the transport layer) is a layer that, e.g., manages end-to-end control and error-checking, etc. Layer-3 (i.e., the network layer) is a layer that, e.g., handles routing and forwarding, etc. Layer-2 (i.e., the data-link layer) is a layer that, e.g., provides synchronization for the physical level, does bit-stuffing and furnishes transmission protocol knowledge and management, etc. The Institute of Electrical and Electronics Engineers (IEEE) sub-divides the data-link layer into two further sub-layers, the MAC (Media Access Control) layer that controls the data transfer to and from the physical layer and the LLC (Logical Link Control) layer that interfaces with the network layer and interprets commands and performs error recovery. Layer 1 (i.e., the physical layer) is a layer that, e.g., conveys the bit stream through the network at the physical level. The IEEE sub-divides the physical layer into the PLCP (Physical Layer Convergence Procedure) sub-layer and the PMD (Physical Medium Dependent) sub-layer.

Wireless networks can incorporate a variety of types of mobile devices, such as, e.g., cellular and wireless telephones, PCs (personal computers), laptop computers, wearable computers, cordless phones, pagers, headsets, printers, PDAs, etc. and suitable for use in a system or communication network that includes one or more CRM devices. For example, mobile devices may include digital systems to secure fast wireless transmissions of voice and/or data. Typical mobile devices include some or all of the following components: a transceiver (for example a transmitter and a receiver, including a single chip transceiver with an integrated transmitter, receiver and, if desired, other functions); an antenna; a processor; display; one or more audio transducers (for example, a speaker or a microphone as in devices for audio communications); electromagnetic data storage (such as ROM, RAM, digital data storage, etc., such as in devices where data processing is provided); memory; flash memory; and/or a full chip set or integrated circuit; interfaces (such as universal serial bus (USB), coder-decoder (CODEC), universal asynchronous receiver-transmitter (UART), phase-change memory (PCM), etc.). Other components can be provided without departing from the scope of the disclosure.

Wireless LANs (WLANs) in which a mobile user can connect to a local area network (LAN) through a wireless connection may be employed for wireless communications between one or more CRM devices. Wireless communications can include communications that propagate via electromagnetic waves, such as light, infrared, radio, and microwave. There are a variety of WLAN standards that currently exist, such as Bluetooth®, IEEE 802.11, and the obsolete HomeRF.

By way of example, Bluetooth products may be used to provide links between mobile computers, mobile phones, portable handheld devices, personal digital assistants (PDAs), and other mobile devices and connectivity to the Internet. Bluetooth is a computing and telecommunications industry specification that details how mobile devices can easily interconnect with each other and with non-mobile devices using a short-range wireless connection. Bluetooth creates a digital wireless protocol to address end-user problems arising from the proliferation of various mobile devices that need to keep data synchronized and consistent from one device to another, thereby allowing equipment from different vendors to work seamlessly together.

An IEEE standard, IEEE 802.11, specifies technologies for wireless LANs and devices. Using 802.11, wireless networking may be accomplished with each single base station supporting several devices. In some examples, devices may come pre-equipped with wireless hardware or a user may install a separate piece of hardware, such as a card, that may include an antenna. By way of example, devices used in 802.11 typically include three notable elements, whether or not the device is an access point (AP), a mobile station (STA), a bridge, a personal computing memory card International Association (PCMCIA) card (or PC card) or another device: a radio transceiver; an antenna; and a MAC (Media Access Control) layer that controls packet flow between points in a network.

In addition, Multiple Interface Devices (MIDs) may be utilized in some wireless networks. MIDs may contain two independent network interfaces, such as a Bluetooth interface and an 802.11 interface, thus allowing the MID to participate on two separate networks as well as to interface with Bluetooth devices. The MID may have an IP address and a common IP (network) name associated with the IP address.

Wireless network devices may include, but are not limited to Bluetooth devices, WiMAX (Worldwide Interoperability for Microwave Access), Multiple Interface Devices (MIDs), 802.11x devices (IEEE 802.11 devices including, 802.11a, 802.11b and 802.11g devices), HomeRF (Home Radio Frequency) devices, Wi-Fi (Wireless Fidelity) devices, GPRS (General Packet Radio Service) devices, 3 G cellular devices, 2.5 G cellular devices, GSM (Global System for Mobile Communications) devices, EDGE (Enhanced Data for GSM Evolution) devices, TDMA type (Time Division Multiple Access) devices, or CDMA type (Code Division Multiple Access) devices, including CDMA2000. Each network device may contain addresses of varying types including but not limited to an IP address, a Bluetooth Device Address, a Bluetooth Common Name, a Bluetooth IP address, a Bluetooth IP Common Name, an 802.11 IP Address, an 802.11 IP common Name, or an IEEE MAC address.

Wireless networks can also involve methods and protocols found in, Mobile IP (Internet Protocol) systems, in PCS systems, and in other mobile network systems. With respect to Mobile IP, this involves a standard communications protocol created by the Internet Engineering Task Force (IETF). With Mobile IP, mobile device users can move across networks while maintaining their IP Address assigned once. See Request for Comments (RFC) 3344. NB: RFCs are formal documents of the Internet Engineering Task Force (IETF). Mobile IP enhances Internet Protocol (IP) and adds a mechanism to forward Internet traffic to mobile devices when connecting outside their home network. Mobile IP assigns each mobile node a home address on its home network and a care-of-address (CoA) that identifies the current location of the device within a network and its subnets. When a device is moved to a different network, it receives a new care-of address. A mobility agent on the home network can associate each home address with its care-of address. The mobile node can send the home agent a binding update each time it changes its care-of address using Internet Control Message Protocol (ICMP).

In basic IP routing (e.g., outside mobile IP), routing mechanisms rely on the assumptions that each network node always has a constant attachment point to the Internet and that each node's IP address identifies the network link it is attached to. Nodes include a connection point, which can include a redistribution point or an end point for data transmissions, and which can recognize, process and/or forward communications to other nodes. For example, Internet routers can look at an IP address prefix or the like identifying a device's network. Then, at a network level, routers can look at a set of bits identifying a particular subnet. Then, at a subnet level, routers can look at a set of bits identifying a particular device. With typical mobile IP communications, if a user disconnects a mobile device from the Internet and tries to reconnect it at a new subnet, then the device has to be reconfigured with a new IP address, a proper netmask and a default router. Otherwise, routing protocols would not be able to deliver the packets properly.

SMS (short message system) SMS engine connected is to at least one of the implantable devices having an optimized micro-generator to create an SMS message about the measurement and transmit the SMS message over a network to a recipient device having a predetermined measurement recipient telephone number, and an email engine connected to at least one of the implantable systems having an optimized micro-generator and the implantable devices having an optimized micro-generator to create an email message about the measurement and transmit the email message over the network to a recipient email having a predetermined recipient email address.

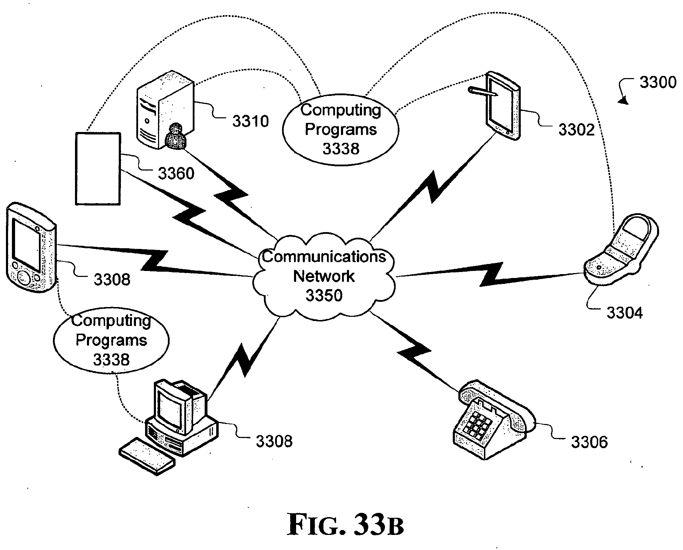

Computing system *3300,* described above, can be deployed as part of a computer network that includes one or more implantable devices having an optimized micro-generator. In general, the above description for computing environments applies to both server computers and client computers deployed in a network environment. **FIG. 33B** illustrates an exemplary illustrative networked computing environment *3300,* with a server in communication with client computers via a communications network *3350.* As shown in **FIG. 33B,** server *3310* may be interconnected via a communications network *3350* (which may be either of, or a combination of a fixed-wire or wireless LAN, WAN, intranet, extranet, peer-to-peer network, virtual private network, the Internet, or other communications network) with a number of client computing environments such as tablet personal computer *3302,* mobile telephone *3304,* telephone *3306*, personal computer *3302,* and personal digital assistant *3308.* In a network environment in which the communications network *3350* is the Internet, for example, server *3310* can be dedicated computing environment servers operable to process and communicate data to and from client computing environments via any of a number of known protocols, such as, hypertext transfer protocol (HTTP), file transfer protocol (FTP), simple object access protocol (SOAP), or wireless application protocol (WAP). Other wireless protocols can be used without departing from the scope of the disclosure, including, for example Wireless Markup Language (WML), DoCoMo i-mode (used, for example, in Japan) and XHTML Basic. Additionally, networked computing environment *3300* can utilize various data security protocols such as secured socket layer (SSL) or pretty good privacy (PGP). Each client computing environment can be equipped with operating system *3238* operable to support one or more computing applications, such as a web browser (not shown), or other graphical user interface (not shown), or a mobile desktop environment (not shown) to gain access to server computing environment *3300*.

In operation, a user (not shown) may interact with a computing application running on a client computing environment to obtain desired data and/or computing applications. The data and/or computing applications may be stored on server computing environment *3300* and communicated to cooperating users through client computing environments over exemplary communications network *3350*. A participating user may request access to specific data and applications housed in whole or in part on server computing environment *3300*. These data may be communicated between client computing environments and server computing environments for processing and storage. Server computing environment *3300* may host computing applications, processes and applets for the generation, authentication, encryption, and communication data and applications and may cooperate with other server computing environments (not shown), third party service providers (not shown), network attached storage (NAS) and storage area networks (SAN) to realize application/data transactions.

## V. KITS

Bundling all devices, tools, components, materials, and accessories needed to use a implantable devices having an optimized micro-generator into a kit may enhance the usability and convenience of the devices. The devices, tools, and components would be sterilized and sealed into suitable packaging designed to prevent contamination. A variety of devices and sizes could be provided in each kit in order to facilitate a surgeon's use of the kit in a sterile patient-treating setting, such as a hospital operating room, or clinic. The kits could contain an implantable device to be powered by an optimized micro-generator. Implantable devices having power needs include, but are not limited to, CRM devices, gastric devices, neural stimulation devices, tissue stimulation devices (e.g., used in conjunction with pain management, food intake, Parkinson's disease, tremors, tissue growth, bone growth, etc.).

Thus, for example, a kit for use with a CRM device could also contain one or more stylets of different diameters, lengths, and flexibility; one or more elongate sheaths having different diameters, lengths, and distal tip (e.g., nose) configurations; one or more pacing leads, having different diameters, different lengths, and with or without the pacing contact incorporated; and/or one or more pacing leads having different tip contact configurations.

Kits configured may be single-use or reusable, or may incorporate some disposable single-use elements and some reusable elements. The kit may contain, but is not limited to, additional components which would be used during a procedure for implanting and/or configuring the CRM device including the following: scissors, scalpels, staples, sutures, electrocautery, needles, syringes, clips, betadine, and tissue preparation material. Additional components can include, for example, alcohol swabs used to clean a tissue surface, prep material to be applied toward a surface and the like. The kit may be supplied in a tray, which organizes and retains all items so that they can be quickly identified and used.

While preferred embodiments of the present invention have been shown and described herein, it will be obvious to those skilled in the art that such embodiments are provided by way of example only. Numerous variations, changes, and substitutions will now occur to those skilled in the art without departing from the invention. It should be understood that various alternatives to the embodiments of the invention described herein may be employed in practicing the invention. It is intended that the following claims define the scope of the invention and that methods and structures within the scope of these claims be covered thereby.

## Claims

1. An implantable micro-generator (970, 1070, 1170) comprising:

   an elongated housing (972, 1072, 1172) adapted and configured to be positioned distally within a tip (226, 326, 426) of a cardiac lead (220, 320, 420) wherein the housing has an elongated interior cavity, a first end and a second end;
   one or more coils (978, 1078, 1178) positioned exteriorly, interiorly or integrally along at least a portion of the housing;
   one or more longitudinally slidable elongated magnets (984, 1084, 1184) having a cross-sectional shape selected from the group comprising: round, triangular, tetragonal, pentagonal, hexagonal, heptagonal, octagonal, nonagonal, decagonal, oval, and ellipsoid, and suspended in the housing between at least one of the one or more coils and at least one of the first end and second end of the housing;
a power wire (432, 532) in electrical communication with the one or more coils and with a medical device, wherein the micro-generator is adapted and configured to generate energy and communicate the generated energy to the medical device; and
further wherein the size of the micro-generator ranges from 7 French to 20 French;
**characterised in that**:

the housing further comprises one or more of at least one of springs (1096, 1096', 1196, 1196') and bumpers (992, 992') at either one or both of the first end and the second end of the cavity of the elongated housing.

2. The implantable micro-generator of claim 1, wherein the one or more coils is an inductive coil.

3. The implantable micro-generator of claim 1, wherein at least one of a first end or a second end of the magnet is configured to be rounded.

4. The implantable micro-generator of claim 1, wherein:

at least one of the one or more of at least one of springs and bumpers is:

highly elastic; or
has a shape selected from conical, cylindrical, straight, square, nub, coiled, spherical, trapezoidal, leaf; or
are adapted and configured to be attached to an end wall (976, 976', 1076, 1076', 1176, 1176') of the housing; or
are adapted and configured to be positioned within the housing without attachment to an interior surface of the housing.

5. The implantable micro-generator of claim 1, wherein the spring:

comprises a gaseous region within the housing adapted and configured to provide a spring characteristic between the magnet and at least one end of the housing; or
is adapted and configured to allow a signal to pass through the micro-generator; or
is at least one of conical, cylindrical, straight, square, nub, coiled, spherical, trapezoidal, leaf; or
is configured to engage the magnet at a first end and a second end to suspend the magnet between the ends of the cavity of the housing

6. The implantable micro-generator of claim 1, wherein the spring is connected to the magnet.

7. The implantable micro-generator of claim 1, wherein the one or more magnets are positioned within the cavity of the elongated housing.

8. The implantable micro-generator of claim 1, further comprising one or more sensors wherein the one or more sensors are at least one of: selected from the group comprising a generator monitor, voltage sensor, a physiological sensor, motion sensor, positional sensor, and a wake-up sensor; in communication with the medical device via one or more signal wires; and are attached to the micro-generator.

9. The implantable micro-generator of claim 1, further wherein the cavity is evacuated and the magnet is suspended in the vacuum.

10. The implantable micro-generator of claim 1, further comprising at least one or more fluid or gas within the cavity.

11. The implantable micro-generator of claim 1, wherein the medical device further comprises electronics adapted and configured to process a power signal received from the micro-generator.

12. The implantable micro-generator of claim 1, wherein the medical device further comprises electronics adapted and configured to buffer a power signal received from the micro-generator.

13. The implantable micro-generator of claim 1, wherein the medical device further comprises a storage component adapted and configured to store energy generated by the micro-generator.

14. The implantable micro-generator of claim 1, wherein the micro-generator is adapted and configured to rectify an oscillating voltage waveform.

15. The implantable micro-generator of claim 1, further comprising one or more rechargeable batteries.

16. The implantable micro-generator of claim 1, further comprising one or more capacitors (614) adapted and configured to at least one of store and buffer a power from the micro-generator.

17. The implantable micro-generator of claim 1, further comprising a multiplexer adapted and configured to multiplex one or more signals along the wires from a tip of the lead to the micro-generator and the control device.

18. An implantable micro-generator of claim 1, further comprising a networked micro-generator:

   a memory;
   a processor;
   a communicator;
   a display; and
   at least one of a cardiac rhythm management system further comprising a cardiac rhythm management device, an elongated housing adapted and configured to be positioned distally within a tip of a cardiac lead wherein the housing has an elongated interior cavity, a first end and a second end, one or more longitudinally slidable elongated magnets, one or more coils positioned exteriorly, interiorly or integrally along at least a portion of the housing, a power wire in electrical communication with the one or more coils and with an medical device, wherein the cardiac rhythm management system is adapted and configured to generate energy and communicate the generated energy to the medical device, and a neural stimulation device comprising a power supply, an electrode, a neural stimulator connected to the power supply, the neural stimulator being adapted to generate a neural stimulation signal for delivery to the neural stimulation target through the electrode, a controller connected to the power supply, and further connected to the neural stimulator to control the neural stimulator according to a neural stimulation protocol to deliver a neural stimulation therapy, a pressure sensor electrically connected to the controller; and an micro-generator comprising, an elongated housing adapted and configured to be positioned distally within a tip of an electrode wherein the housing has an elongated interior cavity, a first end and a second end, one or more longitudinally slidable elongated magnets, one or more coils positioned exteriorly, interiorly or integrally along at least a portion of the housing, a power wire in electrical communication with the one or more coils and with the neural stimulation device, wherein the micro-generator is adapted and configured to generate energy and communicate the generated energy to the neural stimulation device.

19. An implantable micro-generator of claim 1, further comprising a communication system (3300) having:

   a server computer system (3310);
   a measurement module on the server computer system for permitting the transmission of a measurement from the cardiac rhythm management system over a network;
   at least one of an API engine connected to at least one of the cardiac rhythm management system and the cardiac rhythm management device to create a message about a sensed parameter and transmit the message over an API integrated network to a recipient having a predetermined recipient user name, an SMS engine connected to at least one of the cardiac rhythm management system and the cardiac rhythm management device to create an SMS message about the measurement and transmit the SMS message over a network to a recipient device having a predetermined measurement recipient telephone number, and an email engine connected to at least one of the cardiac rhythm management system and the cardiac rhythm management device to create an email message about the measurement and transmit the email message over the network to a recipient email having a predetermined recipient email address.

**Patentansprüche**

1. Implantierbarer Mikrogenerator (970, 1070, 1170), umfassend:

   ein längliches Gehäuse (972, 1072, 1172), welches geeignet und ausgestaltet ist, distal innerhalb einer Spitze (226, 326, 426) einer Herzleitung (220, 320, 420) angeordnet zu werden, wobei das Gehäuse einen länglichen inneren Hohlraum, ein erstes Ende und ein zweites Ende aufweist;

eine oder mehrere Spulen (978, 1078, 1178), welche außerhalb, innerhalb oder integriert entlang zumindest eines Abschnitts des Gehäuses angeordnet sind;

einen oder mehrere in Längsrichtung verschiebbare längliche Magnete (984, 1084, 1184) mit einer Querschnittsform, welche ausgewählt ist aus der Gruppe, welche umfasst: rund, dreieckig, tetragonal, pentagonal, hexagonal, heptagonal, oktagonal, nonagonal, dekagonal, oval und ellipsoid, und welche in dem Gehäuse zwischen mindestens einer der einen oder mehreren Spulen und dem ersten Ende und/oder zweiten Ende des Gehäuses hängen;

einen Stromdraht (432, 532), welcher sich in einer elektrischen Verbindung mit der einen oder den mehreren Spulen und mit einer medizinischen Vorrichtung befindet,

wobei der Mikrogenerator geeignet und ausgestaltet ist, Energie zu erzeugen und die erzeugte Energie zu der medizinischen Vorrichtung zu übertragen; und

wobei die Abmessung des Mikrogenerators von 7 French bis 20 French reicht;

**dadurch gekennzeichnet, dass**:

das Gehäuse ferner eine/einen oder mehrere Federn (1096, 1096', 1196, 1196') und/oder Puffer (992, 992') an entweder einem oder beiden von dem ersten Ende und dem zweiten Ende des Hohlraums des länglichen Gehäuses umfasst.

2. Implantierbarer Mikrogenerator nach Anspruch 1, wobei die eine oder die mehreren Spulen eine induktive Spule ist.

3. Implantierbarer Mikrogenerator nach Anspruch 1, wobei ein erstes Ende und/oder ein zweites Ende des Magneten ausgestaltet ist, abgerundet zu sein.

4. Implantierbarer Mikrogenerator nach Anspruch 1, wobei:

mindestens eine/einer von der einen/dem einen oder den mehreren Federn und/oder Puffern:

hochelastisch ist; oder
eine Form aufweist, welche ausgewählt ist aus konisch, zylindrisch, gerade, eckig, noppenförmig, gewendelt, kugelförmig, trapezoid, blattförmig; oder
geeignet und ausgestaltet sind, an einer Endwand (976, 976', 1076, 1076', 1176, 1176') des Gehäuses angebracht zu werden; oder
geeignet und ausgestaltet sind, innerhalb des Gehäuses ohne eine Anbringung an einer Innenfläche des Gehäuses angeordnet zu werden.

5. Implantierbarer Mikrogenerator nach Anspruch 1, wobei die Feder:

einen gashaltigen Bereich innerhalb des Gehäuses umfasst, welcher geeignet und ausgestaltet ist, eine Federeigenschaft zwischen dem Magnet und mindestens einem Ende des Gehäuses bereitzustellen; oder
geeignet und ausgestaltet ist, einem Signal zu ermöglichen, durch den Mikrogenerator zu verlaufen; oder
konisch, zylindrisch, gerade, eckig, noppenförmig, gewendelt, kugelförmig, trapezoid und/oder blattförmig ist; oder
ausgestaltet ist, den Magnet an einem ersten Ende und an einem zweiten Ende zu koppeln, um den Magnet zwischen die Enden des Hohlraums des Gehäuses zu hängen.

6. Implantierbarer Mikrogenerator nach Anspruch 1, wobei die Feder mit dem Magnet verbunden ist.

7. Implantierbarer Mikrogenerator nach Anspruch 1, wobei der eine oder die mehreren Magnete innerhalb des Hohlraums des länglichen Gehäuses angeordnet sind.

8. Implantierbarer Mikrogenerator nach Anspruch 1, ferner umfassend einen oder mehrere Sensoren, wobei der eine oder die mehreren Sensoren ausgewählt sind aus der Gruppe, umfassend eine Generatorüberwachungsvorrichtung, einen Spannungssensor, einen physiologischen Sensor, einen Bewegungssensor, einen Lagesensor und einen Warnsensor; und/oder in Verbindung mit der medizinischen Vorrichtung über einen oder mehrere Signaldrähte stehen; und/oder an dem Mikrogenerator angebracht sind.

9. Implantierbarer Mikrogenerator nach Anspruch 1, wobei ferner der Hohlraum evakuiert ist und der Magnet in dem Vakuum hängt.

10. Implantierbarer Mikrogenerator nach Anspruch 1, ferner umfassend mindestens ein oder mehrere Fluide oder Gase innerhalb des Hohlraums.

11. Implantierbarer Mikrogenerator nach Anspruch 1, wobei die medizinische Vorrichtung ferner Elektronik umfasst, welche geeignet und ausgestaltet ist, ein von dem Mikrogenerator empfangenes Stromsignal zu verarbeiten.

12. Implantierbarer Mikrogenerator nach Anspruch 1, wobei die medizinische Vorrichtung ferner Elektronik umfasst, welche geeignet und ausgestaltet ist, ein von dem Mikrogenerator erzeugtes Stromsignal zu puffern.

13. Implantierbarer Mikrogenerator nach Anspruch 1, wobei die medizinische Vorrichtung ferner eine Speicherkomponente umfasst, welche geeignet und ausgestaltet ist, von dem Mikrogenerator erzeugte Energie zu speichern.

14. Implantierbarer Mikrogenerator nach Anspruch 1, wobei der Mikrogenerator geeignet und ausgestaltet ist, eine oszillierende Spannungswellenform gleichzurichten.

15. Implantierbarer Mikrogenerator nach Anspruch 1, ferner umfassend eine oder mehrere wiederaufladbare Batterien.

16. Implantierbarer Mikrogenerator nach Anspruch 1, ferner umfassend einen oder mehrere Kondensatoren (614), welche geeignet und ausgestaltet sind, Energie von dem Mikrogenerator zu speichern und/oder zu puffern.

17. Implantierbarer Mikrogenerator nach Anspruch 1, ferner umfassend einen Multiplexer, welcher geeignet und ausgestaltet ist, ein oder mehrere Signale entlang der Drähte von einer Spitze der Leitung zu dem Mikrogenerator und der Steuervorrichtung zu multiplexen.

18. Implantierbarer Mikrogenerator nach Anspruch 1, ferner umfassend einen vernetzten Mikrogenerator:

einen Speicher;
einen Prozessor;
eine Kommunikationsvorrichtung;
eine Anzeigevorrichtung; und
ein Herzrhythmusmanagementsystem, ferner umfassend eine Herzrhythmusmanagementvorrichtung, ein längliches Gehäuse, welches geeignet und ausgestaltet ist, distal innerhalb einer Spitze einer Herzleitung angeordnet zu werden, wobei das Gehäuse einen länglichen inneren Hohlraum, ein erstes Ende und ein zweites Ende aufweist, einen oder mehrere in Längsrichtung verschiebbare längliche Magnete, eine oder mehrere Spulen, welche außerhalb, innerhalb oder integriert entlang zumindest eines Abschnitts des Gehäuses angeordnet sind, einen Stromdraht in elektrischer Verbindung mit der einen oder den mehreren Spulen und mit einer medizinischen Vorrichtung, wobei das Herzrhythmusmanagementsystem geeignet und ausgestaltet ist, Energie zu erzeugen und die erzeugte Energie zu der medizinischen Vorrichtung zu übertragen, und/oder eine Neuralstimulationsvorrichtung umfassend eine Energieversorgung, eine Elektrode, einen Neuralstimulator, welcher mit der Energieversorgung verbunden ist, wobei der Neuralstimulator geeignet und ausgestaltet ist, ein Neuralstimulationssignal für eine Zuführung zu einem Neuralstimulationsziel durch die Elektrode zu erzeugen, eine Steuerung, welche mit der Energieversorgung verbunden ist und ferner mit dem Neuralstimulator verbunden ist, um den Neuralstimulator gemäß einem Neuralstimulationsprotokoll anzusteuern, um eine Neuralstimulationstherapie abzugeben, einen Drucksensor, welcher elektrisch mit der Steuerung verbunden ist; und einen Mikrogenerator, umfassend ein längliches Gehäuse, welches geeignet und ausgestaltet ist, distal innerhalb einer Spitze von einer Elektrode angeordnet zu werden, wobei das Gehäuse einen länglichen inneren Hohlraum, ein erstes Ende und ein zweites Ende aufweist, einen oder mehrere in Längsrichtung verschiebbare längliche Magnete, eine oder mehrere Spulen, welche außerhalb, innerhalb oder integriert entlang zumindest eines Abschnitts des Gehäuses angeordnet sind, einen Stromdraht in elektrischer Verbindung mit der einen oder den mehreren Spulen und der Neuralstimulationsvorrichtung, wobei der Mikrogenerator geeignet und ausgestaltet ist, Energie zu erzeugen und die erzeugte Energie zu der Neuralstimulationsvorrichtung zu übertragen.

19. Implantierbarer Mikrogenerator nach Anspruch 1, ferner umfassend ein Kommunikationssystem (3300) mit:

einem Server-Computersystem (3310);

einem Messmodul auf dem Server-Computersystem, um die Übertragung einer Messung von dem Herzrhythmusverwaltungssystem über ein Netzwerk zu ermöglichen;

eine API-Maschine, welche mit dem Herzrhythmusmanagementsystem und/oder der Herzrhythmusmanagementvorrichtung verbunden ist, um eine Nachricht über einen erfassten Parameter zu erzeugen und die Nachricht über ein API-integriertes Netz zu einem Empfänger zu übertragen, welcher einen vorbestimmten Empfängerbenutzernamen aufweist, und/oder eine SMS-Maschine, welche mit dem Herzrhythmusmanagementsystem und/oder der Herzrhythmusmanagementvorrichtung verbunden ist, um eine SMS-Nachricht über die Messung zu erzeugen und die SMS-Nachricht über ein Netzwerk zu einer Empfängervorrichtung zu übertragen, welche eine vorbestimmte Messempfängertelefonnummer aufweist, und/oder eine E-Mail-Maschine, welche mit dem Herzrhythmusmanagementsystem und/oder der Herzrhythmusmanagementvorrichtung verbunden ist, um eine E-Mail-Nachricht betreffend die Messung zu erzeugen und die E-Mail-Nachricht über das Netz zu einer Empfänger-E-Mail zu übertragen, welche eine vorbestimmte Empfänger-E-Mail-Adresse aufweist.

**Revendications**

1. Micro-générateur implantable (970, 1070, 1170) comprenant :

   un boîtier allongé (972, 1072, 1172) adapté et conçu pour être positionné de façon distale à l'intérieur d'une pointe (226, 326, 426) d'une sonde cardiaque (220, 320, 420), dans lequel le boîtier comporte une cavité interne allongée, une première extrémité et une seconde extrémité ;
   une ou plusieurs bobines (978, 1078, 1178) positionnées à l'extérieur, à l'intérieur ou en étant intégrées le long d'au moins une partie du boîtier ;
   un ou plusieurs aimants allongés (984, 1084, 1184) pouvant coulisser longitudinalement et ayant une forme de section transversale sélectionnée parmi le groupe comprenant : une forme ronde, triangulaire, tétragonale, pentagonale, hexagonale, heptagonale, octogonale, nonagonale, décagonale, ovale, et ellipsoïdale, et suspendus dans le boîtier entre au moins l'une des une ou plusieurs bobines et au moins l'une des première extrémité et seconde extrémité du boîtier ;
   un fil électrique (432, 532) en communication électrique avec les une ou plusieurs bobines et avec un dispositif médical,
   dans lequel le micro-générateur est adapté et conçu pour générer de l'énergie et communiquer l'énergie générée au dispositif médical ; et
   dans lequel la taille du micro-générateur varie en outre de 7 French à 20 French ;
   **caractérisé en ce que** :

   le boîtier comprend en outre un ou plusieurs d'au moins un des ressorts (1096, 1096', 1196, 1196') et butoirs (992, 992') au niveau de l'une ou des deux extrémités parmi la première extrémité et la seconde extrémité de la cavité du boîtier allongé.

2. Micro-générateur implantable selon la revendication 1, dans lequel les une ou plusieurs bobines sont des bobines d'induction.

3. Micro-générateur implantable selon la revendication 1, dans lequel au moins l'une d'une première extrémité ou d'une seconde extrémité de l'aimant est conçue pour être arrondie.

4. Micro-générateur implantable selon la revendication 1, dans lequel :

   au moins l'un des un ou plusieurs d'au moins un des ressorts et butoirs est :

   très élastique ; ou
   aune forme sélectionnée parmi une forme conique, cylindrique, droite, carrée, de nope, enroulée, sphérique, trapézoïdale, ou de feuille ; ou
   sont adaptés et conçus pour être fixés à une paroi d'extrémité (976, 976', 1076, 1076', 1176, 1176') du boîtier ; ou
   sont adaptés et conçus pour être positionnés à l'intérieur du boîtier sans être fixés à une surface intérieure du boîtier.

5. Micro-générateur implantable selon la revendication 1, dans lequel le ressort :

comprend une région gazeuse à l'intérieur du boîtier adaptée et conçue pour fournir une caractéristique élastique entre l'aimant et au moins une extrémité du boîtier ; ou

est adapté et conçu pour permettre le passage d'un signal à travers le micro-générateur ; ou

a au moins une forme parmi une forme conique, cylindrique, droite, carrée, de nope, enroulée, sphérique, trapézoïdale, de feuille ; ou

est conçu pour venir en contact avec l'aimant au niveau d'une première extrémité et d'une seconde extrémité afin de suspendre l'aimant entre les extrémités de la cavité du boîtier.

6. Micro-générateur implantable selon la revendication 1, dans lequel le ressort est relié à l'aimant.

7. Micro-générateur implantable selon la revendication 1, dans lequel les un ou plusieurs aimants sont positionnés à l'intérieur de la cavité du boîtier allongé.

8. Micro-générateur implantable selon la revendication 1, comprenant en outre un ou plusieurs capteurs, dans lequel les un ou plusieurs capteurs sont d'au moins un type sélectionné parmi le groupe comprenant un appareil de contrôle de générateur, un capteur de tension, un capteur physiologique, un capteur de mouvement, un capteur de position, et un capteur de réveil ; en communication avec le dispositif médical via un ou plusieurs câbles de signal ; et sont fixés au micro-générateur.

9. Micro-générateur implantable selon la revendication 1, dans lequel la cavité est en outre évacuée et l'aimant est suspendu dans le vide.

10. Micro-générateur implantable selon la revendication 1, comprenant en outre au moins un ou plusieurs fluides ou gaz à l'intérieur de la cavité.

11. Micro-générateur implantable selon la revendication 1, dans lequel le dispositif médical comprend en outre un circuit électronique adapté et conçu pour traiter un signal de puissance reçu du micro-générateur.

12. Micro-générateur implantable selon la revendication 1, dans lequel le dispositif médical comprend en outre un circuit électronique adapté et conçu pour isoler un signal de puissance reçu du micro-générateur.

13. Micro-générateur implantable selon la revendication 1, dans lequel le dispositif médical comprend en outre un composant de stockage adapté et conçu pour stocker l'énergie générée par le micro-générateur.

14. Micro-générateur implantable selon la revendication 1, dans lequel le micro-générateur est adapté et conçu pour redresser une forme d'onde de tension oscillante.

15. Micro-générateur implantable selon la revendication 1, comprenant en outre une ou plusieurs batteries rechargeables.

16. Micro-générateur implantable selon la revendication 1, comprenant en outre un ou plusieurs condensateurs (614) adaptés et conçus pour au moins stocker et isoler une puissance venant du micro-générateur.

17. Micro-générateur implantable selon la revendication 1, comprenant en outre un multiplexeur adapté et conçu pour multiplexer un ou plusieurs signaux le long des fils allant d'une pointe de la sonde au micro-générateur et au dispositif de commande.

18. Micro-générateur implantable selon la revendication 1, comprenant en outre un micro-générateur connecté en réseau ;

une mémoire ;

un processeur ;

un dispositif de communication ;

un dispositif d'affichage ; et

au moins l'un d'un système de gestion du rythme cardiaque comprenant en outre un dispositif de gestion du rythme cardiaque, un boîtier allongé adapté et conçu pour être positionné de façon distale à l'intérieur d'une pointe d'une sonde cardiaque, où le boîtier comporte une cavité interne allongée, une première extrémité et une seconde extrémité, un ou plusieurs aimants allongés pouvant coulisser longitudinalement, une ou plusieurs bobines positionnées à l'extérieur, à l'intérieur ou en étant intégrées le long d'au moins une partie du boîtier, un fil électrique en communication électrique avec les une ou plusieurs bobines et avec un dispositif médical, dans lequel le système de gestion du rythme cardiaque est adapté et conçu pour générer de l'énergie et communiquer l'énergie générée au dispositif médical, et un dispositif de stimulation neurale comprenant une alimentation, une électrode, un stimulateur neural relié à l'alimentation, le stimulateur neural étant adapté pour générer un signal de stimulation neurale destiné à être appliqué à la cible de stimulation neurale par l'électrode, un dispositif de commande relié à l'alimentation, et relié en outre au stimulateur neural pour commander le stimulateur neural selon un protocole de stimulation neurale pour administrer un traitement de stimulation neurale, un capteur de pression relié électriquement au dispositif de commande ; et un micro-générateur comprenant un boîtier allongé adapté et conçu pour être positionné de façon distale à l'intérieur d'une pointe d'une électrode, où le boîtier comporte une cavité interne allongée, une première extrémité et une seconde extrémité, un ou plusieurs aimants allongés pouvant coulisser longitudinalement, une ou plusieurs bobines positionnées à l'extérieur, à l'intérieur ou en étant intégrées le long d'au moins une partie du boîtier, un fil électrique en communication électrique avec les une ou plusieurs bobines et avec le dispositif de stimulation neurale, dans lequel le micro-générateur est adapté et conçu pour générer de l'énergie et communiquer l'énergie générée au dispositif de stimulation neurale.

**19.** Micro-générateur implantable selon la revendication 1, comprenant en outre un système de communication (3300) comprenant :

un système informatique de serveur (3310) ;
un module de mesure sur le système informatique de serveur pour permettre la transmission d'une mesure venant du système de gestion du rythme cardiaque sur un réseau ;
au moins un d'un moteur API relié à au moins l'un du système de gestion du rythme cardiaque et du dispositif de gestion du rythme cardiaque pour créer un message concernant un paramètre détecté et transmettre le message sur un réseau intégré API à un destinataire ayant un nom d'utilisateur destinataire prédéterminé, un moteur SMS relié à au moins l'un du système de gestion du rythme cardiaque et du dispositif de gestion du rythme cardiaque pour créer un message SMS concernant la mesure et transmettre le message SMS sur un réseau à un dispositif destinataire ayant un numéro de téléphone destinataire de mesure prédéterminé, et un moteur email relié à au moins l'un du système de gestion du rythme cardiaque et du dispositif de gestion du rythme cardiaque pour créer un message email concernant la mesure et transmettre le message email sur le réseau à un email destinataire ayant une adresse email destinataire prédéterminée.

**FIG. 1A**

**FIG. 1B**

Patient survival by ejection fraction vs the service life of ICDs

FIG. 1C

**FIG. 2A**

**FIG. 2B**

328

320

344

340

**FIG. 3A**

328

320

344

340

**FIG. 3B**

FIG. 4A

FIG. 4B

FIG. 4C

FIG. 4D

FIG. 5A

FIG. 5C

FIG. 5B

FIG. 5D

**FIG. 6**

**FIG. 7A**

**FIG. 7B**

**FIG. 7C**

**OUTPUT VS. LOAD**

FIG. 8A

Time vs. Voltage

FIG. 8B

FIG. 9

**FIG. 10**

**FIG. 11A**

**FIG. 11B**

**FIG. 12**

**FIG. 13**

**FIG. 14A**

**FIG. 14B**

**FIG. 15**

**FIG. 16A**

**FIG. 16B**

**FIG. 16C**

**FIG. 16D**

**FIG. 17A**

**FIG. 17B**

**FIG. 17C**

**FIG. 17D**

**FIG. 18A**

**FIG. 18B**

**FIG. 19A**

**FIG. 19B**

**FIG. 19C**

**FIG. 20A**

**FIG. 20B**

**FIG. 20C**

**FIG. 21A**

**FIG. 21B**

**FIG. 22A**

**FIG. 22B**

**FIG. 23A**

**FIG. 23B**

**FIG. 24A**

**FIG. 24B**

**FIG. 25**

**FIG. 26A**

**FIG. 26B**

**FIG. 26C**

**FIG. 27**

**FIG. 28**

**FIG. 29A**

**FIG. 29B**

**FIG. 29C**

$$\Phi_B = FLUX$$

$$\mathcal{E} = VOLTAGE$$

$$\mathcal{E} = -\frac{d}{dt}\Phi_B$$

**FIG. 30A**

**FIG. 30B**

COIL DISPLACEMENT

**FIG. 30C**

**FIG. 30D**

**FIG. 31**

**FIG. 32**

**FIG. 33A**

**FIG. 33B**

## REFERENCES CITED IN THE DESCRIPTION

**Patent documents cited in the description**

- US 20040222637 A1, Bednyak
- US 7105939 B2
- WO 2004032788 A, Holzer
- WO 9913940 A
- US 7616990 B
- US 7555344 A
- US 7346382 A
- US 7494459 A
- US 5941906 A
- US 20090312650 A1